(19) Europäisches Patentamt

European Patent Office

Office européen des brevets

(11) **EP 1 325 955 A1**

(12) **EUROPEAN PATENT APPLICATION**

(43) Date of publication:
**09.07.2003 Bulletin 2003/28**

(51) Int Cl.⁷: **C12N 15/11**, A61K 31/7088,
C12Q 1/68

(21) Application number: **02000357.0**

(22) Date of filing: **04.01.2002**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **atugen AG
13125 Berlin (DE)**

(72) Inventors:
• **Klippel-Giese, Anke
10779 Berlin (DE)**

• **Kaufmann, Jörg
10627 Berlin (DE)**
• **Giese, Klaus
10779 Berlin (DE)**

(74) Representative: **Bohmann, Armin K., Dr.
Bohmann & Loosen,
Anwaltssozietät,
Sonnenstrasse 8
80331 München (DE)**

(54) **Compounds and methods for the identification and/or validation of a target**

(57) The present invention is related to a compound, preferably 14 to 30 nucleobases, preferably 17 to 23 nucleobases and more preferably 17 to 21 nucleobases in length, targeted to a nucleic acid whereby the nucleic acid is heterogeneous nuclear RNA (hnRNA). The present invention is also related to a method for the identification and/or validation of a target wherein the target is part of a tumor suppressor related pathway comprising the following step:

a) applying to an expression system a functional oligonucleotide wherein the functional oligonucleotide is specific for an mRNA encoding the tumor suppressor.

EP 1 325 955 A1

**Description**

The present invention is related to new compounds targeting nucleic acids, a composition comprising the same, a method for the identification and/or validation of a target, a method for generating functional oligonucleotides and a method for screening of a candidate compound interacting with a target.

Modern drug development no longer relies on a more or less heuristic approach but typically involves the elucidation of the molecular mechanism underlying a disease or a condition, the identification of candidate target molecules and the evaluation of said target molecules. Once such a validated target molecule, which is herein referred to also as target, is available, drug candidates directed thereto may be tested. In many cases such drug candidates are members of a compound library which may consist of synthetic or natural compounds. Also the use of combinatorial libraries is common. Such compound libraries are herein also referred to as candidate compound libraries. Although in the past this approach has proven to be successful, it is still time and money consuming. Different technologies are currently applied for target identification and target validation.

One approach for identifying targets is the use of knockout mice. A sizeable number of all knockout mouse experiments, however, show embryonic lethality or no obvious phenotype because of redundant gene function. In addition, knockouts provide only limited information due to the complex and sometimes rather artificial genetic background arising from their generation.

Another common approach for identifying drug targets and/or diagnostic markers is the comparision of gene expression in normal versus tumor cells. However, tumor cells are genetically very unstable and acquire massive changes in their gene expression pattern very quickly. Many of these changes are rather indirect and the result of chromosomal instability, and therefore not necessarily related to the disease. A major proportion of differentially expressed genes in normal versus cancer cells are therefore not causatively linked to the disease.

The problem underlying the present invention was thus to design a strategy for the identification and/or validation of targets which are functionally linked to tumor suppressors.

In a further aspect the problem underlying the present invention was to provide a method for identifying/validating a defined number of key targets that are relevant for the pathological phenotype being related to pathways regulated or influenced by tumor suppressors.

In a first aspect the problem is solved by a compound, preferably 14 to 30 nucleobases, preferably 17 to 23 nucleobases and more preferably 17 to 21 nucleobases in length, targeted to a nucleic acid whereby the nucleic acid is heterogeneous nuclear RNA (hnRNA).

In a second aspect the problem is solved by a compound, preferably 14 to 30 nucleobases, preferably 17 to 23 nucleobases and more preferably 17 to 21 nucleobases in length, targeted to a nucleic acid whereby the nucleic acid is an intron of a nucleic acid molecule.

In a preferred embodiment of both aspects the compound is a functional oligonucleotide.

In a more preferred embodiment the functional oligonucleotide is selected from the group comprising antisense oligonucleotide, ribozyme and RNAi.

In an embodiment of both aspects the nucleic acid is a genomic sequence.

In a preferred embodiment of both aspects the nucleic acid molecule or a part thereof is coding for a polypeptide.

In a more preferred embodiment of both aspects the functional oligonucleotide comprises at least one modified internucleoside linkage.

In an even more preferred embodiment of both aspects the modified internucleoside linkage is a phosphorothioate linkage.

In an embodiment of both aspects the functional oligonucleotide comprises at least one modified sugar moiety.

In a preferred embodiment of both aspects the modified sugar moiety is a 2'-O-methoxy or a 2'O-methoxyethyl sugar moiety.

In an embodiment of both aspects the functional oligonucleotide comprises at least one modified nucleobase.

In a preferred embodiment of both aspects the modified nucleobase is a 5'-methylcytosine.

In an embodiment of both aspects the compound, preferably the functional oligonucleotide, is a chimeric oligonucleotide.

In a preferred embodiment of both aspects the compound shows the following structure:

$$\text{cap-}(n_p)_x(N_s)_y(n_p)_z\text{-cap}$$

whereby cap represents inverted deoxy abasics or similar modifications

n represents 2'-O-methyl ribonucleotides ;
N represents phosphorothioate-linked deoxyribonucleotides ,
subscript p represents phosphodiester linkage, and
subscript s represents phosphorothioate linkage.

subscript x represents an integer from 5 to 7;
subscript y represents an integer from 7 to 9; and
subscript z represents an integer from 5 to 7.

In a third aspect the problem is solved by a composition comprising a compound according to the present invention and a pharmaceutically acceptable carrier or diluent.

In a fourth aspect the problem is solved by a method for inhibiting the expression of a gene in a cell or tissue of a mammal , preferably in vitro, comprising contacting said cells or tissues, preferably in vitro, with a compound according to the present invention so that the expression of the gene is inhibited.

In a preferred embodiment the mammal is selected from the group comprising mice, rats, guinea pigs, hamsters, monkeys, dogs and cats.

In a fifth aspect the problem is solved by a use of the sequence of an intron of a gene comprising at least one intron and at least one exon for the design of a compound targeting said gene, whereby the compound is an functional oligonucleotide, preferably a functional oligonucleotide according to the present invention.

In a sixth aspect the problem is solved by a method for the identification and/or validation of a target comprising the following step:

> a) applying to an expression system a functional oligonucleotide wherein the functional oligonucleotide is specific for PTEN hnRNA,.

In a seventh aspect the problem is solved by a method for the identification and/or validation of a target comprising the following step:

> a) applying to an expression system a functional oligonucleotide wherein the functional oligonucleotide is specific for PTEN mRNA.

In an embodiment of the sixth and the seventh aspect of the present invention the target is part of the PI3K/PTEN related pathway.

In a further embodiment of the sixth and the seventh aspect of the present invention the target is part of a pathway which is selected from the group comprising the Akt related pathway, the EGF-related autocrine loop and the mTOR pathway.

In another embodiment of the sixth and the seventh aspect of the present invention the target is involved in the pathogenetic mechanism of a disease or condition selected from the group comprising glioblastoma, prostate cancer, breast cancer,,lung cancer, liver cancer, colon cancer, pancreatic cancer and leukaemia.

In a preferred embodiment of the sixth and the seventh aspect of the present invention the target is involved in a biological process selected from the group comprising proliferation, cell survival, migration, apoptosis, stress signalling, metastasis, anoikis, cell attachment and processes signalling through modulation of PI3K activity.

In a further embodiment of the sixth and the seventh aspect of the present invention method the target is selected from the group comprising transcription factors, motility factors, cell cycle factors, cell cycle inhibitors, enzymes, growth factors, cytokines, and tumor suppressors.

In a preferred embodiment of the sixth and the seventh aspect of the present invention the target is a tumor suppressor and wherein the tumor suppressor is selected from the group comprising landscapers, gatekeepers and caretakers.

In an even more preferred embodiment of the sixth and the seventh aspect of the present invention the method further comprises as step b)

comparing the expression pattern of the expression system upon application of the functional oligonucleotide with the expression pattern of the expression system under control conditions.

In another embodiment of the sixth and the seventh aspect of the present invention a further expression modifying agent is applied to the expression system, the expression pattern of the expression system is detected and the expression pattern is compared to the expression pattern generated upon steps a) and/or b).

In a preferred embodiment of the sixth and the seventh aspect of the present invention the expression modifying agent is a functional oligonucleotide.

In a further embodiment of the sixth and the seventh aspect of the present invention the expression modifying agent is modifying the expression of a second target, preferably a target as described in the above paragraphs.

In a preferred embodiment of the sixth and the seventh aspect of the present invention the second target is different from the first target.

In an embodiment of the sixth and the seventh aspect of the present invention the target is the molecular target of PTEN, preferably of PTEN acting as a tumor suppressor.

In an eighth aspect the problem is solved by a method for the identification and/or validation of a target wherein the target is part of a tumor suppressor related pathway comprising the following step:

a) applying to an expression system a functional oligonucleotide wherein the functional oligonucleotide is specific for hnRNA of a tumor suppressor, preferably the non-coding part thereof.

In a ninth aspect the problem is solved by a method for the identification and/or validation of a target wherein the target is part of a tumor suppressor related pathway comprising the following step:

a) applying to an expression system a functional oligonucleotide wherein the functional oligonucleotide is specific for an mRNA encoding the tumor suppressor.
In an embodiment of the eighth and ninth aspect of the present invention the target is involved in the pathogenetic mechanism of a disease or condition selected from the group comprising glioblastoma, prostate cancer, breast cancer, lung cancer, liver cancer, colon cancer, pancreatic cancer and leukaemia.

In a preferred embodiment of the eighth and ninth aspect of the present invention the target is involved in a biological process selected from the group comprising proliferation, cell survival, migration, apoptosis, stress signalling, metastasis, anoikis, cell attachment. processes involving activation of PI3K and cancer relevant pathways involving signalling induced by various growth factors or cytokines.

In another embodiment of the eighth and ninth aspect of the present invention the target is a tumor suppressor and the tumor suppressor is selected from the group comprising landscapers, gatekeepers and caretakers.

In a preferred embodiment of the eighth and ninth aspect of the present invention the method further comprises as step b)
comparing the expression pattern of the expression system upon application of the functional oligonucleotide with the expression pattern of the expression system under control conditions.

In an embodiment of the eighth and ninth aspect of the present invention a further expression modifying agent is applied to the expression system, the expression pattern of the expression system is detected and the expression pattern is compared to the expression pattern generated upon steps a) and/or b).

In a preferred embodiment of the eighth and ninth aspect of the present invention the expression modifying agent is a functional oligonucleotide.

In an even more preferred embodiment of the eighth and ninth aspect of the present invention the expression modifying agent is modifying the expression of a second target, preferably a target as specified in any of the preceding paragraphs.

In a tenth aspect the problem underlying the present invention was solved by an antisense oligonucleotide selected from the group comprising

B ugaacug$C_s$T$_s$sA$_s$sG$_s$sC$_s$sC$_s$sT$_s$sC$_s$sT$_s$sggauuug B (SEQ ID No. 1)

B uggacaa$C_s$sA$_s$sA$_s$sG$_s$sT$_s$sG$_s$sT$_s$sC$_s$sAsaaacccu B (SEQ ID No. 2)

B ggaaacc$T_s$sC$_s$sT$_s$sC$_s$sT$_s$sT$_s$sA$_s$sG$_s$sC$_s$scaacugc B (SEQ ID No. 3)

B uguugca$G_s$sA$_s$sA$_s$sG$_s$sG$_s$sT$_s$sT$_s$sC$_s$sAsuuccugu B (SEQ ID No. 4)

B cuuccga$G_{ss}$A$_{ss}$G$_{ss}$G$_{ss}$A$_{ss}$G$_{ss}$A$_{ss}$G$_{ss}$A$_{ss}$acugagc B (SEQ ID No. 5)

B ccacaaa$C_{ss}$T$_{ss}$G$_{ss}$A$_{ss}$G$_{ss}$G$_{ss}$A$_{ss}$T$_{ss}$T$_{ss}$gcaaguu B (SEQ ID No. 6)

B ucugaca$C_{ss}$A$_{ss}$A$_{ss}$T$_{ss}$G$_{ss}$T$_{ss}$C$_{ss}$C$_{ss}$T$_{ss}$auugcca B (SEQ ID No. 7)

B aaggagg$A_{ss}$G$_{ss}$A$_{ss}$G$_{ss}$A$_{ss}$G$_{ss}$A$_{ss}$T$_{ss}$G$_{ss}$gcagaag B (SEQ ID No. 8)


B guccuuu$C_{ss}$C$_{ss}$C$_{ss}$A$_{ss}$G$_{ss}$C$_{ss}$T$_{ss}$T$_{ss}$T$_{ss}$acaguga B (SEQ ID No. 9)

B cuggauc$A_{ss}$G$_{ss}$A$_{ss}$G$_{ss}$T$_{ss}$C$_{ss}$A$_{ss}$G$_{ss}$T$_{ss}$gguguca B (SEQ ID No. 10)

B ucuccuu$T_{ss}$T$_{ss}$G$_{ss}$T$_{ss}$T$_{ss}$T$_{ss}$C$_{ss}$T$_{ss}$G$_{ss}$cuaacga B (SEQ ID No. 11)


B ugaacug$C_s$T$_s$sA$_s$sG$_s$sC$_s$sC$_s$sT$_s$sC$_s$sT$_s$sggauuug B (SEQ ID No. 12)

B ugcugau$C_s$T$_s$sT$_s$sC$_s$sA$_s$sT$_s$sC$_s$sA$_s$sA$_s$saagguuc B (SEQ ID No. 13)

B acuuuga$T_s$sG$_s$sT$_s$sC$_s$sA$_s$sC$_s$sC$_s$sA$_s$sC$_s$sacacagg B (SEQ ID No. 14)

B uggguccT$_s$sG$_s$sA$_s$sG$_s$sT$_s$sT$_s$sG$_s$sG$_s$sA$_s$sggaguag B (SEQ ID No. 15)

B cuucacc$T_s$sT$_s$sT$_s$sA$_s$sG$_s$sC$_s$sT$_s$sG$_s$sG$_s$scagacca B (SEQ ID No. 16)

B ugccacu$G_s$sG$_s$sT$_s$sC$_s$sT$_s$sG$_s$sT$_s$sA$_s$sA$_s$succaggt B (SEQ ID No. 17)

B ucucugg$T_s$sC$_s$sC$_s$sT$_s$sT$_s$sAA$_s$sC$_s$sT$_s$sT$_s$sccccaua B (SEQ ID No. 18)

B ucgucuu$C_s$sA$_s$sC$_s$sT$_s$sT$_s$sA$_s$sG$_s$sC$_s$sC$_s$sauugguc B (SEQ ID No. 19)

B gucuuuc$T_s$sG$_s$sC$_s$sA$_s$sG$_s$sG$_s$sA$_s$sA$_s$sA$_s$succcaua B (SEQ ID No. 20)

whereby B stands for inverted abasic, positions 1 through 7 and positions 17 through 23 are 2'-O-methylated ribonucleotides and are phosphodiester -linked, positions 8 through 17 are phosphorothioate linked, positions 8 through 16 are desoxynucleotides, position 17 is a ribonucleotide;


B g$_s$u$_s$c$_s$cuuu$C_s$C$_s$C$_s$A$_s$G$_s$C$_s$T$_s$T$_s$T$_s$acag$_s$u$_s$g$_s$a B (SEQ ID No. 21)


whereby B stands for inverted abasic, positions 1 through 7 are 2'-O-methylated ribonucleotides, positions 8 through 16 are desoxynucleotides, positions 17 through 23 are 2'-O-methylated ribonucleotides, positions 1 through 4 are phosphorothioate linked, positions 4 through 8 are phosphodiester- -linked, positions 8 through 17 are phosphorothioate -linked, positions 17 through 20 are phosphodiester- linked, and positions 20 through 23 are phosphorothioate linked and

B  agaccaCAAACTGAGgauugc  B  (SEQ  ID  No  50,  also  referred  to  herein  as huPTEN:1686L21),

B  agacgaCTAACTCAGcauugc  B  (SEQ  ID  No  51,  also  referred  to  herein  as huPTEN:1686L21 4MM),

B  cccuuuCCAGCTTTAcaguga  B  (SEQ  ID  No  52,  also  referred  to  herein  as huPTEN:1420L21),

B  ccguuuGCACCTTTAgaguga  B  (SEQ  ID  No  53,  also  referred  to  herein  as huPTEN:1420L21 4MM),

B aagcagCAAAGTCCTaagcag B (SEQ ID No 54, also referred to herein as huPTEN intron),

B cagaauTGGGCTGTAuuuggu B (SEQ ID No 55, also referred to herein as huPTEN intron),

whereby B represents an inverted abasic nucleotide, each and any of the minor letters represents independently from each other a 2'-O-methyl ribonucleotide such as A, G, U and C, and each and any of the capital letters represents independently from each other a phosphorothioate-linked deoxyribonucleotide such as A,G, T and C.

It is to be noted that any of the nucleic acids, more particularly and of the antisense oligonucleotides disclosed herein are preferably third generation antisense oligonucleotides as defined herein unless indicated to the contrary.

In an eleventh aspect the problem underlying the present invention is solved by the use of any of the antisense oligonucleotides as disclosed herein and/or any of the compounds as disclosed herein in a method according as disclosed herein.

In a twelveth aspect the problem underlying the present invention is solved by a method for the generation of a functional oligonucleotide, preferably for use in a method according to any of the preceding claims, comprising the following steps:

a) providing an initial functional oligonucleotide specific for the hnRNA, or the mRNA of a tumor suppressor, preferably PTEN,

b) modifying the initial functional oligonucleotide, and

c) testing the functional oligonucleotide modified in step b) on its specificity for the mRNA.
    In a preferred embodiment of the twelveth aspect of the present invention the testing is done in an expression system.

In a preferred embodiment of the twelveth aspect of the present invention the method further comprises the step of comparing the specificity of the initial and the modified functional oligonucleotide.

In another embodiment of the twelveth aspect of the present invention the initial functional oligonucleotide is any of the inventive antisense oligonucleotides.

In a thirteenth aspect the problem underlying the present invention is solved by a method for the screening of a candidate compound interacting with a target which is either part of a tumor suppressor related pathway or part of a PTEN related pathway, the method comprising the following steps:

-    providing an expression system to which a functional oligonucleotide, preferably the compound according to any of the preceding claims, is added, wherein the functional oligonucleotide is either specific for the hnRNA, preferably the non-coding part thereof, or for the mRNA of a tumor suppressor, whereby preferably the tumor suppressor is PTEN.

-    screening a library of candidate compounds in said expression system to identify one or more elements of the library having activity with regard to interacting with the target and, optionally,

-    identifying said elements.
    It is to be acknowledged that the various features of the embodiments of the inventive methods as disclosed herein as other aspects of the present invention may also be used for the purpose of the method according to the thirteenth aspect of the present invention.

The present invention is based on the surprising finding that compounds targeting to distinct nucleic acids, preferably by a specific interaction such as by hybridisation, may be designed using heterogeneous nuclear RNA (hnRNA). It is within the scope of the present invention that such compound may be addressed to the exon part, the intron part or the region bridging these two parts. It is also within the present invention that the targeting compound is directed to the non-coding parts of a nucleic acid or to the intron part(s) thereof, i. e. those nucleic acid sequences which are not coding for a polypeptide.

The term "targeting" as used herein describes an interaction between the compound and a target nucleic acid. Such interaction may be based on hybridisation using hydrogen bonding, which may be Watson-Crick, Hoogsteen or reversed Hoogsteen hydrogen bonding between essentially complementary nucleoside or nucleotide bases. This complementarity is not necessarily 100 % as known to the one skilled in the art. Rather the degree of complementarity must be such as to allow under the particular conditions that a stable and specific binding occurs between the compound and the target nucleic acid. Specific interaction or targeting is thus realized when upon binding of the compound to the target nucleic acid this interferes with the normal function of the target nucleic acid, such as, e. g., to cause a loss of utility. On the other hand a sufficient degree of complementarity is necessary to avoid non-specific binding of the compound to non-target nucleic acid (sequences) under conditions in which specific binding is desired, i. e. under physiological conditions in the case of in vivo use of the compound such as in *in vivo* assays or therapeutic or diagnostic treatment, and in the case of *in vitro* assays under conditions in which the assays are performed.

Nascent RNA and mRNA intermediates in the nuclei of eukaryotic cells do not exist as free RNA molecules. From the time nascent transcripts first emerge from RNA polymerase II until they are transported into the cytoplasm, they are associated with an abundant set of nuclear proteins, as numerous in growing eukaryotic cells as histones. These proteins were first characterized as being the major protein components of heterogeneous ribonucleoprotein particles (hnRNPs), which contain heterogeneous nuclear RNA (hnRNA), a collective term referring to mRNA precursors (pre-mRNA) and other nuclear RNAs of various sizes. The hnRNA of importance in connection with the present invention, i. e. the one to which the inventive compound is targeted, is thus an intron-containing precursor RNA out of which introns are subsequently removed by the cell's splicing machinery to yield mRNA (Lodish, Baltimore, Berk, Zipursky, Matsudaira, and Darnell; Molecular Cell Biology, 1995).

It is also within the present invention that the compound is directed to hnRNPs and/or to intron(s), or part(s) thereof, of a nucleic acid molecule which is preferably a gene. It is to be acknowledged that factually any hnRNA or gene may thus be targeted by the inventive compounds in view of the technical teaching as disclosed herein. As will be outlined in the following targeting of nucleic acids such as in connection with or by using a functional oligonucleotide or a functional polynucleotide such as an antisense oligonucleotide, ribozyme and RNAi, respectively, is well-known in the art. As used herein the term functional oligonucleotide or functional polynucleotide means any antisense oligonucleotide, any ribozyme and any RNAi. Basically, once a nucleic acid sequence is known the compound according to the present invention may be designed based on the principal of complementarity as outlined above. The target nucleic acid molecule may thus also be a genomic nucleic acid, or at least the inventive compound may be derived from such genomic nucleic acid.

It is within the present invention that such compound targeting this heterogeneous nuclear RNA may be either an antisense oligonucleotide or a ribozyme or RNAi which are as such known to the one skilled in the art.

Using hnRNA and more preferably intron RNA in the design of functional oligonucleotides, the present inventors clearly depart from the approach pursued so far in the art. The reason why hnRNA has not been taken into consideration as a possible target for functional oligonucleotides is that this kind of molecule is very short-lived. Additionally, since intron RNA is non-coding it is generally viewed as somewhat superfluous and unattractive as a target. In view of this the finding of the present inventors was very surprising to see that functional oligonucleotide(s), of which at least some trigger RNAseH activity, on intron sequences could lead to a significant decrease of mRNA levels.

It is within the present invention that the compound preferably comprises about 14 to 30 nucleobases, although, in principle, the nucleic acid forming the compound may be either longer or shorter. A shorter compound, i. e. comprising less than 8 nucleobases, is rather unlikely to exhibit the specificity as required. However, if not only a single but several nucleic acid molecules, more particularly the intron or part thereof, or the hnRNA is to be targeted, compound having less than 14 nucleobases may be useful. Preferred lengths of the inventive compound are from 14 to 30 nucleobases. A length of 17 to 23 is more preferred and a length of 17 to 21 nucleobases is most preferred.

It is also within the present invention that the compound may comprise more than 30 nucleobases. This may become necessary in order to establish an increased specificity to the compound or in case further nucleobases are to be attached which are not necessary for the targeting of the compound but for other purposes. Such other purposes may be cross-linking or providing a substrate to other biological activities such as degradation or non-degradation or specific interaction with other compounds.

Irrespective of the particular use of the compound according to the present invention said compound may be further modified such as by incorporating a label. Typical labeling may be conjugation of an enzyme to the compound and/or radiolabeling of the compound. Other labeling techniques such as non-radiolabeling are also known to the one skilled in the art and, for example, described in Ausubel et al. (Ausubel, F. M. et al. (eds)(1988). Current protocols in molecular biology. New York, Published by Greene Pub. Associates and Wiley-Interscience : J. Wiley).

It is also within the present invention that the inventive compound may be designed such as to be an antisense oligonucleotide according to the second and third antisense oligonucleotide generation as described herein.

Basically, the use of nucleic acids such as polynucleotides for the construction of the functional oligonucleotide is known in the art as well as their use for therapeutic and non-therapeutic purposes. For illustration purposes but not for limiting purposes it is referred to the following publications in relation to the use of antisense oligonucleotides the disclosure of which is incorporated herein by reference (Genasense (Genta Inc), Banerjee D., *Curr Opin Investig Drugs.* 2001 Apr;2(4):574-80; N KC, Wallis AE, Lee CH, De Menezes DL, Sartor J, Dragowska WH, Mayer LD., Effects of Bcl-2 modulation with G3139 antisense oligonucleotide on human breast cancer cells are independent of inherent Bcl-2 protein expression, *Breast Cancer Res Treat.* 2000 Oct;63(3):199-212; Schlagbauer-Wadl H, Klosner G, Heere-Ress E, Waltering S, Moll I, Wolff K, Pehamberger H, Jansen B., Bcl-2 antisense oligonucleotides (G3139) inhibit Merkel cell carcinoma growth in SCID mice, J *Invest Dermatol.* 2000 Apr;114(4):725-30; Cotter FE., Antisense therapy of hematologic malignancies, *Semin Hematol.* 1999 Oct;36(4 Suppl 6):9-14; Tamm I, Dorken B, Hartmann G., Antisense therapy in oncology: new hope for an old idea?, *Lancet.* 2001 Aug 11;358(9280):489-97; Yuen AR, Halsey J, Fisher GA, Holmlund JT, Geary RS, Kwoh TJ, Dorr A, Sikic BI., Phase I study of an antisense oligonucleotide to protein kinase C-alpha (ISIS 3521/CGP 64128A) in patients with cancer, *Clin Cancer Res.* 1999 Nov;5(11):3357-63; Nemunaitis J, Holmlund JT, Kraynak M, Richards D, Bruce J, Ognoskie N, Kwoh TJ, Geary R, Dorr A, Von Hoff D, Eckhardt SG., Phase I evaluation of ISIS 3521, an antisense oligodeoxynucleotide to protein kinase C-alpha, in patients with advanced cancer, J *Clin Oncol.* 1999 Nov;17(11):3586-95; McKay RA, Miraglia LJ, Cummins LL, Owens SR, Sasmor H, Dean NM., Characterization of a potent and specific class of antisense oligonucleotide inhibitor of human protein kinase C-alpha expression, *J Biol Chem.* 1999 Jan 15;274(3):1715-22; Dennis JU, Dean NM, Bennett CF, Griffith JW, Lang CM, Welch DR., Human melanoma metastasis is inhibited following ex vivo treatment with an antisense oligonucleotide to protein kinase C-alpha, *Cancer Lett.* 1998 Jun 5;128(1):65-70; Dean N, McKay R, Miraglia L, Howard R, Cooper S, Giddings J, Nicklin P, Meister L, Ziel R, Geiger T, Muller M, Fabbro D., Inhibition of growth of human tumor cell lines in nude mice by an antisense of oligonucleotide inhibitor of protein kinase C-alpha expression, *Cancer Res.* 1996 Aug 1;56(15):3499-507; Dean NM, McKay R., Inhibition of protein kinase C-alpha expression in mice after systemic administration of phosphorothioate antisense oligodeoxynucleotides, *Proc Natl Acad Sci U S A.* 1994 Nov 22;91(24):11762-6).

Functional oligonucleotides as disclosed and used according to the present invention may also be ribozymes. Ribozymes, their design and general use are known to the one skilled in the art and described, e.g., in *Methods in Molecular Medicine, Vol 11: Therapeutic Applications of Ribozymes,* edited by Kevin J. Scanlon, copyright Humana Press Inc., Totowa, New Jersey, 1998; more particularly the chapters *Methods for Treating HIV by Gene Therapy using an Anti-HIV Type 1 Ribozyme* by Eric M. Poeschla, Mang Yu, Mark C. Leavitt, and Flossie Wong-Staal; *Hammerhead Ribozyme-Mediated Cleavage of Hepatits B Virus RNA* by Fritz von Weizsäcker, Hubert E. Blum, and Jack R. Wands; *Tissue-Specific Delivery of an Anti-Hras Ribozyme against Malignant Melanoma* by Tsukasa Ohkawa and Mohammed Kashani-Sabet; *Anti-c-erb-B-2 Ribozyme for Breast Cancer* by Toshiya Suzuki, Lisa D. Curcio, Jerry Tsai, and Mohammed Kashani-Sabet; *Ribozyme-Mediated Inhibition of Cell Proliferation: A Model for Identifying and Refining a Therapeutic Ribozyme* by Thale C. Jarvis, Dennis Macejak, and Larrz Couture; and *Ribozyme-Mediated Downregulation of Gene Expression in Transgenic Mice* by Shimon Efrat.

Functional oligonucleotides as disclosed and used according to the present invention may also be RNAi. RNAi, its design and general use are known to the one skilled in the art and described, e.g., in WO 00/44895 und WO 01/75164.

The basic structure of the functional oligonucleotides and compounds according to the present invention and more particularly the antisense oligonucleotide(s) as used in connection with the methods according to the present invention are, among others, described in US patent US 5,849,902 (Arrow, A. et al.) issued on December 15, 1998 and US patent US 5,989,912 (Arrow, A. et al.) issued on November 23, 1999. These antisense oligonucleotides typically hybridise to and inhibit the function of nucleic acid such as an RNA, typically a messenger RNA, by activating RNase H. RNase H is activated by both phosphodiester and phosphorothioate-linked DNA. However, phosphodiester-linked DNA is rapidly degraded by cellular nucleases and, with the exception of the phosphorothioate-linked DNA, nuclease resistant, non-naturally occurring DNA derivatives do not activate RNase H when hybridised to RNA. In other words, antisense polynucleotides are effective only in a DNA/RNA hybrid complex.

Chimeric antisense oligonucleotides which may be used in the methods according to the present invention have a short stretch of phosphorothioate DNA (3 to 9 bases). A minimum of 3 DNA bases is required for activation of bacterial RNase H and a minimum of 5 bases is required for mammalian RNase H activation. In these chimeric oligonucleotides there is a central region that forms a substrate for RNase H that is flanked by hybridising "arms" comprised of modified nucleotides that do not form substrates for RNase H. The hybridising arms of the chimeric oligonucleotides may be modified such as by 2'-O-methyl or 2'-fluoro. Alternative approaches used methylphosphonate or phosphoramidate linkages in said arms. Further embodiments of the antisense oligonucleotide useful in the practice of the invention are P-methoxyoligonucleotides, partial P-methoxyoligodeoxyribonucleotides or P-methoxyoligonucleotides.

Of particular relevance and usefulness for the present invention are those antisense oligonucleotides as more particularly described in the above two mentioned US patents. These oligonucleotides contain no naturally occurring 5'→3'-linked nucleotides. Rather the oligonucleotides have two types of nucleotides: 2'-deoxyphosphorothioate, which activate RNase H, and 2'-modified nucleotides, which do not. The linkages between the 2'-modified nucleotides can be phosphodiesters, phosphorothioate or P-ethoxyphosphodiester. Activation of RNase H is accomplished by a contiguous RNase H-activating region, which contains between 3 and 5 2'-deoxyphosphorothioate nucleotides to activate bacterial RNase H and between 5 and 10 2'- deoxyphosphorothioate nucleotides to activate eucaryotic and, particularly, mammalian RNase H. Protection from degradation is accomplished by making the 5' and 3' terminal bases highly nuclease resistant and, optionally, by placing a 3' terminal blocking group.

More particularly, the antisense oligonucleotide comprises a 5' terminus and a 3' terminus; and from 11 to 59 5'→3'-linked nucleotides independently selected from the group consisting of 2'-modified phosphodiester nucleotides and 2'-modified P-alkyloxyphosphotriester nucleotides; and wherein the 5'-terminal nucleoside is attached to an RNase H-activating region of between three and ten contiguous phosphorothioate-linked deoxyribonucleotides, and wherein the 3'-terminus of said oligonucleotide is drawn from the group consisting of an inverted deoxyribonucleotide, a contiguous stretch of one to three phosphorothioate 2'-modified ribonucleotides, a biotin group and a P-alkyloxyphosphotriester nucleotide.

Also an antisense oligonucleotide may be used wherein not the 5' terminal nucleoside is attached to an RNase H-activating region but the 3' terminal nucleoside as specified above. Also, the 5'terminus is drawn from the particular group rather than the 3' terminus of said oligonucleotide.

Suitable and useful antisense oligonucleotides are also those comprising a 5' terminal RNase H activating region and having between 5 and 10 contiguous deoxyphosphorothioate nucleotides; between 11 to 59 contiguous 5'→3'-linked 2'-methoxyribonucleotides; and an exonuclease blocking group present at the 3' end of the oligonucleotide that is drawn from the group consisting of a non-5'-3'-phosphodiester-linked nucleotide, from one to three contiguous 5'-3'-linked modified nucleotides and a non-nucleotide chemical blocking group.

Two classes of particularly preferred antisense oligonucleotides can be characterized as follows:

The first class of antisense oligonucleotides, also referred to herein as second generation of antisense oligonucleotides, comprises a total of 23 nucleotides comprising in 5' → 3' direction a stretch of seven 2'-O-methylribonucleotides, a stretch of nine 2'-deoxyribonucleotides, a stretch of six 2'-O-methylribonucleotides and a 3'-terminal 2'-deoxyribonucleotide. From the first group of seven 2'-O-methylribonucleotides the first four are phosphorothioate linked, whereas the subsequent four 2'-O-methylribonucleotides are phosphodiester linked. Also, there is a phosphodiester linkage between the last, i. e. the most 3'-terminal end of the 2'-O-methylribonucleotides and the first nucleotide of the stretch consisting of nine 2'-deoxyribonucleotides. All of the 2'-deoxyribonucleotides are phosphorothioate linked. A phosphorothioate linkage is also present between the last, i. e. the most 3'-terminal 2'-deoxynucleotide, and the first 2'-O-methylribonucleotide of the subsequent stretch consisting of six 2'-O-methylribonucleotides. From this group of six 2'-O-methylribonucleotides the first four of them, again in 5' → 3' direction, are phosphodiester linked, whereas the last three of them, corresponding to positions 20 to 22 are phosphorothioate linked. The last, i. e. terminal 3'-terminal 2'-deoxynucleotide is linked to the last, i. e. most 3'-terminal 2'-O-methylribonucleotide through a phosphorothioate linkage.

This first class may also be described by reference to the following schematic structure: RRRnnnnNNNNNNNNNnnnRRRN. Hereby, R indicates phosphorothioate linked 2'-O-methyl ribonucleotides (A, G, U, C); n stands for 2'-O-methyl ribonucleotides (A, G, U, C); N represents phosphorothioate linked deoxyribonucleotides (A, G, T, C).

The second class of particularly preferred antisense oligonucleotides, also referred to herein as third generation (of) antisense oligonucleotides, also comprises a total of 17 to 23 nucleotides with the following basic structure (in 5' → 3' direction).

At the 5'-terminal end there is an inverted abasic nucleotide which is a structure suitable to confer resistance against exonuclease activity and, e. g., described in WO 99/54459. This inverted abasic is linked to a stretch of five to seven 2'-O-methylribonucleotides which are phosphodiester linked. Following this stretch of five to seven 2'-O-methylribonucleotides there is a stretch of seven to nine 2'-deoxyribonucleotides all of which are phosphorothioate linked. The linkage between the last, i. e. the most 3'-terminal 2'-O-methylribonucleotide and the first 2'-deoxynucleotide of the 2'-deoxynucleotide comprising stretch occurs via a phosphodiester linkage. Adjacent to the stretch of seven to nine 2'-deoxynucleotides a stretch consistent of five to seven 2'-O-methylribonucleotides is connected. The last 2'-deoxynucleotide is linked to the first 2'-O-methylribonucleotide of the latter mentioned stretch consisting of five to seven 2'-O-methylribonucleotides occurs via a phosphorothioate linkage. The stretch of five to seven 2'-O-methylribonucleotides are phosphodiester linked. At the 3'-terminal end of the second stretch of five to seven 2'-O-methylribonucleotide another inverted abasic is attached.

This second class may also be described by reference to the following schematic structure: (GeneBlocs representing the 3rd generation of antisense oligonucleotides have also the following schematic structure:) cap-$(n_p)_x(N_s)_y$ $(n_p)_z$-cap cap-nnnnnnnNNNNNNNNNNnnnnnnn-cap. Hereby, cap represents inverted deoxy abasics or similar modifications at both ends; n stands for 2'-O-methyl ribonucleotides (A, G, U, C); N represents phosphorothioate-linked deoxyribonucleotides (A, G, T, C); x represents an integer from 5 to 7; y represents an integer from 7 to 9; and z represents an integer from 5 to 7.

It is to be noted that the integers x, y and z may be chosen independently from each other although it is preferred that x and z are the same in a given antisense oligonucleotide. Accordingly, the following basic designs or structures of the antisense oligonucleotides of the third generation can be as follows: cap-$(n_p)_5(N_s)_7(n_p)_5$-cap, cap-$(n_p)_6$ $(N_s)_7(n_p)_5$-cap, cap-$(n_p)_7(N_s)_7(n_p)_5$-cap, cap-$(n_p)_5(N_s)_8(n_p)_5$-cap, cap-$(n_p)_6(N_s)_8(n_p)_5$-cap, cap-$(n_p)_7(N_s)_8(n_p)_5$-cap, cap-$(n_p)_5(N_s)_9(n_p)_5$-cap, cap-$(n_p)_6(N_s)_9(n_p)_5$-cap, cap-$(n_p)_7(N_s)_9(n_p)_5$-cap, cap-$(n_p)_5(N_s)_7(n_p)_6$-cap, cap-$(n_p)_6(N_s)_7$ $(n_p)_6$-cap, cap-$(n_p)_7(N_s)_7(n_p)_6$-cap, cap-$(n_p)_5(N_s)_8(n_p)_6$-cap, cap-$(n_p)_6(N_s)_8(n_p)_6$-cap, cap-$(n_p)_7(N_s)_8(n_p)_6$-cap, cap-$(n_p)_5(N_s)_9(n_p)_6$-cap, cap-$(n_p)_6(N_s)_9(n_p)_6$-cap, cap-$(n_p)_7(N_s)_9(n_p)_6$-cap, cap-$(n_p)_5(N_s)_7(n_p)_7$-cap, cap-$(n_p)_6(N_s)_7$ $(n_p)_7$-cap, cap-$(n_p)_7(N_s)_7(n_p)_7$cap, cap-$(n_p)_5(N_s)_8(n_p)_7$-cap, cap-$(n_p)_6(N_s)_8(n_p)_7$-cap, cap-$(n_p)_7(N_s)_8(n_p)_7$-cap, cap-$(n_p)_5$ $(N_s)_9(n_p)_7$-cap, cap-$(n_p)_6(N_s)_9(n_p)_7$-cap and cap-$(n_p)_7(N_s)_9(n_p)_7$-cap.

Basically, the compound according to the present invention may be used for therapeutic purposes as well as non-therapeutic purposes. Therapeutic purposes may comprise, among others, the use of the compound or of a composition containing such compound for the manufacture of a medicament. In view of the mode of action of the compound according to the present invention any disease, diseased condition or indication may be addressed where modification of the expression of a coding sequence, either directly or indirectly, may affect said disease or condition. A further therapeutic use may be the use of the compound according to the present invention for diagnostic purposes or for the manufacture of a diagnostic agent. The organism subject to the administration of such compound, medicament or diagnostic agent, as well as the organism subject to respective treatment and diagnostic methods, may be selected from the group comprising mice, rats, sheep, goat, dogs, cats, cattle, horses, monkeys and humans.

The compound and compositions containing the same according to the present invention may be formulated in any form known to the one skilled in the art of pharmacy. Such compositions and formulations may be administered in a number of ways depending upon whether local or systemic treatment is desired and upon the way they are to be treated. Administration may be topical (including ophthalmic and to mucous membranes including vaginal and rectal delivery), pulmonary, e.g., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, e.g., intrathecal or intraventricular, administration. Oligonucleotides with at least one 2'-O-methoxyethyl modification are believed to be particularly useful for oral administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

Compositions and formulations for oral administration include powders or granules, suspensions or solutions in water or non-aqueous media, capsules, sachets or tablets. Thickeners, flavoring agents, diluents, emulsifiers, dispersing aids or binders may be desirable.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

For the compounds according to the present invention preferred examples of pharmaceutically acceptable salts include but are not limited to (a) salts formed with cations such as sodium, potassium, ammonium, magnesium, calcium, polyamines such as spermine and spermidine, etc.; (b) acid addition salts formed with inorganic acids, for example hydrochloric acid, hydrobromic acid, sulfuric acid, phosphoric acid, nitric acid and the like; (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (d) salts formed from elemental anions such as chlorine, bromine, and iodine.

Non-therapeutic use of the inventive compounds may preferably reside in the field of diagnostic, analyses, target validation and screening for compounds having an opposite or the same effect as the compound according to the present invention. Preferably such screening is directed to selecting small molecules from a library of small molecules. It is particularly within the present invention to use the inventive compounds for the target validation and target identification methods as disclosed herein.

A further non-therapeutic use of the inventive compounds is the use as research agents and kits. Preferably, such kit comprises at least one compound according to the present invention and either a buffer, any solution, or diluent.

The present invention is also based on the surprising finding that functional oligonucleotides may be generated which allow for the specific or selective reduction of mRNA encoding tumor suppressor(s). This selective reduction or knock down of mRNA coding for tumor suppressor(s) allows for the intense study of all of the pathways to which the tumor suppressor(s) is actually linked and thus for the identification and/or validation of target molecules involved in said pathways. As will be described in more detail below and is also known in the art, the various tumor suppressors such as, e.g., PTEN, Smad 3, SHIP 2 and p53 p16Ink4a, p14Arf, p27, p21, Rb, Smad2, Smad4, APC, Brcal+2, Bcl2, caveolin, VHL, menin, Cpan, DAP kinase, are actually involved in a variety of pathways. These pathways may comprise both upstream and downstream elements or effectors taking tumor suppressor(s) as a reference. All these elements may thus be investigated under conditions where the tumor suppressor(s) or its mRNA is present at normal intracellular levels or at decreased levels. A condition where the tumor suppressor(s) is present at normal cellular levels is preferably taken as a reference against which the pathway or the reaction of the expression system is compared. This may be regarded as a usual control condition although other control conditions may easily be generated as known to one skilled in the art.

Such control conditions can be untreated cells or cells which have been treated with one specific or a mix of several functional oligonucleotides that do not affect the level of the tumor suppressor(s) expression, such as a functional oligonucleotide having a randomised nucleic acid sequence (GBC), mismatch oligos or a functional oligonucleotide against unrelated targets.

It is to be noted that the aforementioned advantages result from the use of any of the functional polynucleotides. In so far antisense oligonucleotides, ribozymes and RNAi may be used for target validation and more particularly in a target validation process where a suppressor such as a tumor suppressor is involved. As used herein the term target validation also means target identification. The particular advantage related to a target validation method which inhibits an inhibitor is that by doing so some targets can be addressed or targeted which otherwise would not be accessible due to the suppressing activity of the suppressor. A particularly preferred group of antisense oligonucleotides used in the methodsfor target identification and target validation are third generation antisense oligonucleotides as described herein.

Also, the identification and/or validation of drug targets specific for metastatic cancer induced by loss of tumor suppressor function, is possible using the technical teaching of the present invention. It is also within the present invention that the various compounds described herein for target validation may also be used as diagnostic tools. Accordingly, a tumor sample may be examined for expression of a specified gene sequence thereby to indicate propensity for metastatic spread (diagnostic markers (e.g. indicative for tumor suppressor negative cells)).

A further most preferred field where the various aspects of the present invention may be applicable is related to genes which are differentially expressed in normal versus cancer cells and are therefore not causatively linked to the disease. To identify the pathologically relevant effector molecules induced by loss of tumor suppressors function such as, e.g., PTEN mutation, it is critical that gene expression profiling experiments are performed under precisely controlled conditions. In this view experimental conditions are required that modulate the pathologically relevant pathway in a way that ensures the functional connection of the identified target genes and the tumor suppressor molecule. Using this novel approach drug targets and/or diagnostic markers can be identified that are specific for the diagnosis and/or treatment of patients with suppressor, more particularly tumor suppressor negative cancers.

The inventive method for the identification and/or validation of a target, more particularly of a target which is linked to the metastatic effects involving or relating to loss of suppressor, more particularly tumor suppressor function, is also particularly advantageous as a subset of downstream targets (representing effectors) of the respective suppressor such as, e. g., the PI 3-kinase/PTEN pathway are likely to represent key regulatory molecules responsible for mediating other important activities such as, in case of PTEN, the metastatic phenotype of cells that have lost PTEN function. It is important to target this particular fraction of effector molecules selectively because targets which act on a parallel branch or further upstream in a signalling cascade are likely to cause unwanted effects. In case of the PTEN pathway this is due to the fact that the PI 3-kinase/PTEN pathway not only regulates cell proliferation and survival, but also processes such as cell migration, intracellular trafficking and insulin signaling. Therefore, it is important to select the downstream effectors which specifically act in the proliferative arm of responses, but not, e.g., in insulin signalling.

Inhibition of insulin signalling is likely to induce unwanted diabetic responses or other side effects. The problem is solved by using a functional oligonucleotide(s) which inhibit the expression of candidate targets in order to validate their functional relevance for the metastatic phenotype. A successful target will be required for invasive cell growth, therefore the inhibition of its expression should interfere with invasive cell growth, but not inhibit other responses mediated by PI 3-kinase. Although illustrated by reference to the PTEN pathway, these considerations basically also apply to other tumor suppressors although they may have other arms of responses as will be acknowledged by the ones skilled in the art which are obvious from the particular regulatory network or pathway in which the respective tumor suppressor is involved.

With this method at hand, the disadvantages of the methods according to the state of the art, i. e. the knockout systems, are thus clearly overcome. This resides in the fact that due to the specific knockout, or better knockdown, of tumor suppressor(s) mediated by functional oligonucleotides, the highly regulated pathways involving any tumor suppressor, can specifically be targeted excluding any possible interference of the genetic background of the knockout animals. In addition, it is well known, particularly when it comes to PTEN knockout mice, that a homozygous knockdown is not viable and die in utero. On the other hand, hemizygous knockouts still have a comparatively high background of tumor suppressor(s) which does not allow for an unambiguous annotation of the effects observed. In addition, it is also well-known that in the generation of a knockout system due to compensational mechanism the number of redundant genes is increased so that only particular rearrangements of genes may actually be obtained. However, in the study of cells stemming from an adult organism which could eventually be the starting point for tumor growth, there are typically no such redundant genes, at least not generated in the early phase of embryogenesis. This latter problem has been overcome by the generation of conditional knockouts which are characterized in that the knockout only happens in the adult animal model. However, the generation of such conditional knockout is very labour intensive and is only applicable to few, specific biological systems.

A further advantage of the methods according to the present invention, as compared to knockout animals, resides in the fact that only by using functional oligonucleotide(s) such as the ones according to the present invention some distinct pathways may actually be targeted. It is known for example that at least in some cases tumor suppressors may only act as such in specific, defined systems. More particularly, certain genes can only act as tumor suppressors in mice, and have never been found to be mutated in human cancers (Macleod, K.; Oncogenes and cell proliferation Current Opinion in Genetics & Development 2000, 10, S. 81 - 93). Such a situation may actually be misleading and supports the need to provide new methods for the identification and/or validation of targets.

Also, it was observed that in some tumor patients the tumor suppressor(s) such as PTEN are not expressed or are undetectable for years. This means that the respective cells are without the tumor suppressive and controlling function of the tumor suppressor(s) for some time. In case of PTEN the lack of this checkpoint is likely to be the determinant for the development into a malignant and invasive tumor from an earlier more benign state. During this period the tumor typically evolves further and the genetic background actually continues to degenerate. Insofar there is a tremendous need to elucidate the early events causing tumorgenesis and the genesis of other diseases or pathological conditions. This need can now be satisfied by the methods and the compounds according to the present invention.

It is to be acknowledged that the above-mentioned advantages are not limited only to those cases where the target is actually related to a PTEN pathway. Rather this concept is generally applicable and beneficial in cases where a target is part of a tumor suppressor-related pathway is to be identified and/or validated and/or the target is a tumor suppressor. To identify the pathologically relevant effector molecules induced by loss of tumor suppressors function such as, e.g., PTEN mutation, it is critical that gene expression profiling experiments are performed under precisely controlled conditions. In this view experimental conditions are required that modulate the pathologically relevant pathway in a way that ensures the functional connection of the identified target genes and the tumor suppressor molecule (e.g. PTEN). This kind of experimental conditions may be realized by the methods according to the present invention.

The methods for the identification and/or validation of a target according to the present invention are superior to a further alternative known in the art to identify and/or validate a target molecule known as the so-called small molecules. By using said small molecules it is not possible to define and, more importantly to change the extent of the knockdown as these small molecules typically exhibit a certain - fixed - binding affinity to the tumor suppressor and to PTEN, respectively. Such a small molecule inhibitor known in the art is, e. g., LY294002. LY290004 (2-(4-morpholinyl)-8-phenylchromone) is one of several chromone derivative small molecule inhibitors developed by Lilly Research Laboratories (Indianapolis) as an inhibitor for PI 3-kinase (Vlahos et al. 1994, JBC 269, 5241-5248). It targets the catalytic subunit of the PI 3-kinase molecule, p110, and functions by competing with ATP binding in the catalytic center. In contrast to the invariable binding affinity of the small molecule inhibitor the use of antisense oligonucleotides allows for the adaptation of the binding affinity by modifying the nucleic acid, i.e. typically the mRNA binding part of the antisense oligonucleotide. This binding part or binding domain may actually be designed so as to hybridise only to a certain number of mRNAs thus allowing for a quantitatively controlled knockdown. This enables the further exploration of gene- and mRNA doses and of mRNA copy number effects, respectively, in connection with the particular pathway or target investigated.

Furthermore, LY290004 cannot distinguish between different isoforms of p110 (alpha, beta, gamma, delta), which are suggested to have different cellular functions. Also, the LY290004 is not entirely specific for p110 molecules in that it also inhibits other members of the family of PI 3-kinase homologs such as DNA-PK and the ATM gene products, which appear to function in DNA repair processes.

To summarize, the methods according to the present invention give a novel, specific and inartificial access to resolving the early events in pathways related to tumor suppressors. The functional oligonucleotides as disclosed and used according to the present invention are thus typically inhibitors of tumor suppressors.

As used herein expression system means any system where the effect of an mRNA, its presence, absence or destruction may actually be monitored or detected. The term "expression system" comprises insofar also any system which may be used for displaying or detecting the action of functional oligonucleotides as defined herein. Such expression system may generally be an in vivo or in vitro assay. The in vivo assay may comprise a cell, either a bacterial or an eucaryotic one, most preferably a mammalian cell, a tissue, an organ or a multicellular organism. Such multicellular organism may preferably be selected from the group comprising C. elegans, insects and mammals. The mammals in turn may be selected for the practice of the present invention from the group comprising mice, rats, rabbits, pigs, dogs, apes and humans.

As used herein, a functional oligonucleotide is said to be specific for a particular, i.e. targeted nucleic acid, such as, e.g., a tumor suppressor encoding mRNA or hnRNA if the functional oligonucleotide hybridises under standard transfection conditions such as described in example 1 herein, to said targeted nucleic acid and, at least to a certain degree, results in a decreased expression of the targeted nucleic acid. Such reduced expression may result from blocking the access of the translation machinery of the expression system such as the cellular machinery, or may be due to the RNase H activity directed to the mRNA/-antisense oligonucleotide-hybrid or any other mechanism.

In a preferred embodiment of the methods for the identification and/or validation of a target molecule according to the present invention the target which is to be identified and/or to be validated, is involved in the pathogenic mechanism of a diseases or a - pathologic - condition. The disease or condition is preferably that in which a tumor suppressor is either directly or indirectly involved, preferably whereby this pathogenic mechanism comprises a tumor suppressor related pathway.

In addition, all and any of the diseases where a tumor suppressor is actually involved may provide a target which is to be identified using the methods according to the present invention.

Other pathways and thus targets to be identified and validated using the methods according to the present invention may also be those involved in any biological processes. It is to be acknowledged that these processes may form part of some conditions or diseases. Insofar it is to be understood that any mechanism underlying the above-mentioned diseases and conditions may provide a biological process which may be targeted by the inventive methods and, vice versa, any of the biological mechanisms involving any tumor suppressors, such as the ones mentioned in the following, may be part of a disease or condition which may thus be investigated such as to identify and validate targets involved therein. The biological processes in which the target to be invalidated and/or validated may be involved are: proliferation, cell survival, migration, apoptosis, stress signalling, metastasis, anoikis, i. e. apoptosis induced upon cell detachment and signalling general processes

A further possibility to define the target besides whether it is related to a disease or a-pathogenic - condition or a distinct biological process as outlined above, is in terms of its chemical nature or its function in a system, regardless whether such system is an artificial system, an in vitro system or a biological system. Accordingly, the target may be an enzyme, preferably a kinase or a phosphatase, a transcription factor, a motility factor, a cell cycle factor, a cell cycle inhibitor or a tumor suppressor.

The methods according to the present invention may be related to tumor suppressor(s) either the way that the target itself is a tumor suppressor or that the pathway comprising the target is a tumor related pathway whereby the tumor suppressor or the pathway is preferably interacting in any of the conditions, diseases or biological processes mentioned herein. Various aspects of tumor suppressors are described, e. g., in Macleod, K. (Macleod, K.; supra). Tumor suppressors as used herein may be landscapers, gatekeepers or caretakers, although it is to be acknowledged that a particular tumor suppressor may fall into two or even all three of these categories.

The "gatekeeper" concept was initially proposed to explain the role of the adenomatous polyposis coli (APC) tumor suppressor gene which is invariably mutated early in colorectal tumorgenesis. Kienzler and Vogelstein (Kienzler, K. W.; Vogelstein, B.; Science 1996, 260: 1036 - 1037) qualified the "gatekeeper" definition of tumor suppressor to include all direct inhibitors of cell growth (suppression proliferation, inducing apoptosis or promoting differentiation). The "gatekeeper" class of tumor suppressor (genes) can be further subdefined as "initiation gatekeepers", "progression gatekeepers" or "metastasis gatekeepers".

In conclusion, "gatekeeper" tumor suppressors are best distinguished from so-called "caretakers" or "landscapers" by the fact that first, their loss of function is rate-limiting for a particular step in multi-stage tumorigenesis; second, they act directly to prevent tumor growth and third, restoring "gatekeeper" function to tumor cells suppresses neoplasia.

By contrast, "caretaker" tumor suppressors (genes) act indirectly to suppress tumor growth by ensuring the fidelity of the DNA code through effective repair of DNA damage or prevention of genomic instability (such as microsatellite or chromosome instability). Consequently, a large number of "caretaker" tumor suppressor genes are DNA repair genes, such as the "HNPCC genes" MSH2 and MLH1. Loss of "caretaker" function predisposes to cancer by increasing the DNA mutation rate, thereby increasing the chances that gatekeeper gene function will be lost. Restoration of the function of a "caretaker" gene would not stop tumor growth if mutation of a "gatekeeper" gene had already taken place.

The "landscaper" phenomenon was first described following analysis of the histology and mutations occurring in juvenile polyposis syndrome (JPS) wherein the initiating lesions appeared to occur in the stromal cells surrounding the tumor and not in the tumor cells themselves. Landscaper tumor suppressors were predicted to act by modulating the microenvironment in which tumor cells grow, perhaps by direct/indirect regulation of extracellular matrix proteins, cell surface markers, adhesion proteins or secreted growth/survival factors. Loss of function of such a "landscaper" tumor suppressor gene would cause the microenvironment to either function or grow aberrantly, promoting the neoplastic conversion of an adjacent epithelia. Consequently, the tumor would appear polyclonal for the mutation (Macleod, K.; supra).

It is within the skills of the one of the art that the inventive methods may be applied several times to the same system only changing the specifity of the expression modifying agent such as the functional oligonucleotide(s) actually used. By these changes different elements of the pathway may be addressed and the relationship between the various elements can thus actually be deduced from the overall readout of the expression system. The same functional oligonucleotide approach as described herein is preferably used in/as a further differentiating step, although other methods to identify/validate targets may also be used in such a differential expression system. Comparing and more particularly detecting the expression pattern of the expression system under the influence of a distinct functional oligonucleotide or any other means to identify/validate a target is well-known to the one skilled in the art. The methods and techniques required for this comprise RT-PCR (Sambrook, Fritsch, Maniatis, Molecular Cloning - A laboratory Manual, 2nd Ed. 1989, Cold Spring Harbor Laboratory Press), DNA-microchip-arrays (Schena, M et al. (1995) Quantitative monitoring of gene expression patterns with a complementary DNA microarray, Science 270, 467-470) and Western blot analysis (Sambrook et al., supra).

The methods for the identification and validation of a target wherein the target is part of a tumor suppressor related pathway as disclosed herein are factually applicable to any tumor suppressor. Tumor suppressors as such are known in the art. Some preferred tumor suppressors are p53, Smad3, SHIP2, and PTEN. p53 is, e.g. described in Balint E E, Vousden KH. Activation and activities of the p53 tumour suppressor protein. Br J Cancer. 2001 Dec;85(12): 1813-1823. The p53 tumour suppressor protein inhibits malignant progression by mediating cell cycle arrest, apoptosis or repair following cellular stress. One of the major regulators of p53 function is the MDM2 protein, and multiple forms of cellular stress activate p53 by inhibiting the MDM2-mediated degradation of p53. Mutations in p53, or disruption of the pathways that allow activation of p53, seem to be a general feature of all cancers. Balint et al. review recent advances in the understanding of the pathways that regulate p53 and the pathways that are induced by p53, as well as their implications for cancer therapy.

Smad3 is, e.g., described in Weinstein M, Yang X, Deng C. Protein Functions of mammalian genes as revealed by targeted gene disruption in mice. Cytokine Growth Factor Rev. 2000 Mar-Jun;11(1-2):49-58. The Smad genes are the intracellular mediators of TGF-beta signals.

Targeted mutagenesis in mice has yielded valuable new insights into the functions of this important gene family. These experiments have shown that Smad2 and Smad4 are needed for gastrulation, Smad5 for angiogenesis, and Smad3 for establishment of the mucosal immune response and proper development of the skeleton. In addition, these experiments have shown the importance of gene dosage in this family, as several of its members yielded haploinsufficiency phenotypes. These include gastrulation and craniofacial defects for Smad2, accelerated wound healing for Smad3, and the incidence of gastric cancer for Smad4. Combinatorial genetics has also revealed functions of Smads in left/right isomerism and liver development.

SHIP2 is, e.g. , described in Huber M, Helgason CD, Damen JE, Scheid M, Duronio V, Liu L, Ware MD, Humphries RK, Krystal G. The role of SHIP in growth factor induced signalling. Prog Biophys Mol Biol. 1999;71(3-4): 423-34. The recently cloned, hemopoietic-specific, src homology 2 (SH2)-containing inositol phosphatase, SHIP, is rapidly gaining prominence as a potential regulator of all phosphatidylinositol (PI)-3 kinase mediated events since it has been shown both in vitro and in vivo to hydrolyze the 5' phosphate from phosphatidylinositol-3,4,5-trisphosphate (PI-3,4,5-P3). Thus SHIP, and its more widely expressed counterpart, SHIP2, could play a central role in determining PI-3,4,5-P3 and PI-3,4-P2 levels in many cell types.

The methods according to the present invention are also applicable to upstream or downstream effectors of a tumor related pathway. Such upstream effectors may be growth factors and cytokines, respectively. Growth factors and cytokines which may be addressed by the methods according to the present invention include but are not limited to EGF, VEGF, PDGF, FGF and TGFbeta. Other upstream effectors are insulin, IGF, CSF, IL-2, IL-3, IL-4, IL-6 and IL-7.

EGF is, e.g., described in Prenzel N, Fischer OM, Streit S, Hart S, Ullrich A. The epidermal growth factor receptor family as a central element for cellular signal transduction and diversification. Endocr Relat Cancer. 2001 Mar; 8(1):11-31. Homeostasis of multicellular organisms is critically dependent on the correct interpretation of the plethora of signals which cells are exposed to during their lifespan. Various soluble factors regulate the activation state of cellular receptors which are coupled to a complex signal transduction network that ultimately generates signals defining the required biological response. The epidermal growth factor receptor (EGFR) family of receptor tyrosine kinases represents both key regulators of normal cellular development as well as critical players in a variety of pathophysiological phenomena. Since the EGFR and HER2 were recently identified as critical players in the transduction of signals by a variety of cell surface receptors, such as G-protein-coupled receptors and integrins, a present special focus is the mechanisms and significance of the interconnectivity between heterologous signalling systems.

VEGF is, e.g., described in Connolly DT. Vascular permeability factor: a unique regulator of blood vessel function. J Cell Biochem. 1991 Nov;47(3):219-23. Vascular permeability factor (VPF), also known as vascular endothelial growth factor (VEGF), is a potent polypeptide regulator of blood vessel function. VPF promotes an array of responses in endothelium, including hyperpermeability, endothelial cell growth, angiogenesis, and enhanced glucose transport. VPF regulates the expression of tissue factor and the glucose transporter. All of the endothelial cell responses to VPF are evidently mediated by high affinity cell surface receptors. Thus, endothelial cells have a unique and specific spectrum of responses to VPF. Since each of the responses of endothelial cells to VPF are also elicited by agonists, such as bFGF, TNF, histamine and others, it remains a major challenge to determine how post-receptor signalling pathways maintain both specificity and redundancy in cellular responses to various agonists.

PDGF is, e.g., described in Westermark B, Heldin CH, Nister M. Platelet-derived growth factor in human glioma. Glia. 1995 Nov;15(3):257-63. Platelet-derived growth factor (PDGF) is a 30 kDa protein consisting of disulfide-bonded dimers of A- and B-chains. PDGF receptors are of two types, alpha- and beta-receptors, which are members of the proteintyrosine kinase family of receptors. The receptors are activated by ligand-induced dimerization, whereby the receptors become phosphorylated on tyrosine residues. These form attachment sites for signalling molecules, which inter alia activate the Ras.Raf pathway. PDGF has important functions in development and is required for a proper timing of oligodendrocyte differentiation. The v-sis oncogene of simian sarcoma virus (SSV) is a retroviral homolog of the B-chain gene, and induces transformation by an autocrine activation of PDGF receptors at the cell surface. SSV induces malignant glioma in experimental animals, suggesting a role for autocrine PDGF in glioma development. PDGF and PDGF receptors are frequently coexpressed in human glioma cell lines. Specific and nonspecific PDGF antagonists block the growth of some glioma cell lines in vitro and in vivo, suggesting that autocrine PDGF is involved in transformation and tumorigenesis. In situ studies of human gliomas show overexpression of alpha-receptors in glioma cells of high-grade tumors. In a few cases, overexpression is caused by receptor amplification. Since high-grade glioma cells also express the PDGF A-chain, an autocrine activation of the alpha-receptor may drive the proliferation of glioma cells in vivo.

PDGF is also described by Khachigian LM, Chesterman CN. Platelet-derived growth factor and alternative splicing: a review. Pathology. 1992 Oct;24(4):280-90. According to Khachigian et al. the mitogenic and chemotactic potency of platelet-derived growth factor (PDGF) has linked this polypeptide to the pathogenesis of several disease states including atherosclerosis and neoplasia. In addition to platelets, several normal and tumor cells secrete the mitogen in one or more of three possible dimeric configurations. Alternative splicing of exon 6 in PDGF A-chain RNA results in the formation of two protein species with different arboxy-termini. Initially, it was thought that the longer A-chain variant was processed only by transformed cells. However, recent evidence indicates that alternative splicing occurs in several cells which express the A-chain, including early Xenopus embryos. The functional significance of the exon 6 product, a highly basic region spanned by 18 amino acid residues (A194-211), is not precisely clear. Recent findings are summarized which implicate roles for A194-211 in the processing, secretion, and mitogenesis of the A-chain homodimer, nuclear transport signalling, and heparin binding. Thus, alternative splicing could play an important role in the modulation of the functional properties of the PDGF A-chain variants per se and in the complex interactive network of polypeptide growth factors and cytokines.

FGF is, e.g., described in Dickson C, Spencer-Dene B, Dillon C, Fantl V. Tyrosine kinase signalling in breast cancer: fibroblast growth factors and their receptors. Breast Cancer Res. 2000;2(3):191-6. The fibroblast growth factors [Fgfs (murine), FGFs (human)] constitute a large family of ligands that signal through a class of cell-surface tyrosine kinase receptors. Fgf signalling has been associated in vitro with cellular differentiation as well as mitogenic and motogenic responses. In vivo, Fgfs are critical for animal development, and some have potent angiogenic properties. Several Fgfs have been identified as oncogenes in murine mammary cancer, where their deregulation is associated with proviral insertions of the mouse mammary tumour virus (MMTV). Thus, in some mammary tumours of MMTV-infected mouse strains, integration of viral genomic DNA into the somatic DNA of mammary epithelial cells was found to have caused the inappropriate expression of members of this family of growth factors. Although examination of human breast cancers has shown an altered expression of FGFs or of their receptors in some tumours, their role in

EP 1 325 955 A1

the causation of breast disease is unclear and remains controversial.

TGFbeta is, e.g. described in Topper JN. TGF-beta in the cardiovascular system: molecular mechanisms of a context-specific growth factor. Trends Cardiovasc Med. 2000 Apr;10(3):132-7. Review. Transforming growth factor beta-1 is the prototypical member of a class of growth factors whose actions have been strongly implicated in a number of pathophysiologic processes including chronic vascular diseases such as atherosclerosis and hypertension. One of the hall-marks of this class of growth factors is the diverse nature of their actions; a characteristic that is thought to arise from the fact that the effects of these factors are very dependent upon the particular cellular context in which they operate. There has been substantial progress in understanding the molecular signalling mechanisms utilized by these factors. These findings are beginning to provide a mechanistic framework with which to understand the complex and pleiotropic actions of these factors on cells and tissues of the cardiovascular system.

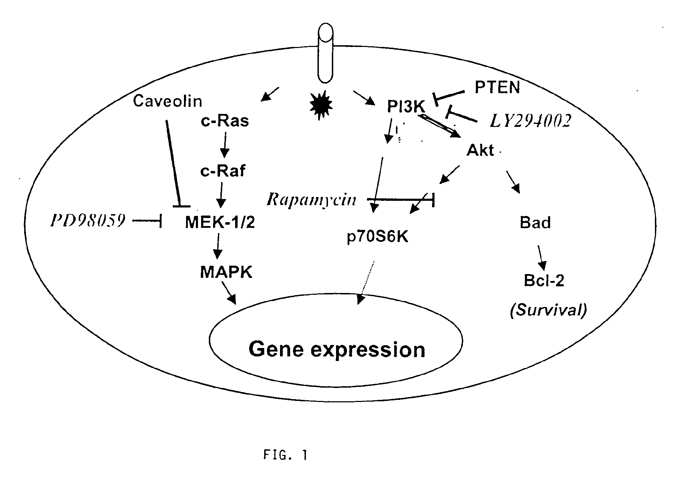

PTEN is another tumor suppressor which is involved in the phosphatidylinositol (PI) 3-kinase pathway which has been extensively studied in the past for its role in regulating cell growth and transformation (for reviews see, Stein, R. C. and Waterfield, M. D. (2000). PI3-kinase inhibition: a target for drug development? Mol Med Today 6, 347-357; Vazquez, F. and Sellers, W. R. (2000). The PTEN tumor suppressor protein: an antagonist of phosphoinositide 3-kinase signaling. Biochim Biophys Acta 1470, M21-35; Roymans, D. and Slegers, H. (2001). Phosphatidylinositol 3-kinases in tumor progression. Eur J Biochem 268, 487-498). The tumor suppressor PTEN functions as a negative regulator of PI 3-kinase by reversing the PI 3-kinase-catalyzed reaction and thereby ensures that activation of the pathway occurs in a transient and controlled fashion (Fig. 1).

A chronic activation of the PI 3-kinase pathway through loss of PTEN function is a major contributor to tumorigenesis and metastasis indicating that this tumor suppressor represents an important checkpoint for a controlled cell proliferation. PTEN knock out cells show similar characteristics as cells in which the PI 3-kinase pathway has been chronically induced via activated forms of PI 3-kinase (Di Cristofano, A., Pesce, B., Cordon-Cardo, C. and Pandolfi, P. P. (1998). Pten is essential for embryonic development and tumour suppression. Nat Genet 19, 348-355. Klippel, A., Escobedo, M. A., Wachowicz, M. S., Apell, G., Brown, T. W., Giedlin, M. A., Kavanaugh, W. M. and Williams, L. T. (1998). Activation of phosphatidylinositol 3-kinase is sufficient for cell cycle entry and promotes cellular changes characteristic of oncogenic transformation. Mol Cell Biol 18, 5699-5711. Kobayashi, M., Nagata, S., Iwasaki, T., Yanagihara, K., Saitoh, I., Karouji, Y., Ihara, S. and Fukui, Y. (1999). Dedifferentiation of adenocarcinomas by activation of phosphatidylinositol 3-kinase. Proc Natl Acad Sci USA 96, 4874-4879.

The use of the methods as disclosed herein for inhibiting the tumor suppressor PTEN allows thus to overcome the limitations arising from the use of knockout models. PTEN knock out mice generated by several laboratories are not viable and die in utero ( Di Cristofano, A., Pesce, B., Cordon-Cardo, C. and Pandolfi, P. P. (1998). Pten is essential for embryonic development and tumour suppression. Nat Genet 19, 348-355; Suzuki, A., de la Pompa, J. L., Stambolic, V., Elia, A. J., Sasaki, T., del Barco Barrantes, I., Ho, A., Wakeham, A., Itie, A., Khoo, W., Fukumoto, M. and Mak, T. W. (1998). High cancer susceptibility and embryonic lethality associated with mutation of the PTEN tumor suppressor gene in mice. Curr Biol 8, 1169-1178.; Podsypanina, K., Ellenson, L. H., Nemes, A., Gu, J., Tamura, M., Yamada, K. M., Cordon-Cardo, C., Catoretti, G., Fisher, P. E. and Parsons, R. (1999). Mutation of Pten/Mmacl in mice causes neoplasia in multiple organ systems. Proc Natl Acad Sci USA 96, 1563-1568.). Hemizygous knock out (PTEN+/-) mice which are difficult to generate and do not allow for a higher degree of knockdown of the compound in question, are viable and develop tumors in various organs. The fact that these mice having half the regular level of PTEN protein exhibit a high susceptibility for developing tumors suggests that inhibition of PTEN expression of 50% or more as possible using the methods as disclosed herein, will cause a strong activation of the PI 3-kinase signaling pathway resulting in enhanced metastatic growth potential. This induced increase in metastatic behavior then allows the detailed analysis of the underlying molecular mechanisms.

Also, a considerable subset of human cancers has a high incidence for loss of PTEN function, especially in late stage tumors ( Cantley, L. C. and Neel, B. G. (1999). New insights into tumor suppression: PTEN suppresses tumor formation by restraining the phosphoinositide 3-kinase/AKT pathway. Proc Natl Acad Sci USA 96, 4240-4245; Ali, I. U. (2000). Gatekeeper for endometrium: the PTEN tumor suppressor gene. J Natl Cancer Inst 92, 861-863). Loss of PTEN correlates with increased aggressive and invasive behavior of the respective tumor cells. Using the methods according to the present invention it is possible to mimic the loss of PTEN function in its early cellular consequences by inhibiting gene expression in an induced fashion. Additionally, the methods according to the present invention allow for the identification and validation of drug targets and/or diagnostic markers that are specific for the diagnosis and/or treatment of patients with PTEN negative cancers (and/or other tumor suppressors).

Another advantage of the methods according to the present invention is to allow the identification and/or validation of a target which is linked to the metastatic effects involving or relating to loss of PTEN function. A subset of these downstream targets (representing effectors) of the PI 3-kinase/PTEN pathway are likely to represent key regulatory molecules responsible for mediating the metastatic phenotype of cells that have lost PTEN function. It is important to target this particular fraction of effector molecules selectively, because targets which act on a parallel branch or further upstream in this signalling cascade are likely to cause unwanted effects. This is due to the fact that the PI 3-kinase/PTEN pathway not only regulates cell proliferation and survival, but also processes such as cell migration, intracellular trafficking and insulin signalling. Therefore, it is important to select the downstream effectors which specifically act in the proliferative arm of responses, but not e.g. in insulin signalling. Inhibition of insulin signalling is likely to induce unwanted diabetic responses or other side effects. A successful target will be required for invasive cell growth, therefore the inhibition of its expression should interfere with invasive cell growth, but not inhibit other responses mediated by PI 3-kinase.

As mentioned above PTEN is involved in several pathways which are also referred to as PTEN related pathways such as the PI3K/PTEN pathway, the Akt pathway, the EGF-related autocrine loop and the mTOR pathway.

A PTEN related pathway is factually any pathway which involves PTEN, either directly or indirectly. PTEN may act either as an inhibitor or as an activator in such a pathway, or it may as such be regulated by other elements of a pathway. The same definition applies accordingly to any tumor suppressor related pathways or Akt related pathways. Hence, a tumor suppressor related pathway is any pathway which involves whichever tumor suppressor, either directly or indirectly. Said tumor suppressor may act in such a pathway either as a regulator such as an inhibitor or an activator, or it may as such be regulated by other elements of the pathway.

There is ample of prior art describing diseases and conditions involving PTEN and are thus deemed as PTEN related pathways in the meaning of this description. Any of these conditions and diseases may thus be addressed by the inventive methods. For reasons of illustration but not limitation it is referred to the following: endometrial cancer, colorectal carcinomas, gliomas, endometrial cancers, adenocarcinomas, endometrial hyperplasias, Cowden's syndrome, hereditary non-polyposis colorectal carcinoma, Li-Fraumene's syndrome, breast-ovarian cancer, prostate cancer (Ali, I. U., Journal of the National Cancer Institute, Vol. 92, no. 11, June 07, 2000, page 861 - 863), Bannayan-Zonana syndrome, LDD (Lhermitte-Duklos' syndrome) (Macleod, K., supra) hamartoma-macrocephaly diseases including Cow disease (CD) and Bannayan-Ruvalcaba-Rily syndrome (BRR), mucocutaneous lesions (e. g. trichilemmonmas), macrocephaly, mental retardation, gastrointestinal harmatomas, lipomas, thyroid adenomas, fibrocystic disease of the breast, cerebellar dysplastic gangliocytoma and breast and thyroid malignancies (Vazquez, F., Sellers, W. R., supra).

Akt is a downstream target of PI-3K activation and actually represents a family of serine-threonine kinases. This family consists of three isoforms, namely Akt-1, -2 and -3. Akt-1 was initially identified as the cellular homologue of the retroviral oncogene v-Akt. Akt proteins contain the so-called "pleckstrin homology domain" (PH domain) at their amino terminus. PH domains are a conserved protein-lipid interaction domain that can be found in a wide variety of proteins. The PH domain of Akt can bind with high affinity to PI 3,4) $P_2$ and PI(3,4,5)$P_3$, resulting in translocation of Akt from the cytosol to the plasma membrane and a conformational change in Akt. When activated, Akt phosphorylates proteins on serine and threonine residues. The majority of these phosphorylations render the target substrates inactive. Akt seems to potentiate cell survival in a number of systems by inhibiting substrates such as BAD, Caspase 9, FKHR and FKHRL 1.

The epidermal growth factor (EGF) receptor autocrine loop is frequently induced in a variety of human tumors and has been linked to invasive cell growth and transformation. This induction is caused by up-regulation of growth factors of the EGF family in these tumor cells which in turn bind and activate EGF receptor molecules. The autocrine production of these factors causes a chronic activation of the pathway and its signalling responsee (Yarden Y. and Slikowski M.X. 2001, Nature Reviews Molecular Cell Biology 2, 127-137).

mTOR (mammalian Target Of Rapamycin), also known as Raft or FRAP, is acting downstream of PI 3-kinase to regulate processes such as the pp70 S6 kinase dependent entry into the cell cycle. mTOR acts as a sensor for growth factor and nutrient availability to control translation through activating pp70 S6 kinase and initiation factor 4E. mTOR function is inhibited by the bacterial macrolide rapamycin which blocks growth of T-cells and certain tumor cells (Kuruvilla and Schreiber 1999, Chemistry & Biology 6, R129-R136).

It is within the present invention that all of the particular advantages, embodiments and conditions recited in connection with any specific tumor suppressor are also applicable for any other tumor suppressor. This applies also to the particular groups of patients whose condition may be mimicked by using the methods according to the present invention and thus provide an appropriate model for an effective target identification and/or validation process.

The methods according to the present invention are also useful in so far as they allow for a transient knockdown of tumor suppressors and thus allow for mimicking the early stages after loss of such tumor suppressors and for resolving the time course of the generation of a tumor and other diseases related to the tumor suppressor. In doing so the direct as well as indirect molecular changes may be identified and studied which have never been accessible by earlier methods which only analysed and compared the various endpoints. The approach realized by the practicing of the methods according to the present invention is unbiased and does not select for compensatory or clonal effects or induce chromosomal instabilities since it is induced and the resulting changes are transient. Therefore, it does not have the typical problems of endpoint studies as outlined above in detail. This allows for the identification of a defined subset of direct and indirect downstream effectors specific for PTEN or other tumor suppressor deficient tumors. The direct effectors are likely to represent the most relevant target molecules, since they act in the initial phase of the changes induced. The indirect secondary and tertiary effectors will comprise of targets as well as diagnostic markers which represent more the resulting rather than the causative molecular changes responsible for the phenotypic changes. In summary these downstream effector molecules will consist of diagnostic markers specific for the respective class of tumors and of molecules that represent targets for specialized drug development for a defined patient population classified by the loss of tumor suppressor function. A drug therapy which selectively targets a defined class of tumors will result in fewer side effects and allow for a more effective treatment of a given patient population. The above described molecular changes may be related to downstream effectors as well as upstream effectors or any loops linked thereto such as, e.g., autocrine loops. An example for this kind of autocrine loop is the epidermal growth factor (EGF) receptor autocrine loop which is also linked to the PTEN pathway.

The possibility to mimic either early stages of tumor suppressor loss and/or a distinct group of patients (e.g. tumor suppressor deficient patients) provides for a diagnosis and therapy specific for the particular tumors and patients, respectively.

It is also within the present invention that various tumor suppressors are targeted by the functional oligonucleotides. This allows for an even more accurate resolution of the molecular mechanisms compared to the use of standard techniques such as small molecule inhibitors like LY 294002 used for knocking out PTEN. By knocking down several tumor suppressors using various functional polynucleotides in combination it is possible to mimic what is going on in, for example, more advanced invasive tumors where one checkpoint after another is lost such as PTEN/Smad2 or 3 or 4; PTEN/p16; PTEN/SHIP-2

It is to be understood that, starting from the functional oligonucleotide(s) proven effective in a method according to the present invention, it is possible to further modify these functional oligonucleotides. This modification may be related to the primary sequence, i.e. the sequence specificity of the functional oligonucleotide hybridising to the target nucleic acid. By performing this modification the discriminatory capacity or capability of the functional oligonucleotide may be both either increased or decreased. This then allows for either an increase of the specificity or for a reduction of the specificity of the functional olgonucleotide and thus also for a reduction of the extent to which the functional oligonucleotide targeted nucleic acid is actually blocked for transcription or even degraded upon RNase H activity or by any other mechanism. If the functional oligonucleotide is a chimeric one as described above such modifications may be related to the length of the DNA or the RNA part thereof. Additionally, the kind of linkage connecting the various nucleotides of the functional oligonucleotide as well as the 3' of 5' end of it may be modified.

As far as the invention is related to a method for screening of a candidate compound library, the library used for such purpose may consist of naturally occurring compounds or synthetic compounds or any combination of both of them. The number of elements contained in such library is not critical to the practice of said screening method and may be as little as one to several thousands or even million of elements. A particularly advantageous library is a combinatorial library. Typically the screening method is in the format of a high throughput system. The expression system may actually be any of the expression systems described herein although it has to be acknowledged that an in vitro assay is most suitable, particularly in view of the envisaged screening format as high throughput system. In principle, it is possible to add more than one candidate compound to the expression system and to analyse the reaction of the expression system either as a whole or after addition of the single candidate compound. Typically, candidate compounds are tested in succession in the screening system. Due to the addition of the functional oligonucleotide to the expression system the molecule against which the functional oligonucleotide is directed, is actually decreased in its concentration or its bioavailability. Due to this reduced presence or activity its effect on the pathway is no longer exerted so that, for example in the case of PTEN, there is no longer an inhibition on PI3K activity resulting in the up and downregulation, respectively, of other compounds or elements of the pathway in which the tumor suppressor is involved, which would normally not be absent or present, respectively. Therefore, target compounds possibly present in the expression system and thus accessible to the screening process to which they are normally not accessible, are detected by this particular approach. In fact, a prerequisite for targeting all possible candidates or for discriminating one or several of them maybe accomplished due to this changed balance in the components or elements of the pathway.

The present invention is further illustrated by the figures and examples, wherein

Fig. 1     shows some metabolic pathways leading to a change in gene expression and inhibitors used for that purpose as well as a pathway resulting in survival of the cell all starting from the binding of a growth factor (e.g. PDGF, EGF, insulin).

Fig. 2     shows the regulation of PI 3-kinase activity;

Fig. 3     shows a Western blot analysis using different antisense oligonucleotides and monitoring the generation of P-Akt;

Fig. 4     shows a Western blot analysis investigating the impact of changes in the mRNA binding part of the antisense oligonucleotide;

Fig. 5     shows a Western blot analysis of an experiment where two different antisense oligonucleotides were used together with further compounds, namely DMSO (D), Ly294002 (Ly) and PD98059 (PD) being an inhibitor for MEK-1/2 kinase;

Fig. 6     shows a Western blot analysis of an experiment wherein different human cell lines (Hela and PC-3) were exposed to antisense oligonucleotide PTEN 53 and a control antisense oligonucleotide (GBC);

Fig. 7 A     shows a flow diagram illustrating the pathway leading to apoptosis; and

Fig. 7 B     shows a Western blot analysis of an experiment designed to monitor the impact of PTEN directed antisense oligonucleotides on UV-induced apoptosis;

Fig. 8 A     shows basically the same metabolic pathway as displayed in Fig. 7 A; and

Fig. 8 B     shows the Western blot analysis of an experiment designed to monitor the impact of PTEN directed antisense oligonucleotides TNF induced apoptosis;

Fig. 9     shows an overview of signalling and phenotypic changes induced after tumor suppression knocked down by PTEN directed antisense oligonucleotides, and more particularly,

Fig. 9 A     shows the schematic pathway involving PDGF-R with PTEN inhibiting PI-3K activity;

Fig. 9 B     shows the immunoblot analysis; and

Fig. 9 C     shows the phenotypic analysis;

Fig. 10 A     shows a Western immunoblot analysis comparing the effect of PTEN 48 using chemically different antisense oligonucleotides;

Fig. 10B     shows micrographs of cells treated with said different antisense oligonucleotides;

Fig. 11A     shows the incorporation of BrdU by cells treated with various antisense molecules as a measure for the entry into the S phase;

Fig. 11B     shows the ratio of PTEN/actin RNA in transfected cells treated with various antisense molecules as a measure for the specific inhibition of mRNA expression;

Fig. 12     shows the Western Blot analysis result of an experiment where the impact of antisense molecule induced inhibition of PTEN expression on UV-induced apoptosis was studied;

Fig. 13     shows the stimulated growth of HeLa cells on matrigel upon antisense molecule knock down of PTEN expression;

Fig. 14     shows the Western Blot analysis result of an experiment where the impact of antisense molecules interfering with p110 expression on signalling induced by endogenous or recombinant PI 3-kinase;

Fig. 15     shows the positions of antisense molecules relative to intron-exon boundaries of JAK-1 mRNA;

Fig. 16     shows JAK-1 mRNA knock down relative to actin mRNA;

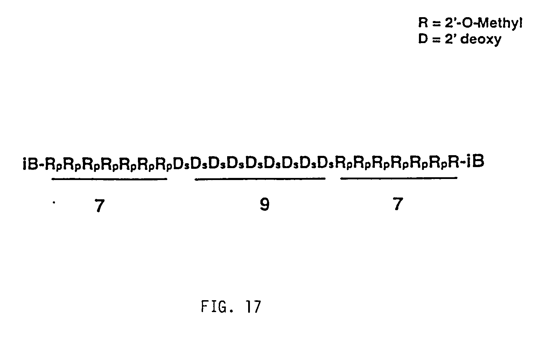

Fig. 17     shows the basic structure of the third generation antisense molecule;

Fig. 18     shows a Western Blot analysis result of an experiment where the impact of RNAi specific for PTEN on PTEN expression was compared to the impact of an antisense oligonucleotide specific for PTEN on PTEN expression;

Fig. 19    shows the dose response of RNAi specific for PTEN;

Fig. 20    shows the Western Blot analysis result of the functional knock down of tumor suppressor Smad3 by antisense oligonulceotides modulating signal transduction;

Fig. 21    shows the Western Blot analysis result of the functional knock down of tumor suppressor p16INK4a by antisense oligonulceotides modulating signal transduction; and

Fig. 22    shows the Western Blot analysis result of the functional knock down of tumor suppressor SHIP2 by antisense oligonulceotides modulating signal transduction;

Fig. 1 shows some metabolic pathways leading to a change in gene expression and inhibitors used for that purpose as well as a pathway, all starting from the binding of a growth factor resulting in survival of the cells. The pathways illustrated may be grouped either with regard to the final biological process (gene expression) or by having a common key molecule (PIK3 and Akt, respectively). Gene expression may be modified via the cRas pathway which may be inhibited by the use of Caveolin and PD98059 both acting on MEK-1/2, an important kinase in the cRAS signalling pathway. An alternative pathway resulting in a change of gene expression involves PI-3K. The PI-3K pathway splits up into two branches one involving Akt, the other one not involving Akt. However, both pathways pass through p70S6K which may be inhibited by rapamycin. Akt itself is also involved in a different pathway related to the survival of the cell via Bad and Bcl-2. It can be taken from Fig. 1 that PTEN is actually an inhibitor reducing PI3 kinase activity in an expression system. LY 294002 is a small molecule also acting as an inhibitor to PI3K.

As mentioned above, PTEN may be targeted by the functional oligonucleotides described herein, i. e. by antisense oligonucleotides, ribozymes and/or RNAi. By inhibiting the inhibitory effect of PTEN the elements of the pathway where PTEN is involved, may be either up regulated or down regulated so that upon differential display of the respective compounds conclusions can be drawn on further targets involved in the respective pathway.

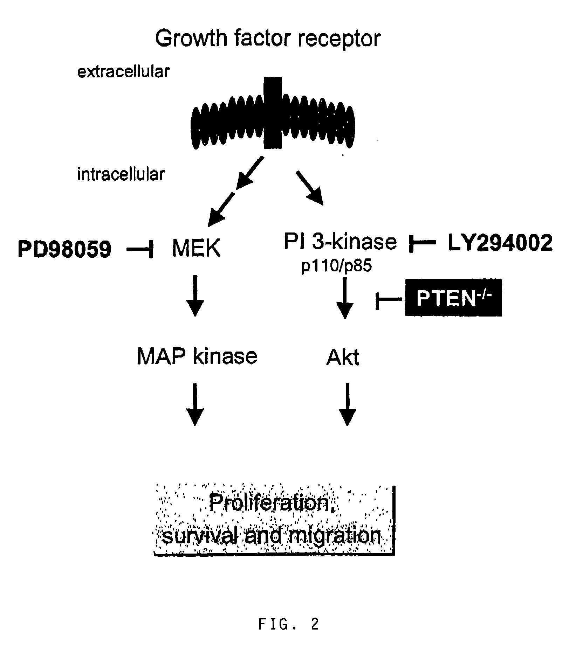

Figure 2 shows the regulation of PI 3-kinase activity upon growth factor induction and a parallel signaling pathway. Growth factor stimulation of cells leads to activation of their cognate receptors at the cell membrane which in turn associate with and activate intracellular signaling molecules such as PI 3-kinase. Activation of PI 3-kinase (consisting of a regulatory p85 and a catalytic p110 subunit) results in activation of Akt by phosphorylation, thereby supporting cellular responses such as proliferation, survival or migration further downstream. PTEN interferes with PI 3-kinase mediated downstream responses and ensures that activation of the pathway occurs transiently. Chronic hyperactivation of PI 3-kinase signaling is caused by functional inactivation of PTEN. PI 3-kinase activity can be blocked by addition of the small molecule inhibitor LY294002. The activity and downstream responses of the signaling kinase MEK which acts in a parallel pathway, can be inhibited by the small molecule inhibitor PD98059.

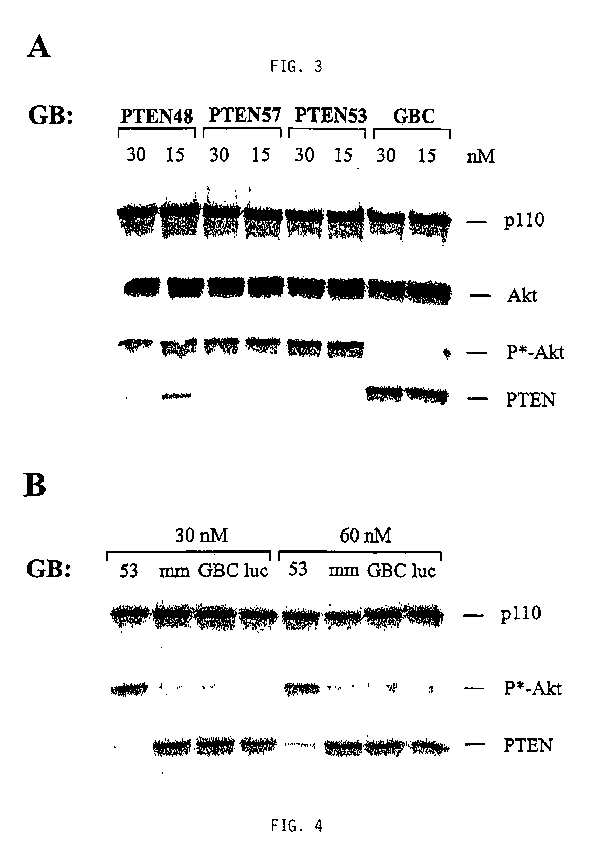

Fig. 3 shows a Western blot analysis of an experiment corresponding to the method according to the present invention. A total of three different antisense oligonucleotides, also referred to as geneblocs herein, namely PTEN 48, PTEN 57 and PTEN 53 were transfected into ratembryo fibroblast cell line together with a negative control comprising a randomised sequence (also referred to herein as GBC). More particularly, 3Y1 cells on 10 cm plates were transfected with 15 or 30 nM of the three different PTEN specific Geneblocs 48, 57 and 53 and with GBC. After 48 h cells were harvested and the cell extracts were analyzed by immunoblotting for the relative protein amounts of p110, Akt, phosphorylated Akt (P*-Akt) and PTEN as indicated on the right. All three antisense oligonucleotides were active such that PTEN was actually no longer present after administration of the antisense oligonucleotides to the expression system. Due to the lack of the inhibitor to PI3K, namely PTEN, the unopposed PI-3K activity resulted in phosphorylation of Akt. Accordingly, phosphorylated Akt could be detected upon usage of all three antisense oligonucleotides whereas the negative control (GBC) did not result in generation of phosphorylated Akt. As expected, under these control conditions the PTEN mRNA was translated into the active PTEN inhibiting PI-3K activity and thus preventing generation of phosphorylated Akt. Fig. 3 also shows that all three antisense oligonucleotides were active. The concentration dependency of the antisense oligonucleotides can be particularly well seen from the use of PTEN 57 and PTEN 53 and was confirmed by further experiments. The most powerful antisense oligonucleotide among said three antisense oligonucleotides was PTEN 53. P110 was used as a marker to reflect the loading of each of the lanes.

Fig. 4 shows a Western blot analysis investigating the impact of changes in the mRNA binding part of the antisense oligonucleotide. In the corresponding experiment PTEN 53 was used as the antisense oligonucleotide and a total of four mismatches were introduced to the particular sequence with the total length of PTEN 57 being 23 nucleotides. Again, GBC was used as a negative control. The sequence of the mismatch oligonucleotide is BucucauuT$_s$T$_s$C$_s$T$_s$T$_s$T$_s$G$_s$T$_s$G$_s$scucacgaB (SEQ ID No. 33) with B representing an inverted abasic nucleotide. In addition an antisense oligonucleotide was prepared specific for the mRNA of luciferase inhibiting luciferase expression in cells engineered to express the recombinant firefly gene. The respective lane on the Western blot is designated as "luc". The same experiment was carried out using either 30 nM or 60 nM antisense oligonucleotide. As mentioned in connection with Fig. 3, P110 serves as an internal loading standard. It can be clearly taken from the Western Blot that only the addition of PTEN 53 (designated in lanes 1 and 4 as "53") resulted in the phosphorylation of Akt, designated as P*-Akt in Fig. 4. The fact that the mismatch control (lanes 2 and 5) neither resulted in phosphorylation of Akt nor in degradation of PTEN mRNA confirmed the specificity of the system used thus excluding any cross reactivity with similar sequences.

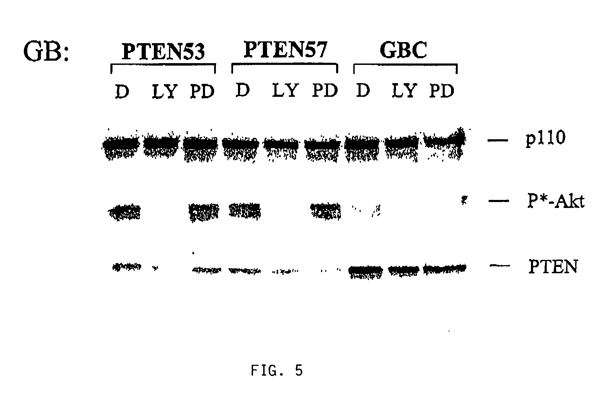

Fig. 5 shows a Western blot demonstrating that Akt-phopshorylation induced by PTEN knockdown is still dependent on PI3-kinase. In this experiment two different antisense oligonucleotides (PTEN 53 and PTEN 57) were used together with further compounds, namely DMSO (D), Ly294002 (Ly) and PD98059 (PD) being an inhibitor for MEK-1/2 kinase. More particularly, the first test was carried out using the antisense oligonucleotide PTEN 53 in combination either with DMSO, LY or PD. The same experiment was carried out using PTEN 57 also directed against PTEN mRNA, and using GBC as defined above as a control antisense oligonucleotide. More particularly, 3Y1 cells on 10 cm plates were transfected in triplicates with 30 nM of the GeneBlocs PTEN 53, 57 or with GBC for 48 h. 30 min before lysis the cells were treated with 10 µM PI 3-kinase inhibitor LY294002 (LY), 50 µM MEK inhibitor PD98059 (PD) or with the vehicle DMSO (D). The positions of p110, phosphorylated Akt (P*-Akt) and PTEN are indicated on the right. PD98059 mediated inhibition of MAP kinase phosphorylation by MEK was confirmed in parallel (not shown).The present data entails that the Akt phosphorylation induced by knockdown of PTEN expression is dependent on PI3-kinase activation.

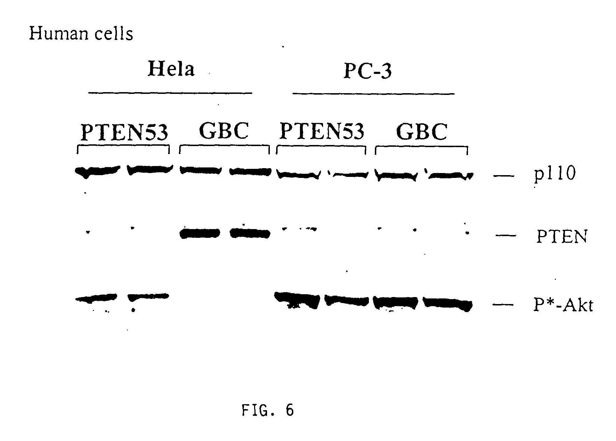

Fig. 6 shows a Western blot analysis of an experiment wherein different human cell lines (Hela and PC-3) were exposed to antisense oligonucleotide PTEN 53 and to a control antisense oligonucleotide (GBC). More particularly, HeLa cells (380.000 cells/plate) and PC-3 cells (450.000 cells/plate) were transfected in parallel on 10 cm plates. Each cell line was treated for 48 h in duplicate samples with 30 nM of PTEN GeneBloc 53 or GBC. The cell extracts were analyzed by immunoblotting for the relative amounts of p110, PTEN and phosphorylated Akt (P*-Akt) as indicated on the right. The band which migrates just above the PTEN signal was caused by unspecific reaction of the secondary antibody. It can be taken from Fig. 6 that in case of Hela cells which are PTEN positive, administration of PTEN 53 as an antisense oligonucleotide specific for PTEN mRNA, results in the degradation of PTEN mRNA and in the generation of phosphorylated Akt due to the prevailing PI3K activity. In contrast to this, PC-3 cells which are PTEN negative, generate phosphorylated Akt irrespective to whether the specific antisense oligonucleotide PTEN 53 or the control antisense oligonucleotide (GBC) is used. The level of Akt phosphorylation induced after knock down of PTEN expression in HeLa cells is comparable to the chronically high level in PTEN-deficient PC-3 cells.

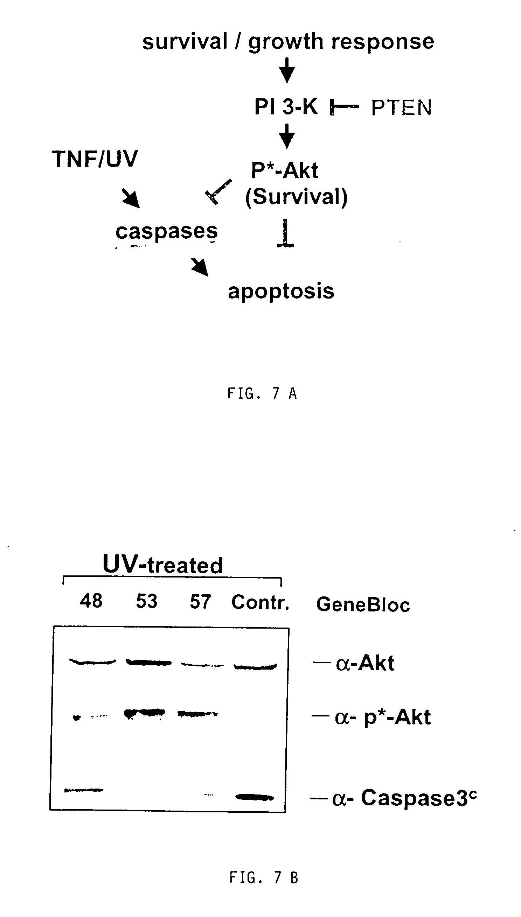

Fig. 7A shows a schematic diagram indicating that PTEN knockdown activates the cell's survival pathway. Activation of the PI3K pathway interferes with caspase-mediated apoptosis through Akt-activation. Apoptotic stimuli may either be cytokines like TNF, or UV-irradiation. In the present example of which the results of a Western blot analysis are shown in Fig. 7B, UV-irradiation was used as to induce apoptosis. PTEN is an inhibitor of PI-3K action. Upon inhibition of PTEN by PTEN specific antisense oligonucleotides such as PTEN 48, PTEN 53 and PTEN 57 phosphorylated Akt is generated as shown. Activated Akt blocks caspases-mediated apoptosis. Apoptosis was monitored by the presence of cleaved Caspase 3.

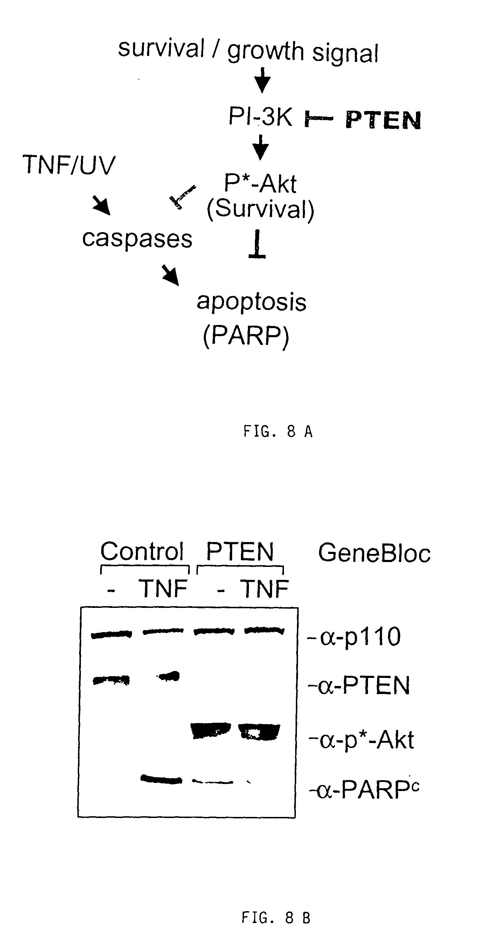

Fig. 8 A shows a schematic diagram indicating that PTEN knockdown interferes with TNF-induced apoptosis. Fig. 8B shows a Western Blot in which Hela cells were treated with tumor necrosis factor TNF as a stimulus. In this experiment, cleaved PARP is indicative of apoptosis. Lane 4 of the Western blot shows that only upon addition of the PTEN specific antisense oligonucleotide the phosphorylated form of Akt was generated and thus the amount of α-PARPC was accordingly reduced.

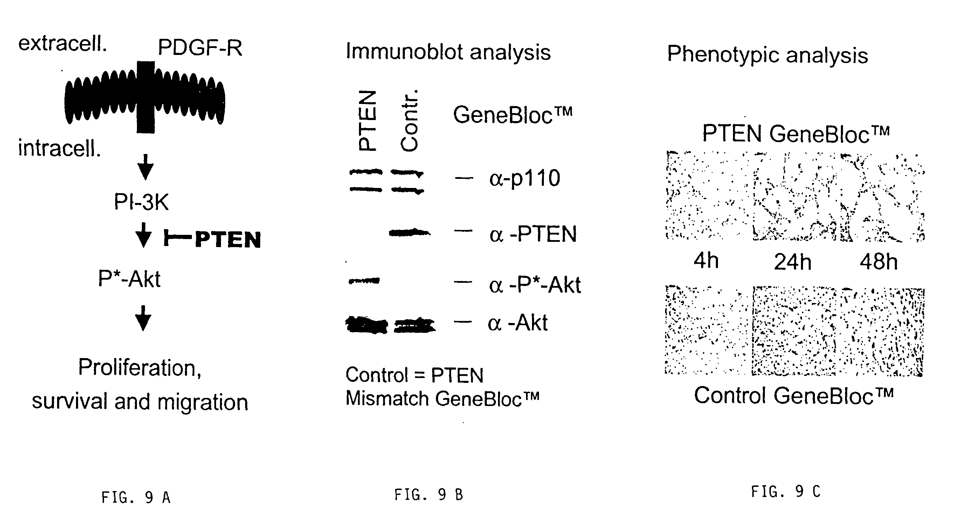

Fig. 9 A shows an overview of signalling and phenotypic changes induced after tumor suppression knocked down by PTEN specific antisense oligonucleotides. More particularly, a schematic pathway with PTEN inhibiting PI-3K activity is shown which would normally phosphorylate Akt thus leading to proliferation, survival and migration of HeLa cells as a model system for PTEN +/+ cells. Fig. 9 B presents the Western immunoblot analysis of an experiment studying the influence of PTEN specific antisense oligonucleotide or a control antisense nucleotide designated as mismatch GeneBlocTM. PTEN is only present in the control sample, but is absent when PTEN-specific antisense oligonucleotides are used. The successful knockdown of PTEN causes phosphorylation of Akt, indicative of induction of the signalling pathway. The phenotypic analysis as illustrated in Fig. 9 C shows the growth of the cells in matrigel. Growth on matrigel is used as a surrogate in vitro assay for metastatic or invasive cell growth. The cells treated with PTEN third generation antisense oligonucleotide ("PTEN GeneBlocTM") shown in Fig. 9C have a significant growth advantage over the control-treated cells.

The Western Blot in Fig. 10A shows the improved chemistry of the third generation antisense molecules ('Atugen chemistry') allowing for the specific knockdown of gene expression in growing cells compared to DNA/RNA chimera as known from the state of the art. The two PTEN antisense oligonucleotide species used in the experiment differ from each other in that the Atugen oligonucleotide chemistry is an improved, third generation form of the second generation DNA/RNA chimera one. Treatment with both antisense oligonucleotides designated as PTEN48 which have the same DNA sequence but differ in their chemical composition, result in the down-regulation of PTEN expression, and in the formation of the phosphorylated form of Akt.

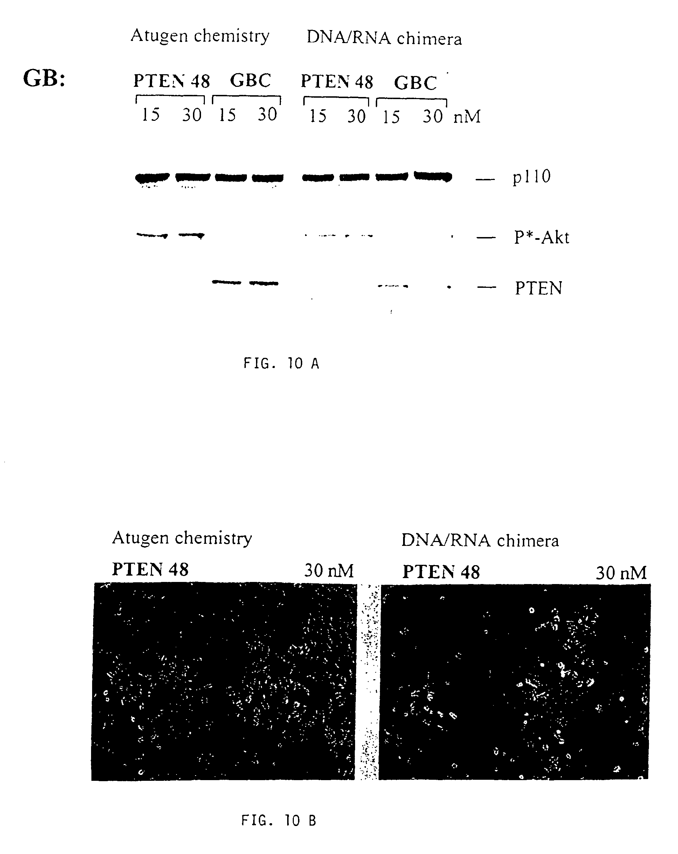

Fig. 10B shows cell-micrographs demonstrating that PTEN knockdown and its biological consequences can be analysed in normal growing cells. In contrast, DNA/RNA chimera antisense molecules typically arrest cells in the cell cycle or even cause apoptosis.

The invention is further illustrated in the examples from which further features, embodiments and advantages may be taken.

## Example 1: Materials and methods

The following materials and methods were used in connection with the various examples described herein. Whenever the various materials and methods are used the conditions are the one as outlined in the following if not indicated differently.

**Reagents.** 4-Hydroxytamoxifen (4-OHT) was purchased from Sigma. LY294002, PD98059 and aphidicolin were obtained from Calbiochem.

**Cell culture.** 3Y1 rat embryo fibroblasts (Kimura, G., Itagaki, A. and Summers, J. (1975). Rat cell line 3Y1 and its virogenic polyoma- and SV40- transformed derivatives. Int J Cancer **15**, 694-706.) were cultured at 37°C in Dulbecco's modified Eagle medium (DMEM) containing 10% bovine calf serum (CS), penicillin (50 µg/ml) and streptomycin (50 µg/ml). The human cervix carcinoma line HeLa and human prostate carcinoma PC-3 cells were obtained from the American Type Culture Collection (ATCC). HeLa cells were grown in Minimum essential medium Eagle with 2 mM L-glutamine, Earle's BSS, 1 mM sodium pyruvat, 0.1 mM non-essential amino acids, 10 % fetal calf serum (FCS), gentamycin (50 µg/ml) and amphotericin (50 ng/ml). PC-3 cells were cultured in F12K Nutrient Mixture (Kaighn's modification) containing, 10 % fetal calf serum (CS), gentamycin (50 µg/ml) and amphotericin (50 ng/ml).

Transfections were carried out in 96 well or 10-cm plates (at 30% to 50% confluency) by using various cationic lipids such as Oligofectamine, Lipofectamine (Life Technologies), NC388 (Ribozyme Pharmaceuticals, Inc., Boulder, CO), or FuGene 6 (Roche) according to the manufacturer's instructions. Antigen molecules, including third generation antisense molecules, were transfected by adding pre-formed 5x concentrated complex of GeneBloc and lipid in serum-free medium to cells in complete medium. The total transfection volume was 100 µl for cells plated in 96 wells and 10 ml for cells in 10 cm plates. The final lipid concentration was 0.8 to 1.2 µg/ml depending on cell density; the GeneBloc concentration is indicated in each experiment.

Stable HeLa cells were established by selection in hygromycin B (200 µg/ml) after transfection of 4 µg of a vector co-expressing M·p110*ER and hygromycin B phosphotransferase (see below). The cells were transfected with the plasmid overnight and then passaged at a dilution of 1:4 or 1:5 into selective medium. The medium was renewed every four days; after approximately two weeks the resistant colonies were trypsinized, combined and cultured in selective medium.

Pools of stably transfected HeLa cells in growth medium were stimulated with 200 nM 4-OHT in dimethylsulfoxide (DMSO) or with DMSO at 37°C for 20 h. In experiments in which the effect of LY294002 or PD98059 was analyzed, the reagents were added in DMSO at 10 µg/ml or 50 µg/ml 30 min to 1 h before lysis. Control cells were mock treated with DMSO.

**Antibodies.** The murine monoclonal anti-p110 antibody U3A and the murine monoclonal anti-p85 antibody FIA have been described (Klippel, A., Escobedo, J. A., Hirano, M. and Williams, L. T. (1994). The interaction of small domains between the subunits of phosphatidylinositol 3-kinase determines enzyme activity. Mol Cell Biol 14, 2675-2685. Rabbit polyclonal anti-Akt and anti-phospho Akt (S473) antibodies were obtained from Cell Signaling Technology. The murine monoclonal anti-PTEN antibody was from Santa Cruz Biotechnology.

**Plasmids and GeneBlocs.** A vector expressing an inducible form of the constitutively active PI 3-kinase, M•p110*ER, has been described (Klippel, A., Escobedo, M. A., Wachowicz, M. S., Apell, G., Brown, T. W., Giedlin, M. A., Kavanaugh, W. M. and Williams, L. T. (1998). Activation of phosphatidylinositol 3-kinase is sufficient for cell cycle entry and promotes cellular changes characteristic of oncogenic transformation. Mol Cell Biol **18,** 5699-5711). It was further modified by addition of an IRES sequence followed by the coding region for hygromycin B phosphotransferase. The IRES-hygro fragment was isolated via *Bgl* II ends from pIREShyg (Clontech) and ligated into the *Bam* HI site located 3' to the stop codon of M·p110*ER. Plasmid constructs which conferred hygromycin resistance in an M·p110*ER dependent manner were isolated and used for transfections.

The GeneBlocs used in the study have the following sequences whereby the structure of these antisense oligonucleotides corresponds to the one of the third generation of antisense oligonucleotides:

PTEN 48                        guccuuuCCCAGCTTTacaguga (SEQ ID No. 34)

| | |
|---|---|
| PTEN 52 | cuggaucAGAGTCAGTgguguca (SEQ ID No. 35) |
| PTEN 53 | ucuccuuTTGTTTCTGcuaacga (SEQ ID No. 36) |
| PTEN 57 | ugccacuGGTCTGTAAuccaggt ((SEQ ID No. 37) |
| mm PTEN 52 | cuggaugAGACTGAGTgcuguca (SEQ ID No. 38) |
| mm PTEN 53 | ucucauuTTCTTTGTGcucacga (SEQ ID No. 39) |
| Luciferase | cagaaugTAGCCATCCauccuug (SEQ ID No. 40 |
| p110alpha 79 | acuccaaAGCCTCTTGcucaguu (SEQ ID No. 41) |
| p110alpha 82 | uaccacaCTGCTGAACcagucaa (SEQ ID No. 42) |
| p110beta 88 | caaauucCAGTGGTTCauuccaa (SEQ ID No. 43) |
| p110beta 93 | ggcuaacTTCATCTTCcuuccca (SEQ ID No. 44) |
| mm p110alpha 79 | acugcaaACCCTGTTGcucacuu (SEQ ID No. 45) |
| mm p110alpha 93 | ggcuaagTTCTTCATCcuugcca (SEQ ID No. 46) |
| GBC | nnnnnnnNNNNNNNNnnnnnnn |

Syntheses of this type of nucleic acid molecules and their chemical modifications have been described by Thompson, J., Beigelman, L., McSwiggen, J. A., Karpeisky, A., Bellon, L., Reynolds, M., Zwick, M., Jarvis, T., Woolf, T., Haeberli, P., and Matulic-Adamic, J. (1999). Nucleic acid molecules with novel chemical compositions capable of modulating gene expression. World Intellectual Property Organization, International Patent, Ribozyme Pharmaceuticals Inc., International Publication Number, **WO 99/54459..** Briefly, third generation of antisense oligonucleotides have the following schematic structure: cap-(n)$_{5-7}$(N) $_{7-9}$(n)$_{5-7}$-cap. Hereby, cap represents inverted deoxy abasics or similar modifications at both ends; n stands for 2'-O-methyl ribonucleotides (A, G, U, C); N represents phosphorothioate linked deoxyribonucleotides (A, G, T, C). The respective mismatch positions in the mismatch control oligomers (mm) are underlined. GBC is composed of random 2'-O-methyl ribonucleotides and random phosphorothioate linked deoxyribonucleotides. GeneBlocs of the second generation of antisense molecules have the schematic structure: RRRnnnnNNNNNNNNNNnnnRRRN. Hereby, R indicates phosphorothioate linked 2'-O-methyl ribonucleotides (A, G, U, C); n stands for 2'-O-methyl ribonucleotides (A, G, U, C); N represents phosphorothioate linked deoxyribonucleotides (A, G, T, C). GBC is composed of random 2'-O-methyl ribonucleotides with or without phosphorothioate linkages and random phosphorothioate linked deoxyribonucleotides.

**Preparation of cell extracts and immunoblotting.** Cells were washed twice with cold phosphate-buffered saline and lysed at 4°C in lysis buffer containing 20 mM Tris (pH 7.5), 137 mM NaCl, 15% (vol/vol) glycerol, 1% (vol/vol) Nonidet P-40 (NP-40), 2 mM phenylmethylsulfonyl fluoride, 10 mg aprotinin per ml, 20 mM leupeptin, 2 mM benzamidine, 1 mM sodium vanadate, 25 mM β-glycerol phosphate, 50 mM NaF and 10 mM NaPPi. Lysates were cleared by centrifugation at 14,000 x g for 5 minutes and aliquots of the lysates were analyzed for protein expression by Western-blotting: Samples were separated by SDS-PAGE and transferred to nitrocellulose-filters (Schleicher & Schuell). Filters were blocked in TBST buffer (10 mM Tris-HCl (pH 7.5), 150 mM NaCl, 0.05% (vol/vol) Tween 20, 0.5% (wt/vol) sodium azide) containing 5% (wt/vol) dried milk. The respective antibodies were added in TBST at appropriate dilutions. Bound antibody was detected using anti-mouse- or anti-rabbit-conjugated horse radish peroxidase (Transduction Laboratories, BD) in TBST, washed, and developed using the SuperSignal West Dura (Pierce) or ECL (Amersham) chemoluminescence substrates.

**Determination of the rate of DNA synthesis by incorporation of bromodeoxyuridine.** 3Y1 cells plated in 96-wells at 1600 cells per well were transfected in triplicate samples with GeneBloc molecules. After ca. 30h the cells were starved in DMEM containing 10 mM HEPES (pH 7.2) under continuous GeneBloc treatment. The cells were released into the cell cycle at various time points by addition of 10% serum. Control cells were incubated with 5 µg/ml aphidicolin for 18 h. After pulse-labeling with 10 µM 5-bromo-2'-deoxyururidine (BrdU) for the last 2 h the cells were fixed and permeabilized. The relative amount of BrdU incorporated into each sample was determined by using the Cell Proliferation ELISA, BrdU (colorimetric) (Roche) according to the manufacturer's instructions. Incorporated BrdU was detected by measuring the absorbance at 405 nM and 490 nM using the Spectra Max 190 (Molecular Devices).

**Determination of the relative amounts of RNA levels by Taqman analysis.** The RNA of cells transfected in 96-wells was isolated and purified using the Invisorb RNA HTS 96 kit (InVitek GmbH, Berlin). Inhibition of PTEN mRNA expression was detected by real time RT-PCR (Taqman) analysis using PTEN 5' primer CACCGCCAAATTTAACTGCAGA (SEQ ID No. 47), PTEN 3' primer AAGGGTTTGATAAGTTCTAGCTGT (SEQ ID No. 48) and the Taqman probe Fam-TGCACAGTATCCTTTTGAAGACCATAACCCA-Tamra (SEQ ID No. 34). The reaction was carried out in 50 µl and assayed on the ABI PRISM 7700 Sequence detector (Applied Biosystems) according to the manufacturer's instructions under the following conditions: 48°C for 30 min, 95°C for 10 min, followed by 40 cycles of 15 sec at 95°C and 1 min at 60°C.

**Assaying cell growth on matrigel.** HeLa cells were transfected with GeneBlocs or treated with 200 nM 4-OHT for 48h. After trypsinization the cells were seeded into duplicate 24-wells (100.000 cells per well) pre-coated with 250 µl matrigel (Becton Dickinson). After continuing the transfection or the 4-OHT treatment for 48 to 72 h photographs were taken at 5x magnification with an Axiocam camera attached to an Axiovert S100 microscope (Zeiss). (Petersen, O. W., Ronnov-Jessen, L., Howlett, A. R. and Bissell, M. J. (1992). Interaction with basement membrane serves to rapidly distinguish growth and differentiation pattern of normal and malignant human breast epithelial cells. Proc Natl Acad Sci USA 89, 9064-9068.)

**Example 2: Improved antisense molecules allow to study the knock down of gene expression in normally proliferating cells**

As depicted in Fig. 10 B cells treated with the third generation antisense molecules as disclosed herein continued to grow and reached approximately 70 % confluence with few apoptotic cells. In contrast, 3Y1 cells treated with the 2nd generation antisense molecules barely grew and many rounded apoptotic cells were visible after 48 h. Older generations of antisense molecules are notorious for inducing cell cycle arrest or even exhibit toxic effects. In so far the third generation antisense oligonucleotides as disclosed herein allow for the very first time a target validation or target identification process in which there is no cell cycle arrest.

Fig. 10 A reveals that antisense molecules of both classes allowed for a significant knock down of PTEN protein. The protein level of p 10, the catalytic subunit of PI3-kinase, serve as loading control to ensure that the input signal of the pathway was not affected. PTEN functions as negative regulator of the PI3-kinase pathway.

Its loss of function is known to activate the protein kinase Akt (also known as PKB or Rac-protein kinase), a well characterized downstream effector of PI 3-kinase (ref). Activation of Akt was detected using a phospho-specific antibody that specifically recognizes the activated phosphorylated form. As expected, inhibition of PTEN expression resulted in an increase in phosphorylated Akt. The total level of Akt protein remained unchanged (not shown). Despite equal loading of total protein amounts the level of PTEN and phospho-Akt appeared somewhat reduced in extracts from cells treated with the older GeneBloc generation. This is most likely due to the fact that these cells were not able to grow normally. This experiment suggests that GeneBlocs of the third generation allow for efficient knock down of PTEN expression wihout interfering with cell proliferation.

**Example 3: Cells treated with antisense molecules exhibit a normal entry into S phase**

To study the knock down of gene expression and its effects in cells which progress normally through the cell cycle, it is essential to use antisense reagents and transfection conditions that do not arrest cells in the G1 phase or even impose toxicity.

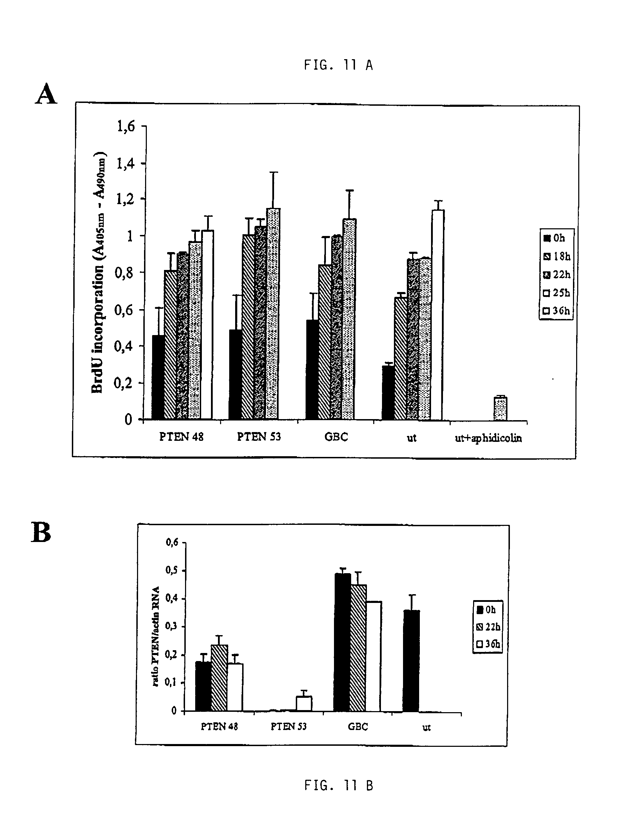

The results of respective experiments are described in Fig. 11 A and 11 B.

3Y1 cells were used, because these untransformed rat fibroblasts are very sensitive to transfection conditons and are prone to arrest or even undergo apoptosis. Subconfluent 3Y1 cells in 96 wells were treated with PTEN GeneBloc 48, GeneBloc 53 or GBC as a control. On day 2 the cells were synchronized in low serum. During days 3 and 4 the cells were released into the cell cycle by serum stimulation at various time points. BrdU was added for the last 2h of incubation. Untreated cells and cells that were treated with Aphidicolin for 24 h were used as controls. RNA knock down was analyzed in parallel samples. The rate of synthesis was determined via the amount of incorporated BrdU. Figure 3A shows a similar time course for the rate of DNA synthesis in each set of samples: All cells showed a strong increase in DNA synthesis after 18h with a plateau at approximately 25h. Unsynchronized cells exhibited a comparable rate of DNA synthesis, whereas the DNA synthesis in the Aphidicolin treated samples was strongly inhibited due to an arrest in the G1 phase of the cell cycle. Under these conditions PTEN mRNA levels remained downregulated (Fig. 11 B). This indicates that under the conditions used knock down of gene expression can be studied in cells which enter the cell cycle normally and continue to proliferate.

**Example 4: Phosphorylation of Akt is specifically induced in response to down regulation of PTEN expression**

In the past studies using antisense molecules have suffered from the lack of a sufficient number of suitable controls and the lack of reproducibility. Therefore, the intention was to make sure that activation of the PI 3-kinase pathway in the system disclosed herein is truly dependent on reduced PTEN function after treatment with third generation antisense molecules (GeneBlocs). Since hyperactivation of the pathway is caused by loss of the inhibitor PTEN, more than one third generation antisense molecule (GeneBloc) reducing PTEN expression of at least 50% can be expected to cause an increase in Akt phosphorylation. 3Y1 rat fibroblasts were treated with 15 and 30 nM of PTEN GeneBlocs 48, 53, 57 or with GBC. After 48h the cells had undergone several divisions, which allowed for the detection of reduced PTEN protein expression and activation of its effector, Akt. Cell extracts were analyzed by immunoblotting for the protein levels of p110, Akt and PTEN.

The results are shown in Fig. 2.

As shown in Figure 2 A a decrease in PTEN protein levels correlated with an increase in Akt phosphorylation with all three PTEN GeneBlocs. Total Akt protein levels remained unchanged indicating that the activation status of Akt was changed. PTEN GeneBloc 53 induced the most efficient knock down in PTEN expression and the strongest increase in Akt phosphorylation. The reduction in PTEN protein expression inversely correlates with increased Akt phosphorylation in a dose-dependent manner, with a drop in efficiency around 10 nM and below (Fig. 4A and data not shown).

To further ensure that Akt phosphorylation is not caused by an artefact or by stress imposed through the transfection conditions we tested various negative control third generation antisense molecules (negative control Gene-Blocs). We analyzed PTEN GeneBloc 53 in comparison to its 4 nucleotide mismatch control (mm), GBC or to a Gene-Bloc which successfully inhibits expression of Firefly luciferase (not shown). Firefly luciferase is not endogenously expressed in mammalian cells, therefore a GeneBloc targeting its sequence should have no specific effect, if any. None of the three negative control GeneBloc s either reduced the amount of PTEN protein or increased Akt phosphorylation, even at a concentration as high as 60 nM (Fig. 3 B). By contrast, PTEN GeneBloc 53 inhibited PTEN expression and caused an increase in Akt phosphorylation. This set of data indicates that only GeneBloc s against PTEN can successfully interfere with PTEN expression thereby activating downstream effectors of the PI 3-kinase pathway.

**Example 5: Akt phosphorylation induced by third generation antisense molecules-mediated down regulation of PTEN expression is dependent on PI3-kinase activation**

It has been reported that Akt can be activated e.g. in response to stress through PI 3-kinase independent mechanisms (Konishi, H., Fujiyoshi, T., Fukui, Y., Matsuzaki, H., Yamamoto, T., Ono, Y., Andjelkovic, M., Hemmings, B. A. and Kikkawa, U. (1999). Activation of protein kinase B induced by $H(2)O(2)$ and heat shock through distinct mechanisms dependent and independent of phosphatidylinositol 3-kinase. J Biochem 126, 1136-1143.) (Haas-Kogan, D., Shalev, N., Wong, M., Mills, G., Yount, G. and Stokoe, D. (1998). Protein kinase B (PKB/Akt) activity is elevated in glioblastoma cells due to mutation of the tumor suppressor PTEN/MMAC. Curr Biol 8, 1195-1198.) We therefore intended to test whether the increase in Akt phosphorylation observed after treatment with PTEN GeneBloc s was either dependent on the presumed 'input' signal, i.e. PI 3-kinase activity, or caused by stress induced by the respective GeneBlocs. The results are shown in Fig. 5 3Y1 cells were transfected with PTEN GeneBloc s 53, 57 or with GBC. After 48h incubation the cells were treated with the PI 3-kinase inhibitor LY294002, the MEK inhibitor PD98059, or vehicle for the last 30 min before lysis. The increase in Akt phosphorylation induced by the PTEN GeneBlocs was completely abrogated in the presence of LY294002, whereas the MEK inhibitor or vehicle had no effect (Fig. 4). This result suggests that activation of Akt induced after PTEN GeneBloc treatment still depends on PI 3-kinase signalling and on knock down of the PTEN inhibitory function.

**Example 6: Treatment with PTEN third generation antisense molecules interfere with UV-induced apoptosis**

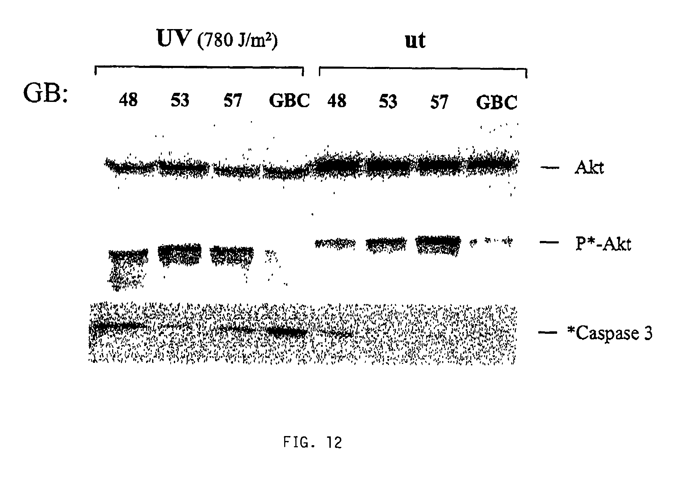

If knock down of PTEN expression mediated by third generation antisense molecules (GeneBlocs) results in a functional activation of PI 3-kinase signalling, then increased phosphorylation and activation of Akt should interfere with the effects of apoptotic stimuli. Activated Akt has been shown to mediate cell survival after treatment with apoptotic stimuli by inhibiting pro-apoptotic molecules such as caspases, Bad or ASK1 (Datta, S. R., Brunet, A. and Greenberg, M. E. (1999). Cellular survival: a play in three Akts. Genes Dev **13**, 2905-2927; Kim, A. H., Khursigara, G., Sun, X., Franke, T. F. and Chao, M. V. (2001). Akt phosphorylates and negatively regulates apoptosis signal-regulating kinase 1. Mol Cell Biol 21, 893-901)). It was previously shown that rat embryo fibroblasts undergo apoptosis after UV irradiation and that this response can be inhibited by activation of the PI 3-kinase pathway, and specifically by activated Akt (Kulik, G., Klippel, A. and Weber, M. J. (1997). Antiapoptotic signalling by the insulin-like growth factor I receptor, phosphatidylinositol 3-kinase, and Akt. Mol Cell Biol 17, 1595-1606)). We exposed 3Y1 cells to UV light after 48h GeneBloc treatment. 3h after irradiation cell extracts were harvested and analyzed for the presence of cleaved caspase 3 which is indicative for apoptosis. The results are shown in Fig. 12.

Cells which had been treated with PTEN GeneBlocs before UV irradiation had a lower amount of cleaved caspase 3 signal than control GeneBloc treated cells (Fig. 12). The amount of cleaved caspase 3 inversely correlated with the amount of Akt phosphorylation as demonstrated by the observation that GeneBloc 48 was less potent in activating Akt (also compare Fig. 2) and protecting against apoptosis than GeneBlocs 57 or 53. Cells which were not exposed to UV light after transfection contained very little amounts of cleaved caspase 3. This experiment suggests that reduced PTEN expression after GeneBloc treatment leads to a functional activation of Akt which can interfere with apoptosis.

**Example 7: Inhibition of PTEN expression by transient knock down mimics the loss of PTEN function in human tumor cells**

The effect of GeneBloc induced transient knock down of PTEN expression was to be compared to the loss of PTEN function in human tumor cells. To this end HeLa cells, a human tumor cell line with low metastatic growth potential that still expresses PTEN, were compared with PC-3 cells, a metastatic prostate cancer line which has lost PTEN expression. Due to the fact that PTEN has a high degree of homology between mammalian species, several lead GeneBlocs, i. e. third generation antisense molecules, also matched the human PTEN sequence. HeLa and PC-3 cells were treated in parallel with PTEN GeneBloc 53 or its mismatch control in duplicate samples. After 2 days the cell extracts were analyzed by Western-blotting. PTEN GeneBloc treated HeLa cells showed a strong reduction in PTEN protein that was associated with a substantial increase in Akt phosphorylation (Fig. 5). The extent of GeneBloc mediated Akt phosphorylation in Hela cells was comparable to the one observed in PTEN deficient PC-3 cells which exhibit a chronically high degree of Akt phosphorylation. PTEN GeneBloc treatment had no effect on PC-3 cells.

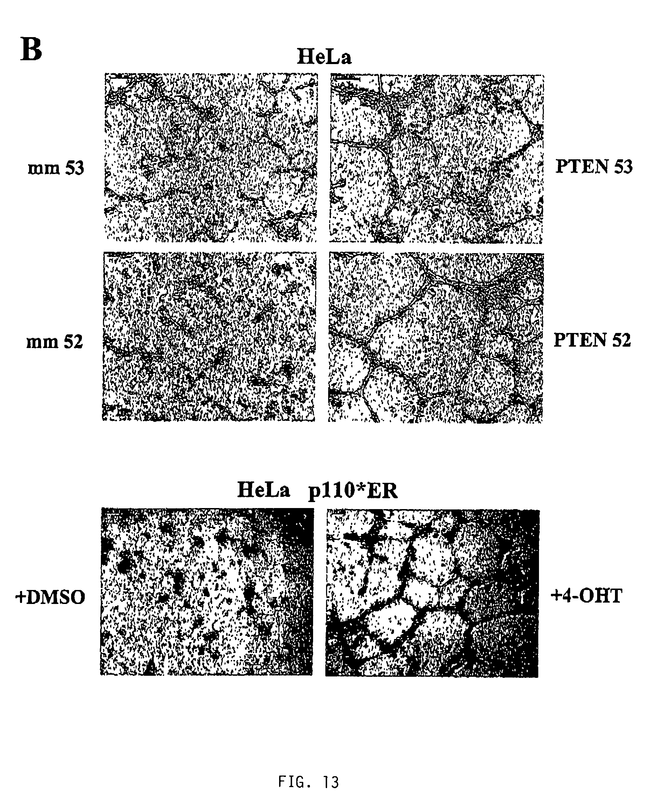

Loss of PTEN function has been correlated with an increased metastatic growth potential in late stage human cancers (Cantley, L. C. and Neel, B. G. (1999). New insights into tumor suppression: PTEN suppresses tumor formation by restraining the phosphoinositide 3-kinase/AKT pathway. Proc Natl Acad Sci USA 96, 4240-4245; Ali, I. U. (2000). Gatekeeper for endometrium: the PTEN tumor suppressor gene. J Natl Cancer Inst 92, 861-863). The intention was to test whether GeneBloc induced reduction in PTEN expression was sufficient to increase HeLa cell growth in matrigel. HeLa cells do not easily form metastases in a nude mouse tumor model (Bather, R., Becker, B. C., Contreras, G. and Furesz, J. (1985). Heterotransplantation studies with tissue culture cell lines in various animal and in vitro host systems. J Biol Stand 13, 13-22); and do not grow well on matrigel, an extracellular matrix that facilitates growth of invasive cell types. HeLa cells were transfected with PTEN GeneBlocs 52, 53 or their respective mismatch controls. After 48h incubation to allow for phenotypic activation the cells were trypsinized and seeded on matrigel in 24 wells. HeLa cells expressing an inducible version of a constitutively active PI 3-kinase, p110* (ref) were analyzed in parallel. Switching on p110* activity by adding 4-hydroxytamoxifen (4-OHT) results in a chronic activation of the PI 3-kinase pathway and anchorage independent cell growth as well as enhanced growth in matrigel. After 48h to 72h on matrigel photographs of the cells were taken. Cells treated with PTEN GeneBloc exhibited an increased potential to form network structures on matrigel and grew better, since more cells were present compared to cells that had been treated with the respective control GeneBloc (Fig. 13). A similar result was obtained with cells in which the PI 3-kinase pathway was switched on via p110*. Taken together the data indicate that a transient knock down of PTEN expression mediated by GeneBlocs can mimic the effects of loss of PTEN tumor suppressor function in human tumor cells.

**Example 8: Third generation antisense molecules (GeneBlocs) can effectively interrupt signal transduction induced by endogenous or recombinant PI 3-kinase**

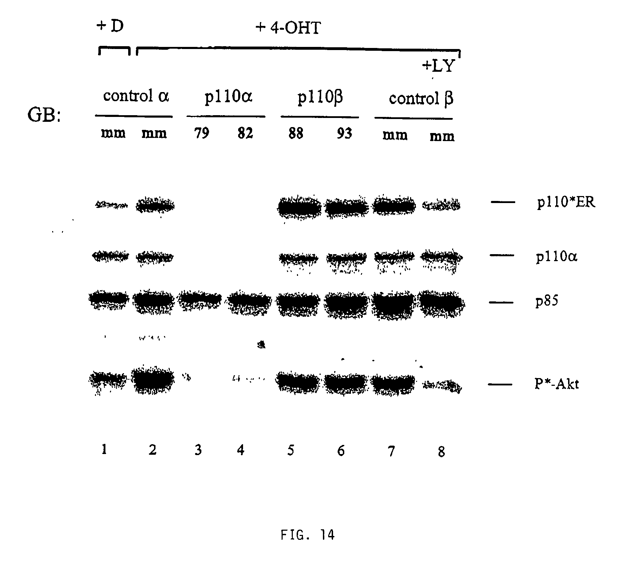

In the previous examples it was shown that third generation antisense molecules (GeneBlocs) can be used for activation of signal transduction responses. It was important to establish this before looking at inhibitory responses, because of the problems using antisense technologies mentioned above. Next it was tested whether GeneBlocs against the catalytic subunit of PI 3-kinase, p110, can inhibit phosphorylation of Akt in HeLa cells stably expressing the inducible p110* molecule in addition to endogenous p110. Most cells express two of the four known p110 isoforms, alpha and beta. Therefore, GeneBlocs against both isoforms were targeted. The cells were treated with two different GeneBlocs against each isoform or with the respective mismatch controls. p110* was activated by addition of 4-OHT after 30h, and cell extracts were analyzed after 50h. GeneBlocs against p110alpha efficiently knocked down expression of endogenous p110alpha as well as the larger recombinant p110*, which had been generated from p110 alpha (Figure 14). The inhibition of p110alpha expression correlated with a dramatic inhibition of Akt phosphorylation even though Akt was hyperphosphorylated in response to p110* activation compared to vehicle treated control cells. The inhibitory effect of Akt was as efficient as the one observed with the small molecule PI 3-kinase inhibitor, LY294002. Neither p110beta GeneBlocs nor the control GeneBlocs had any effect. p110beta knock down could not be observed on protein level since no suitable antibody was available. However, both p110beta GeneBlocs efficienty reduced mRNA expression in HeLa cells and inhibited Akt phosphorylation in other cell lines (data not shown). This suggests that p110alpha is the predominant isoform in HeLa cells, whereas p110beta can act as the predominant form in other cells. The experiment shows that p110 GeneBlocs can efficiently block PI 3-kinase dependent signal transduction.

**Example 9: Use of intron sequences for mediating a specific mRNA knock down**

As disclosed herein, it has been surprisingly found that intron specific antisense molecules such as the third generation antisense molecules are mediating mRNA knock down. Accordingly, the hnRNA is also a target for antisense molecule treatment including both therapeutic and non-therapeutic uses (such as, e. g. target validation) indicating that the antisense molecules are entering the nucleus and demonstrating that RNAse H activity is present in the nucleus.

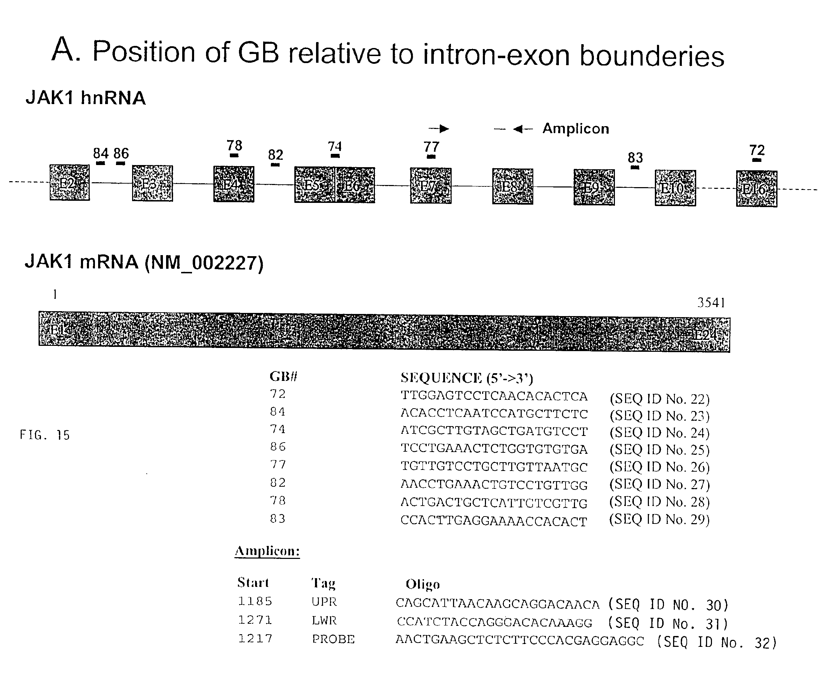

As shown in Fig. 15, third generation antisense molecules (GeneBlocs) were directed against both intron and exon sequences. GeneBloc 84 (SEQ ID No. 23), GeneBloc 86 (SEQ ID No. 25), GeneBloc 82 (SEQ ID No. 27) and GeneBloc 83 (SEQ ID No. 29) represent intron specific GeneBloc whereas GeneBloc 72 (SEQ ID No. 22), 74 (SEQ ID No. 24), 77 (SEQ ID No. 26), 78 (SEQ ID No. 28) are exon specific GBGeneBlocs. GBGeneBloc 74 (SEQ ID No. 24) is specific for mRNA overlapping exon 5 and 6.

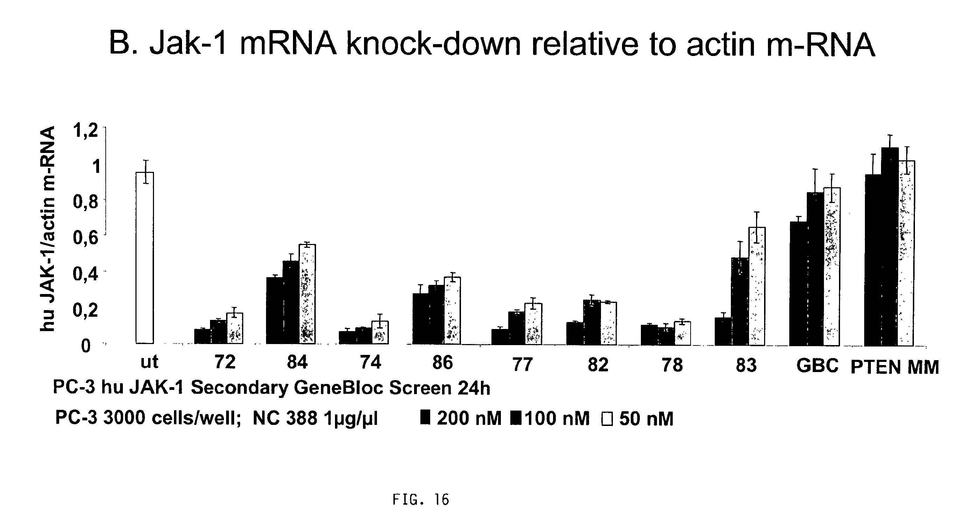

The particular gene is JAK-1 (Janus kinase 1), a protein tyrosine kinase as described by Modi et al. (Modi, B. S. et al.; Cytogenet. Cell Genetc. 69: 232-234, 1995.) The GeneBlocs were transfected in three different concentrations (200 nM, 100 nM and 50 nM) into PC3 cells (3000 cells/well) A third generation antisense molecule comprising 21 nucleotides as a random sequence and the mismatch PTEN GeneBloc as disclosed herein were used as negative controls. After 24 h incubation the RNA was isolated using a standard protocol (Invitek 96 well kit). A real time PCR (Taq-Man, Applied Biosystem) was performed using a beta-actin amplicon set in combination with a JAK-1 specific amplicon set with the primers as specified in Fig. 15. The JAK-1 mRNA was quantitated and is normalized by beta-actin. The results are shown in Fig. 16.

**Example 10: Use of RNAi for target validation**

This example proves that RNAi specific for tumor suppressor(s) such as PTEN, may be used to effectively knock down mRNA of such tumor suppressor(s).

The PTEN specific RNAi consisted of RNA only with 2 deoxy ribonucleotides at each end having the following sequence:

5'-    cuccuuuuguuucugcuaacg-TT (SEQ.ID.No 56)

3'-TT-gaggaaaacaaagacgauugc

As a control a RNAi molecule was generated having several missmatches which is referred to as PTEN non-specific RNAi and has the following sequence:

> 5'    cucauuuucuuugugcucacg-TT
>
> 3'-TT-gaguaaaagaaacacgagugc

The cells were infected using any of these two constructs according to the methods described herein above.

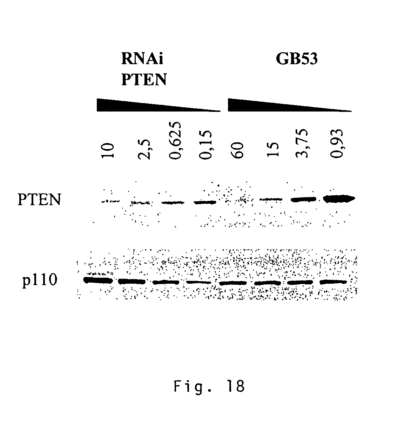

The result of this experiment is depicted in Fig. 18. More particularly, Fig. 18 shows the effect of various concentrations of PTEN specific RNAi (10 nM, 2.5 nM, 0.625 nM, and 0.15 nM) on the expression of PTEN and of p110, respectively. p110 serves an internal control. It may be taken from Fig. 18 that as little as 0.15 nM PTEN specific RNAi still efficiently knocks down PTEN mRNA whereas the amount of p110 is factually not influenced. As a positive control an antisense oligonucleotide specific for PTEN referred to as GeneBloc 53 (disclosed above) was also applied to the respective expression system at different concentrations (60 nM, 15 nM, 3.75 nM and 0.93 nM). It is to be noted that also the use of this antisense oligonucleotide allows for a specific knockdown of PTEN although the antisense oligonucleotide requires higher concentrations compared to RNAi in the present example.

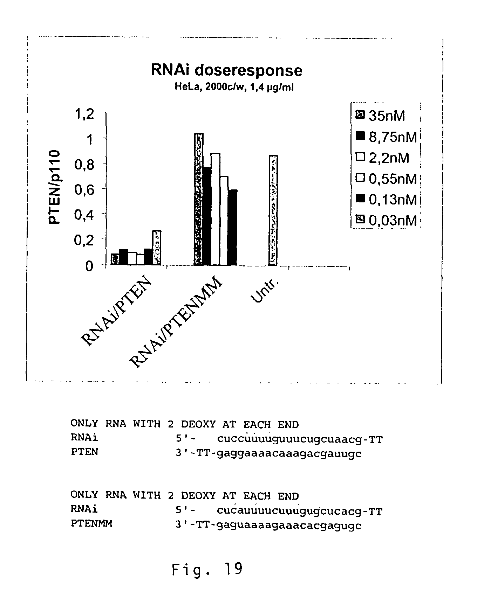

To further illustrate the impact of RNAi which is specific for PTEN, on the ratio of PTEN to p110 a dose response was generated which is shown in Fig. 19. From Fig. 19 it may be taken that even at the lowest concentration, i. e. 0.03 nM, the PTEN specific RNAi still reduces the ratio of PTEN to p110 to about one third of the respective control. This effect is more pronounced with higher concentrations of RNAi. This is in clear contrast to RNAi which is not specific for PTEN. This non-specific RNAi exhibits certain mismatches compared to the oligonucleotide sequence of the corresponding PTEN mRNA as may be taken from above. The effect of this negative control is about in the same range as observed for those HeLa cells not treated with either PTEN specific RNAi or PTEN non-specific RNAi.

**Example 11: Modulation of signal transduction by functional knock down of tumor suppressor Smad3 using antisense oligonucleotides**

This example shows that third generation antisense oligonucleotide (GeneBloc, GB) mediated knock down of Smad3 expression interferes with transforming growth factor (TGF)-β induced dephosphorylation/activation of the retinoblastoma cell cycle checkpoint.

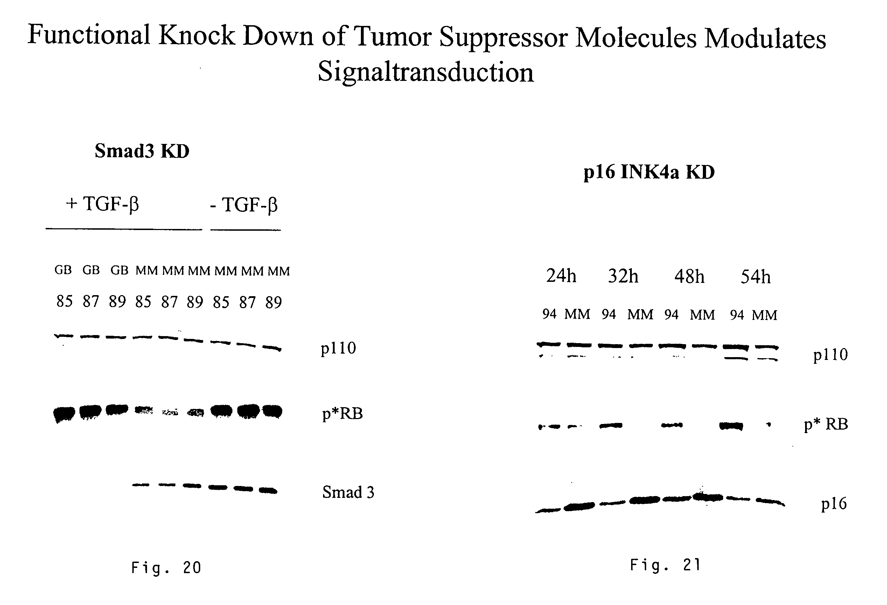

Smad3 mediates growth inhibitory or differentiation signals induced by TGF-β or activin receptor molecules and is a proposed tumor suppressor due to its mutational inactivation in many invasive human tumors. Human keratinocytes were transfected with three different SMAD3 specific GBs 85, 87 and 89 as well as their corresponding mismatch controls. After 24 h cells were synchronized in low serum under continued transfection. After 48 h the cells were released into the cell cycle by addition of 10% FCS in the presence (2 ng/ml) or absence of TGF-β. The cells were harvested on day three and the cell extracts were analyzed by immunoblotting for the relative protein amounts of p110, phosphorylated retinoblastoma (P*-Rb) and Smad3 as indicated on the right of Fig. 20 representing the results of this example.

The level of p110 did not change and served as loading control. TGF-β treatment resulted in the activation = dephosphorylation of Rb protein. In samples in which Smad3 levels were downregulated in response to GB treatment, the TGF-β induced effect on Rb was inhibited, and Rb remained inactive and fully phosphorylated. Rb is the checkpoint for G1 to S phase transition and itself a tumor suppressor. Chronic inactivation of Rb by phosphorylation or by loss of function enhances proliferation and contributes to tumorigenesis.

**Example 12: Modulation of signal transduction by functional knock down of tumor suppressor p16Ink4a using antisense oligonucleotides**

This example shows that third generation antisense oligonucleotide (GeneBloc, GB) mediated knock down of p16Ink4a results in increased Rb phosphorylation.

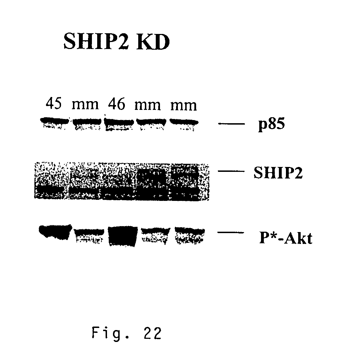

HeLa cells were transfected in quadruplicate samples with a p16 specific GB (p16 GB 94) or its mismatch derivative. The cells were lysed after 24 h, 32 h, 48 h and 54h, respectively. The cell extracts were analyzed by immunoblotting. The particular reaction conditions were the same as indicated in the other examples related to knock down of tumor suppressors. The result of this example is shown in Fig. 21 whereby the positions of p110 (loading control), phosphorylated Rb (P*-Rb) and p16 are indicated on the right.

This example proves that lack of the cyclin-dependent kinase (Cdk) inhibitor p 16Ink4a results in increased phosphorylation/inactivation of Rb by Cdk molecules.

**Example 13: Modulation of signal transduction by functional knock down of tumor suppressor SHIP2 using antisense oligonucleotides**

This example shows that third generation antisense oligonucleotides (GeneBlocs, GBs) cause reduced expression of the phospholipid phosphatase SHIP2 which results in increased PI 3-kinase signalling.

SHIP2 is a proposed tumor suppressor that dephosphorylates the PI 3-kinase product $PI3,4,5P_3$ at the 5' position of the inositol ring, thereby generating $PI3,4P_2$. For comparison, PTEN removes the 3' phosphate of the PI 3-kinase phospholipid products.

Human prostate cells were treated with the SHIP2 specific GBs 45 and 46 as well as their respective mismatch controls and one additional unrelated mismatch GB. After 72h the cells were lysed and the extracts analysed by Western-blotting. p85 served as loading control. The particular reaction conditions were the same as indicated in the other examples related to knock down of tumor suppressors. The results are shown in Fig. 22.

Samples with reduced amounts of SHIP2 protein exhibit an increased level of phosphorylated Akt. This indicates that SHIP2 also functions as negative regulator of PI 3-kinase signalling in these cells similar to PTEN.

The features of the present invention disclosed in the specification, the sequence listing, the claims and/or the drawings may both separately and in any combination thereof be material for realizing the invention in various forms thereof.

**Claims**

1. A compound, preferably 14 to 30 nucleobases, preferably 17 to 23 nucleobases and more preferably 17 to 21 nucleobases in length, targeted to a nucleic acid whereby the nucleic acid is heterogeneous nuclear RNA (hnRNA).

2. A compound, preferably 14 to 30 nucleobases, preferably 17 to 23 nucleobases and more preferably 17 to 21 nucleobases in length, targeted to a nucleic acid whereby the nucleic acid is an intron of a nucleic acid molecule.

3. The compound according to claim 1 or 2 which is a functional oligonucleotide.

4. The compound according to claim 3, wherein the functional oligonucleotide is selected from the group comprising antisense oligonucleotide, ribozyme and RNAi.

**5.** The compound according to any of claims 1 to 4 wherein the nucleic acid is a genomic sequence.

**6.** The compound according to any of claims 1 to 5 **characterized in that** the nucleic acid molecule or a part thereof is coding for a polypeptide.

**7.** The compound according to any of claims 3 to 6, wherein the functional oligonucleotide comprises at least one modified internucleoside linkage.

**8.** The compound according to claim 7, wherein the modified internucleoside linkage is a phosphorothioate linkage.

**9.** The compound according to any of claims 3 to 8, wherein the functional oligonucleotide comprises at least one modified sugar moiety.

**10.** The compound according to claim 9, wherein the modified sugar moiety is a 2'-O-methoxy or a 2'O-methoxyethyl sugar moiety.

**11.** The compound according to any of claims 3 to 10, wherein the functional oligonucleotide comprises at least one modified nucleobase.

**12.** The compound according to claim 11, wherein the modified nucleobase is a 5'-methylcytosine.

**13.** The compound according to any of claims 1 to 12, wherein the compound, preferably the functional oligonucleotide, is a chimeric oligonucleotide.

**14.** The compound according to claim 13, showing the following structure:

$$\text{cap-}(n_p)_x(N_s)_y(n_p)_z\text{-cap}$$

whereby cap represents inverted deoxy abasics or similar modifications
n represents 2'-O-methyl ribonucleotides ;
N represents phosphorothioate-linked deoxyribonucleotides ,
subscript p represents phosphodiester linkage,
subscript s representsphosphorothioate linkage,
subscript x represents an integer from 5 to 7;
subscript y represents an integer from 7 to 9; and
subscript z represents an integer from 5 to 7.

**15.** A composition comprising a compound according to any of claims 1 to 14 and a pharmaceutically acceptable carrier or diluent.

**16.** A method for inhibiting the expression of a gene in a cell or tissue of a mammal , preferably in vitro, comprising contacting said cells or tissues, preferably in vitro, with a compound of any of claims 1 to 14 so that the expression of the gene is inhibited.

**17.** Method according to claim 16, wherein the mammal is selected from the group comprising mice , rats, guinea pigs, hamsters, monkeys, dogs and cats.

**18.** Use of the sequence of an intron of a gene comprising at least one intron and at least one exon for the design of a compound targeting said gene, whereby the compound is an functional oligonucleotide preferably a functional oligonucleotide according to any of claims 3 to 14.

**19.** A method for the identification and/or validation of a target comprising the following step:

a) applying to an expression system a functional oligonucleotide wherein the functional oligonucleotide is specific for PTEN hnRNA,.

**20.** A method for the identification and/or validation of a target comprising the following step:

   a) applying to an expression system a functional oligonucleotide wherein the functional oligonucleotide is specific for PTEN mRNA.

**21.** The method according to claim 19 or 20, wherein the target is part of the PI3K/PTEN related pathway.

**22.** The method according to claim 19 or 20, wherein the target is part of a pathway which is selected from the group comprising the Akt related pathway, the EGF-related autokrine loop and the mTOR pathway.

**23.** The method according to any of claims 19 to 22, wherein the target is involved in the pathogenetic mechanism of a disease or condition selected from the group comprising glioblastoma, prostate cancer, breast cancer,, lung cancer, liver cancer, colon cancer, pancreatic cancer and leukaemia.

**24.** The method according to any of claims 19 to 23, wherein the target is involved in a biological process selected from the group comprising proliferation, cell survival, migration, apoptosis, stress signalling, metastasis, anoikis, cell attachment and processes signalling through modulation of PI3K activity.

**25.** The method according to any of claims 19 to 24, wherein the target is selected from the group comprising transcription factors, motility factors, cell cycle factors, cell cycle inhibitors, enzymes, growth factors, cytokines, and tumor suppressors.

**26.** The method according to any of claims 19 to 25, wherein the target is a tumor suppressor and wherein the tumor suppressor is selected from the group comprising landscapers, gatekeepers and caretakers.

**27.** The method according to any of claims 19 to 26, wherein the method further comprises as step b)
   comparing the expression pattern of the expression system upon application of the functional oligonucleotide with the expression pattern of the expression system under control conditions.

**28.** The method according to any of claims 19 to 27, wherein a further expression modifying agent is applied to the expression system, the expression pattern of the expression system is detected and the expression pattern is compared to the expression pattern generated upon steps a) and/or b).

**29.** The method according to claim 28, wherein the expression modifying agent is a functional oligonucleotide.

**30.** The method according to claim 28 or 29, wherein the expression modifying agent is modifying the expression of a second target, preferably according to any of claims 21 to 26.

**31.** The method according to any of claims 28 to 30, wherein the second target is different from the target according to any of claims 19 to 30.

**32.** The method according to claims 19 to 31, wherein the target is the molecular target of PTEN, preferably of PTEN acting as a tumor suppressor.

**33.** A method for the identification and/or validation of a target wherein the target is part of a tumor suppressor related pathway comprising the following step:

   a) applying to an expression system a functional oligonucleotide wherein the functional oligonucleotide is specific for hnRNA of a tumor suppressor, preferably the non-coding part thereof.

**34.** A method for the identification and/or validation of a target wherein the target is part of a tumor suppressor related pathway comprising the following step:

   a) applying to an expression system a functional oligonucleotide wherein the functional oligonucleotide is specific for an mRNA encoding the tumor suppressor.

**35.** The method according to claim 33 or 34, wherein the target is involved in the pathogenetic mechanism of a disease or condition selected from the group comprising glioblastoma, prostate cancer, breast cancer, lung cancer, liver cancer, colon cancer, pancreatic cancer and leukaemia.

**36.** The method according to claim 33 or 35, wherein the target is involved in a biological process selected from the group comprising proliferation, cell survival, migration, apoptosis, stress signalling, metastasis, anoikis, cell attachment. processes involving activation of PI3K and cancer relevant pathways involving signalling induced by various growth factors or cytokines.

**37.** The method according to any of claims 33 to 36, wherein the target is a tumor suppressor and the tumor suppressor is selected from the group comprising landscapers, gatekeepers and caretakers.

**38.** The method according to any of claims 33 to 37, wherein the method further comprises as step b)
comparing the expression pattern of the expression system upon application of the functional oligonucleotide with the expression pattern of the expression system under control conditions.

**39.** The method according to any of claims 33 to 38, wherein a further expression modifying agent is applied to the expression system, the expression pattern of the expression system is detected and the expression pattern is compared to the expression pattern generated upon steps a) and/or b).

**40.** The method according to claim 39, wherein the expression modifying agent is a functional oligonucleotide.

**41.** The method according to claim 39 or 40, wherein the expression modifying agent is modifying the expression of a second target, preferably a target according to any of claims 21 to 26.

**42.** Antisense oligonucleotide selected from the group comprising

B ugaacug$C_s$$T_s$$A_s$$G_s$$C_s$$C_s$$T_s$$C_s$$T_s$ggauuug B (SEQ ID No. 1)

B uggacaa$C_s$$A_s$$A_s$$G_s$$T_s$$G_s$$T_s$$C_s$$A_s$aaacccu B (SEQ ID No. 2)

B ggaaacc$T_s$$C_s$$T_s$$C_s$$T_s$$T_s$$A_s$$G_s$$C_s$caacugc B (SEQ ID No. 3)

B uguugca$G_s$$A_s$$A_s$$G_s$$G_s$$T_s$$T_s$$C_s$$A_s$uuccugu B (SEQ ID No. 4)

B cuuccga$G_s$$A_s$$G_s$$G_s$$A_s$$G_s$$A_s$$G_s$$A_s$acugagc B (SEQ ID No. 5)

B ccacaaa$C_s$$T_s$$G_s$$A_s$$G_s$$G_s$$A_s$$T_s$$T_s$gcaaguu B (SEQ ID No. 6)

B ucugaca$C_s$$A_s$$A_s$$T_s$$G_s$$T_s$$C_s$$C_s$$T_s$auugcca B (SEQ ID No. 7)

B aaggagg$A_s$$G_s$$A_s$$G_s$$A_s$$G_s$$A_s$$T_s$$G_s$gcagaag B (SEQ ID No. 8)

B guccuuu$C_s$$C_s$$C_s$$A_s$$G_s$$C_s$$T_s$$T_s$$T_s$acaguga B (SEQ ID No. 9)

B cuggauc$A_s$$G_s$$A_s$$G_s$$T_s$$C_s$$A_s$$G_s$$T_s$gguguca B (SEQ ID No. 10)

B ucuccuuT$_s$T$_s$G$_s$T$_s$T$_s$T$_s$C$_s$T$_s$G$_s$cuaacga B (SEQ ID No. 11)

B ugaacugC$_s$T$_s$A$_s$G$_s$C$_s$C$_s$T$_s$C$_s$T$_s$ggauuug B (SEQ ID No. 12)

B ugcugauC$_s$T$_s$T$_s$C$_s$A$_s$T$_s$C$_s$A$_s$A$_s$aagguuc B (SEQ ID No. 13)

B acuuugaT$_s$G$_s$T$_s$C$_s$A$_s$C$_s$C$_s$A$_s$C$_s$acacagg B (SEQ ID No. 14)

B uggguccT$_s$G$_s$A$_s$G$_s$T$_s$T$_s$G$_s$G$_s$A$_s$ggaguag B (SEQ ID No. 15)

B cuucaccT$_s$T$_s$T$_s$A$_s$G$_s$C$_s$T$_s$G$_s$G$_s$cagacca B (SEQ ID No. 16)

B ugccacuG$_s$G$_s$T$_s$C$_s$T$_s$G$_s$T$_s$A$_s$A$_s$uccaggt B (SEQ ID No. 17)

B ucucuggT$_s$C$_s$C$_s$T$_s$T$_s$A$_s$C$_s$T$_s$T$_s$ccccaua B (SEQ ID No. 18)

B ucgucuuC$_s$A$_s$C$_s$T$_s$T$_s$A$_s$G$_s$C$_s$C$_s$auugguc B (SEQ ID No. 19)

B gucuuucT$_s$G$_s$C$_s$A$_s$G$_s$G$_s$A$_s$A$_s$A$_s$ucccaua B (SEQ ID No. 20)

whereby B stands for inverted abasic, positions 1 through 7 and positions 17 through 23 are 2'-O-methylated ribonucleotides and are phosphodiester -linked, positions 8 through 17 are phosphorothioate linked, positions 8 through 16 are desoxynucleotides, position 17 is a ribonucleotide,

B g$_s$u$_s$c$_s$cuuuC$_s$C$_s$C$_s$A$_s$G$_s$C$_s$T$_s$T$_s$T$_s$acag$_s$u$_s$g$_s$a B (SEQ ID No. 21)

whereby B stands for inverted abasic, positions 1 through 7 are 2'-O-methylated ribonucleotides, positions 8 through 16 are desoxynucleotides, positions 17 through 23 are 2'-O-methylated ribonucleotides, positions 1 through 4 are phosphorothioate linked, positions 4 through 8 are phosphodiester- -linked, positions 8 through 17 are phosphorothioate -linked, positions 17 through 20 are phosphodiester- linked, and positions 20 through 23 are phosphorothioate linked,

B agaccaCAAACTGAGgauugc B (SEQ ID No 50, also referred to herein as huPTEN:1686L21),

B agacgaCTAACTCAGcauugc B (SEQ ID No 51, also referred to herein as huPTEN:1686L21 4MM),

B cccuuuCCAGCTTTAcaguga B (SEQ ID No 52, also referred to herein as huPTEN:1420L21),

ccguuuGCACCTTTAgaguga (SEQ ID No 53, also referred to herein as huPTEN:1420L21 4MM),

B aagcagCAAAGTCCTaagcag B (SEQ ID No 54, also referred to herein as huPTEN intron),

B cagaauTGGGCTGTAuuuggu B (SEQ ID No 55, also referred to herein as huPTEN intron),

whereby B represents an inverted abasic nucleotide, each and any of the minor letters represents independently from each other a 2'-O-methyl ribonucleotide such as A, G, U and C, and each and any of the capital letters represents independently from each other a phosphorothioate-linked deoxyribonucleotide such as A,G, T and C.

43. Use of any of the antisense oligonucleotide according to claim 42 and/or the compound according to any of claims 1 to 14 in a method according to any of claims 29 to 41.

44. A method for the generation of a functional oligonucleotide, preferably for use in a method according to any of the preceding claims, comprising the following steps:

a) providing an initial functional oligonucleotide specific for the hnRNA,, or the mRNA of a tumor suppressor, preferably PTEN,

b) modifying the initial functional oligonucleotide, and

c) testing the functional oligonucleotide modified in step b) on its specificity for the mRNA.

45. The Method according to claim 44, wherein the testing is done in an expression system.

46. The Method according to claim 44 or 45 further comprising the step of comparing the specificity of the initial and the modified functional oligonucleotide.

47. The Method according to any of claims 44 to 46, wherein the initial functional oligonucleotide is an oligonucleotide according to claim 44.

48. A method for the screening of a candidate compound interacting with a target which is either part of a tumor suppressor related pathway or part of a PTEN related pathway, the method comprising the following steps:

- providing an expression system to which a functional oligonucleotide, preferably the compound according to any of the preceding claims, is added, wherein the functional oligonucleotide is either specific for the hnRNA, preferably the non-coding part thereof, or for the mRNA of a tumor suppressor, whereby preferably the tumor suppressor is PTEN.

- screening a library of candidate compounds in said expression system to identify one or more elements of the library having activity with regard to interacting with the target and, optionally,

- identifying said elements.

FIG. 1

# Growth factor receptor

extracellular

intracellular

**PD98059** ⊣ MEK    PI 3-kinase ⊢ **LY294002**
p110/p85

⊢ PTEN[-/-]

MAP kinase    Akt

Proliferation, survival and migration

FIG. 2

A

FIG. 3

B

FIG. 4

FIG. 5

Human cells

FIG. 6

survival / growth response

↓

PI 3-K ⊢— PTEN

↓

TNF/UV

P*-Akt
(Survival)

caspases

apoptosis

FIG. 7 A

UV-treated

| 48 | 53 | 57 | Contr. | GeneBloc |

— α-Akt

— α- p*-Akt

— α- Caspase3c

FIG. 7 B

survival / growth signal

PI-3K ⊢ **PTEN**

TNF/UV

P*-Akt
(Survival)

caspases

apoptosis
(PARP)

FIG. 8 A

Control   PTEN   GeneBloc
-   TNF   -   TNF

-α-p110

-α-PTEN

-α-p*-Akt

-α-PARPc

FIG. 8 B

extracell. PDGF-R

intracell.

↓

PI-3K

↓ ⊢PTEN

P*-Akt

↓

Proliferation,
survival and migration

**FIG. 9 A**

Immunoblot analysis

PTEN Contr. GeneBloc™

— α-p110

— α -PTEN

— α -P*-Akt

— α -Akt

Control = PTEN
Mismatch GeneBloc™

**FIG. 9 B**

Phenotypic analysis

PTEN GeneBloc™

4h   24h   48h

Control GeneBloc™

**FIG. 9 C**

Atugen chemistry     DNA/RNA chimera

GB:     PTEN 48   GBC     PTEN 48   GBC

15   30   15   30     15   30   15   30   nM

—— pl10

—— P*-Akt

—— PTEN

FIG. 10 A

Atugen chemistry                    DNA/RNA chimera

**PTEN 48**          30 nM        **PTEN 48**          30 nM

FIG. 10 B

FIG. 11 A

FIG. 11 B

FIG. 12

**B**

**HeLa**

mm 53 — PTEN 53

mm 52 — PTEN 52

**HeLa p110*ER**

+DMSO — +4-OHT

FIG. 13

FIG. 14

# A. Position of GB relative to intron-exon bounderies

**JAK1 hnRNA**

**JAK1 mRNA (NM_002227)**

FIG. 15

| GB# | SEQUENCE (5'->3') | |
|---|---|---|
| 72 | TTGGAGTCCTCAACACACTCA | (SEQ ID No. 22) |
| 84 | ACACCTCAATCCATGCTTCTC | (SEQ ID No. 23) |
| 74 | ATCGCTTGTAGCTGATGTCCT | (SEQ ID No. 24) |
| 86 | TCCTGAAACTCTGGTGTGTGA | (SEQ ID No. 25) |
| 77 | TGTTGTCCTGCTTGTTAATGC | (SEQ ID No. 26) |
| 82 | AACCTGAAACTGTCCTGTTGG | (SEQ ID No. 27) |
| 78 | ACTGACTGCTCATTGTCGTTG | (SEQ ID No. 28) |
| 83 | CCACTTGAGGAAAACCACACT | (SEQ ID No. 29) |

**Amplicon:**

| Start | Tag | Oligo | |
|---|---|---|---|
| 1185 | UPR | CAGCATTAACAAGCAGGACAACA | (SEQ ID NO. 30) |
| 1271 | LWR | CCATCTACCAGGGACACAAAGG | (SEQ ID No. 31) |
| 1217 | PROBE | AACTGAAGCTCTCTTCCCACGAGGAGGC | (SEQ ID No. 32) |

EP 1 325 955 A1

# B. Jak-1 mRNA knock-down relative to actin m-RNA

FIG. 16

EP 1 325 955 A1

R = 2'-O-Methyl
D = 2' deoxy

iB-R$_p$R$_p$R$_p$R$_p$R$_p$R$_p$R$_p$D$_s$D$_s$D$_s$D$_s$D$_s$D$_s$D$_s$D$_s$D$_s$R$_p$R$_p$R$_p$R$_p$R$_p$R$_p$R-iB

| 7 | 9 | 7 |

FIG. 17

Fig. 18

EP 1 325 955 A1

**RNAi doseresponse**

HeLa, 2000c/w, 1,4 µg/ml

Legend: 35nM, 8,75nM, 2,2nM, 0,55nM, 0,13nM, 0,03nM

Y-axis: PTEN/p110

X-axis: RNAi/PTEN, RNAi/PTENMM, Untr.

```
ONLY RNA WITH 2 DEOXY AT EACH END
RNAi          5'-    cuccuuuuguuucugcuaacg-TT
PTEN          3'-TT-gaggaaaacaaagacgauugc


ONLY RNA WITH 2 DEOXY AT EACH END
RNAi          5'-    cucauuuucuuugugcucacg-TT
PTENMM        3'-TT-gaguaaaagaaacacgagugc
```

Fig. 19

# Functional Knock Down of Tumor Suppressor Molecules Modulates Signaltransduction

**Smad3 KD**

+ TGF-β        - TGF-β

GB  GB  GB  MM MM MM MM MM MM

85  87  89  85  87  89  85  87  89

p110

p*RB

Smad 3

Fig. 20

**p16 INK4a KD**

24h      32h      48h      54h

94  MM   94  MM   94  MM   94  MM

p110

p* RB

p16

Fig. 21

SHIP2 KD

45 mm 46 mm mm

p85

SHIP2

P*-Akt

Fig. 22

**European Patent Office**

## PARTIAL EUROPEAN SEARCH REPORT

which under Rule 45 of the European Patent Convention shall be considered, for the purposes of subsequent proceedings, as the European search report

**Application Number**

EP 02 00 0357

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 97 38728 A (HOKE GLENN D JR ;DYAD PHARMACEUTICAL CORP (US)) 23 October 1997 (1997-10-23) * page 2 - page 3, line 20 * * page 14, line 1 - page 17 * | 1,3,4, 6-9, 15-17,44 | C12N15/11 A61K31/7088 C12Q1/68 |
| Y | * claims * | 14,33-41 | |
| X | WO 00 20645 A (BUTLER MADELINE M ;SHANAHAN WILLIAM J JR (US); BAKER BRENDA F (US)) 13 April 2000 (2000-04-13) * the whole document * | 1-4, 6-13, 15-18 | |
| X | EP 0 691 402 A (HOECHST SCHERING AGREVO GMBH) 10 January 1996 (1996-01-10) * the whole document * | 1,3-6 | |
| X | WO 01 07457 A (COWSERT LEX M ;MONIA BRETT P (US); ISIS PHARMACEUTICALS INC (US)) 1 February 2001 (2001-02-01) | 20-29, 32,42-48 | |
| Y | * the whole document * | 19-32,42 | |
| | ---<br>-/-- | | **TECHNICAL FIELDS SEARCHED (Int.Cl.7)**<br><br>C12N |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claims 16, 17 (as far as in vivo methods are concerned) are directed to a method of treatment of the human/animal body (Article 52(4) EPC), the search has been carried out and based on the alleged effects of the compound/composition.

| Place of search | Date of completion of the search | Examiner |
|---|---|---|
| THE HAGUE | 14 June 2002 | Andres, S |

EPO FORM 1503 03.82 (P04C07)

## PARTIAL EUROPEAN SEARCH REPORT

**Application Number**

EP 02 00 0357

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | WO 98 13526 A (ARROW AMY ;WOOLF TOD M (US); OLIGOS ETC INC (US); DALE RODERIC M K) 2 April 1998 (1998-04-02) * the whole document * --- | 14,42 | |
| Y | PRONK BERT: "GeneblocTM tools for target discovery and validation." ABSTRACTS OF PAPERS AMERICAN CHEMICAL SOCIETY, vol. 222, no. 1-2, 2001, page MEDI296 XP008003801 222nd National Meeting of the American Chemical Society;Chicago, Illinois, USA; August 26-30, 2001 ISSN: 0065-7727 * the whole document * --- | 19-41 | |
| X | WO 01 90341 A (COWSERT LEX M ;MONIA BRETT P (US); MCKAY ROBERT (US); ISIS PHARMAC) 29 November 2001 (2001-11-29) * the whole document * --- | 20-29, 32,42-48 | |
| D,A | WO 99 54459 A (RIBOZYME PHARM INC) 28 October 1999 (1999-10-28) * page 27, line 19 - page 28, line 10 * * claims * --- | 1-48 | |
| D,A | VAZQUEZ FRANCISCA ET AL: "The PTEN tumor suppressor protein: An antagonist of phosphoinositide 3-kinase signaling." BIOCHIMICA ET BIOPHYSICA ACTA., vol. 1470, no. 1, 14 February 2000 (2000-02-14), pages M21-M35, XP002202192 ISSN: 0006-3002 ----- | | |

TECHNICAL FIELDS SEARCHED (Int.Cl.7)

**EP 1 325 955 A1**

## ANNEX TO THE EUROPEAN SEARCH REPORT
## ON EUROPEAN PATENT APPLICATION NO.

EP 02 00 0357

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2002

| Patent document cited in search report | | Publication date | Patent family member(s) | | Publication date |
|---|---|---|---|---|---|
| WO 9738728 | A | 23-10-1997 | AU | 2666797 A | 07-11-1997 |
| | | | WO | 9738728 A1 | 23-10-1997 |
| | | | US | 5994319 A | 30-11-1999 |
| WO 0020645 | A | 13-04-2000 | US | 6080580 A | 27-06-2000 |
| | | | US | 6228642 B1 | 08-05-2001 |
| | | | AU | 6290399 A | 26-04-2000 |
| | | | EP | 1119643 A1 | 01-08-2001 |
| | | | WO | 0020645 A1 | 13-04-2000 |
| EP 0691402 | A | 10-01-1996 | DE | 4420298 A1 | 14-12-1995 |
| | | | AU | 695371 B2 | 13-08-1998 |
| | | | AU | 2059995 A | 21-12-1995 |
| | | | CA | 2151446 A1 | 11-12-1995 |
| | | | EP | 0691402 A2 | 10-01-1996 |
| | | | HU | 72483 A2 | 29-04-1996 |
| | | | JP | 8056678 A | 05-03-1996 |
| | | | NZ | 272307 A | 26-03-1996 |
| | | | ZA | 9504772 A | 08-02-1996 |
| WO 0107457 | A | 01-02-2001 | US | 6020199 A | 01-02-2000 |
| | | | AU | 2180800 A | 13-02-2001 |
| | | | EP | 1200456 A1 | 02-05-2002 |
| | | | WO | 0107457 A1 | 01-02-2001 |
| | | | US | 6284538 B1 | 04-09-2001 |
| | | | US | 2002058638 A1 | 16-05-2002 |
| WO 9813526 | A | 02-04-1998 | US | 5849902 A | 15-12-1998 |
| | | | AU | 712680 B2 | 11-11-1999 |
| | | | AU | 4504397 A | 17-04-1998 |
| | | | CN | 1230998 A | 06-10-1999 |
| | | | EP | 0961837 A1 | 08-12-1999 |
| | | | JP | 2001501614 T | 06-02-2001 |
| | | | NO | 991328 A | 25-05-1999 |
| | | | WO | 9813526 A1 | 02-04-1998 |
| | | | US | 5989912 A | 23-11-1999 |
| WO 0190341 | A | 29-11-2001 | US | 6284538 B1 | 04-09-2001 |
| | | | AU | 6180801 A | 03-12-2001 |
| | | | WO | 0190341 A1 | 29-11-2001 |
| | | | US | 2002058638 A1 | 16-05-2002 |
| WO 9954459 | A | 28-10-1999 | AU | 3751299 A | 08-11-1999 |
| | | | CA | 2326823 A1 | 28-10-1999 |
| | | | EP | 1071753 A2 | 31-01-2001 |
| | | | WO | 9954459 A2 | 28-10-1999 |

EPO FORM P0459

For more details about this annex : see Official Journal of the European Patent Office, No. 12/82

105

SEQUENCE LISTING

<110> atugen AG

<120> Compounds and methods for the identification and/or validation of a target

<130> A 19001 EP

<160> 56

<170> PatentIn version 3.1

<210> 1
<211> 23
<212> DNA
<213> Artificial Sequence

<220>
<223> antisense oligonucleotide hPTEN2120L23

<220>
<221> modified_base
<222> (1)..(7)
<223> 2'-O-methylated RNA-oligonucleotides and phosphodiester internucl
      eotide-linked

<220>
<221> modified_base
<222> (17)..(23)
<223> 2'-O-methylated RNA-oligonucleotides and phosphodiester internucl
      eotide-linked

<220>
<221> misc_feature
<222> (8)..(17)
<223> phosphorothioate-internucleotide linked

<220>

<221> misc_feature

<222> (8)..(16)

<223> DNA oligonucleotides


<220>

<221> misc_feature

<222> (1)..(1)

<223> covalently linked to an inverted abasic


<220>

<221> misc_feature

<222> (23)..(23)

<223> covalently linked to an inverted abasic


<400> 1

ugaacugcta gcctctggau uug                                    23


<210> 2

<211> 23

<212> DNA

<213> Artificial Sequence


<220>

<223> antisense oligonucleotide hPTEN2430L23


<220>

<221> modified_base

<222> (1)..(7)

<223> 2'-O-methylated RNA oligonucleotides and phosphodiester internucl
      eotide-linked


<220>

<221> misc_feature

<222> (8)..(17)

<223> phosphorothioate-internucleotide linked

```
<220>
<221> misc_feature
<222> (8)..(16)
<223> DNA oligonucleotides


<220>
<221> modified_base
<222> (17)..(23)
<223> 2'-O-methylated RNA oligonucleotides and phosphodiester internucl
       eotide-linked


<220>
<221> misc_feature
<222> (1)..(1)
<223> covalently linked to an inverted abasic


<220>
<221> misc_feature
<222> (23)..(23)
<223> covalently linked to an inverted abasic


<400> 2
uggacaacaa gtgtcaaaac ccu                                         23


<210> 3
<211> 23
<212> DNA
<213> Artificial Sequence

<220>
<223> antisense oligonucleotide hPTEN2706L23

<220>
<221> modified_base
```

```
<222> (1)..(7)
<223> 2'-O-methylated RNA-oligonucleotides and phosphodiester internucl
       eotide-linked


<220>
<221> misc_feature
<222> (8)..(17)
<223> phosphorothioate-internucleotide linked


<220>
<221> misc_feature
<222> (8)..(16)
<223> DNA oligonucleotides


<220>
<221> modified_base
<222> (17)..(23)
<223> 2'-O-methylated RNA-oligonucleotides and phosphodiester internucl
       eotide-linked


<220>
<221> misc_feature
<222> (1)..(1)
<223> covalently linked to an inverted abasic


<220>
<221> misc_feature
<222> (23)..(23)
<223> covalently linked to an inverted abasic


<400> 3
ggaaacctct cttagccaac ugc                              23


<210> 4
```

<211> 23

<212> DNA

<213> Artificial Sequence

<220>

<223> antisense oligonucleotide hPTEN2991L23

<220>

<221> modified_base

<222> (1)..(7)

<223> 2'-O-methylated RNA oligonucleotide and phosphodiester internucle
otide-linked

<220>

<221> misc_feature

<222> (8)..(17)

<223> phosphorothioate-internucleotide linked

<220>

<221> misc_feature

<222> (8)..(16)

<223> DNA oligonucleotides

<220>

<221> modified_base

<222> (17)..(23)

<223> 2'-O-methylated RNA oligonucleotide and phosphodiester internucle
otide-linked

<220>

<221> misc_feature

<222> (1)..(1)

<223> covalently linked to a inverted abasic

<220>

<221> misc_feature

<222> (23)..(23)

<223> covalently linked to a inverted abasic

<400> 4

uguugcagaa ggttcauucc ugu                          23

<210> 5

<211> 23

<212> DNA

<213> Artificial Sequence

<220>

<223> antisense oligonucleotide hPTEN205L23

<220>

<221> modified_base

<222> (1)..(7)

<223> 2'-O methylated RNA-oligonucleotide and phosphodiester-linked

<220>

<221> misc_feature

<222> (8)..(17)

<223> phosphorothioate-internucleotide linked

<220>

<221> misc_feature

<222> (8)..(16)

<223> DNA oligonucleotides

<220>

<221> modified_base

<222> (17)..(23)

<223> 2'-O methylated RNA-oligonucleotide and phosphodiester-linked

<220>

<220>

<221> misc_feature

<222> (1)..(1)

<223> covalently linked to an inverted abasic

<220>

<221> misc_feature

<222> (23)..(23)

<223> covalently linked to an inverted abasic

<400> 5

cuuccgagag gagagaacug agc                      23

<210> 6

<211> 23

<212> DNA

<213> Artificial Sequence

<220>

<223> antisense oligonucleotide hPTEN1683L23

<220>

<221> modified_base

<222> (1)..(7)

<223> 2'-O-methylated RNA-oligonucleotides phosphodiester internucleoti
de-linked

<220>

<221> misc_feature

<222> (8)..(17)

<223> phosphorothioate-internucleotide linked

<220>

<221> misc_feature

<222> (8)..(16)

<223> DNA oligonucleotides

```
<220>
<221> modified_base
<222> (17)..(23)                    .
<223> 2'-O-methylated RNA-oligonucleotides phosphodiester internucleoti
       de-linked


<220>
<221> misc_feature
<222> (1)..(1)
<223> covalently linked to an inverted abasic


<220>
<221> misc_feature
<222> (23)..(23)
<223> covalently linked to an inverted abasic


<400> 6
ccacaaactg aggattgcaa guu                                    23


<210> 7
<211> 23
<212> DNA
<213> Artificial Sequence


<220>
<223> antisense oligonucleotide hPTEN2346L23


<220>
<221> modified_base
<222> (1)..(7)
<223> 2'-O-methylated RNA oligonucleotides phosphodiester internucleoti
       de-linked


<220>
<221> misc_feature
```

<220>

<221> misc_feature

<222> (8)..(17)

<223> phosphorothioate-internucleotide linked


<220>

<221> misc_feature

<222> (8)..(16)

<223> DNA oligonucleotides


<220>

<221> modified_base

<222> (17)..(23)

<223> 2'-O-methylated RNA oligonucleotides phhosphodiester internucleotide-linked


<220>

<221> misc_feature

<222> (1)..(1)

<223> covalently linked to an inverted abasic


<220>

<221> misc_feature

<222> (23)..(23)

<223> covalently linked to an inverted abasic


<400> 7

ucugacacaa tgtcctauug cca                                          23


<210> 8

<211> 23

<212> DNA

<213> Artificial Sequence


<220>

<223> antisense oligonucleotide hPTEN1009L23

<220>

<221> modified_base

<222> (1)..(7)

<223> 2'-O-methylated RNA-oligonucleotides phosphodiester internucleoti de-linked


<220>

<221> misc_feature

<222> (8)..(17)

<223> phosphorothioate-internucleotide-linked


<220>

<221> misc_feature

<222> (8)..(16)

<223> DNA oligonucleotide


<220>

<221> modified_base

<222> (17)..(23)

<223> 2'-O-methylated RNA-oligonucleotides phosphodiester internucleoti de-linked


<220>

<221> misc_feature

<222> (1)..(1)

<223> covalently linked to an inverted abasic


<220>

<221> misc_feature

<222> (23)..(23)

<223> covalently linked to an inverted abasic


<400> 8

aaggaggaga gagatggcag aag                                        23

<210> 9
<211> 23
<212> DNA
<213> Artificial Sequence

<220>
<223> antisense oligonucleotide rPTEN388L23

<220>
<221> modified_base
<222> (1)..(7)
<223> 2'-O-methylated RNA-oligonucleotides phosphodiester internucleoti
de-linked

<220>
<221> misc_feature
<222> (8)..(17)
<223> phosphorothioate-internucleotide linked

<220>
<221> misc_feature
<222> (8)..(16)
<223> DNA-oligonucleotides

<220>
<221> modified_base
<222> (17)..(23)
<223> 2'-O-methylated RNA-oligonucleotides phosphodiester internucleoti
de-linked

<220>
<221> misc_feature
<222> (1)..(1)
<223> covalently linked to an inverted abasic

```
<220>
<221> misc_feature
<222> (23)..(23)
<223> covalently linked to an inverted abasic


<400> 9
guccuuuccc agctttacag uga                                    23


<210> 10
<211> 23
<212> DNA
<213> Artificial Sequence

<220>
<223> antisense oligonucleotide rPTEN1162L23

<220>
<221> modified_base
<222> (1)..(7)
<223> 2'-O-methylated RNA-oligonucleotides phosphodiester internucleoti
       de-linked


<220>
<221> misc_feature
<222> (8)..(17)
<223> phosphorothioate-internucleotide linked


<220>
<221> misc_geature
<222> (8)..(16)
<223> DNA oligonucleotides


<220>
<221> modified_base
<222> (17)..(23)
<223> 2'-O-methylated RNA-oligonucleotides phosphodiester internucleoti
```

de-linked

<220>

<221> misc_feature

<222> (1)..(1)

<223> covalently linked t oan inverted abasic


<220>

<221> misc_feature

<222> (23)..(23)

<223> covalently linked t oan inverted abasic


<400> 10

cuggaucaga gtcagtggug uca                                    23


<210> 11

<211> 23

<212> DNA

<213> Artificial Sequence


<220>

<223> antisense oligonucleotide rPTEN45L23


<220>

<221> modified_base

<222> (1)..(7)

<223> 2'-O-methylated RNA-oligonucleotides phosphodiester internucleoti

de-linked


<220>

<221> misc_feature

<222> (8)..(17)

<223> phosphorothioate-internucleotide linked


<220>

<221> misc_feature

<222> (8)..(16)

<223> DNA-oligonucleotides


<220>

<221> modified_base

<222> (17)..(23)

<223> 2'-O-methylated RNA-oligonucleotides phosphodiester internucleoti
de-linked


<220>

<221> misc_feature

<222> (1)..(1)

<223> covalently linked to an inverted abasic

•


<220>

<221> misc_feature

<222> (23)..(23)

<223> covalently linked to an inverted abasic


<400> 11

ucuccuuttg tttctgcuaa cga                               23


<210> 12

<211> 23

<212> DNA

<213> Artificial Sequence


<220>

<223> antisense oligonucleotide rPTEN1086L23


<220>

<221> modified_base

<222> (1)..(7)

<223> 2'-O-methylated RNA-oligonucleotides phosphodiester internucleoti
de-linked

<220>

<221> misc_feature

<222> (8)..(17)

<223> phosphorothioate-internucleotide linked

<220>

<221> misc_feature

<222> (8)..(16)

<223> DNA-oligonucleotides

<220>

<221> modified_base

<222> (17)..(23)

<223> 2'-O-methylated RNA-oligonucleotides phosphodiester internucleoti
de-linked

<220>

<221> misc_feature

<222> (1)..(1)

<223> covalently linked to an inverted abasic

<220>

<221> misc_feature

<222> (23)..(23)

<223> covalently linked to an inverted abasic

<400> 12

ugaacugcta gcctctggau uug                                        23

<210> 13

<211> 23

<212> DNA

<213> Artificial Sequence

```
<220>
<223> antisense oligonucleotide rPTEN1190L23


<220>
<221> modified_base
<222> (1)..(7)
<223> 2'-O-methylated RNA-oligonucleotides phosphodiester internucleoti
        de-linked



<220>
<221> misc_feature
<222> (8)..(17)
<223> phosphorothiate-internucleotide linked



<220>
<221> misc_feature
<222> (8)..(16)
<223> DNA-oligonucleotides



<220>
<221> modified_base
<222> (17)..(23)
<223> 2'-O-methylated RNA-oligonucleotides phosphodiester internucleoti
        de-linked



<220>
<221> misc_feature
<222> (1)..(1)
<223> covalently linked to an inverted abasic



<220>
<221> misc_feature
<222> (23)..(23)
<223> covalently linked to an inverted abasic
```

<400> 13

ugcugauctt catcaaaagg uuc                                23


<210> 14
<211> 23
<212> DNA
<213> Artificial Sequence


<220>
<223> antisense oligonucleotide rPTEN763L23


<220>
<221> modified_base
<222> (1)..(7)
<223> 2'-O-methylated RNA-oligonucleotides phosphodiester internucleoti
      de-linked


<220>
<221> misc_feature
<222> (8)..(17)
<223> phosphorothioate-internucleotide linked


<220>
<221> misc_feature
<222> (8)..(16)
<223> DNA-oligonucleotides


<220>
<221> modified_base
<222> (17)..(23)
<223> 2'-O-methylated RNA-oligonucleotides phosphodiester internucleoti
      de-linked


<220>
<221> misc_feature

<222> (1)..(1)
<223> covalently linked to an inverted abasic


<220>
<221> misc_feature
<222> (23)..(23)
<223> covalently linked to an inverted abasic


<400> 14
acuuugatgt caccacacac agg                    23


<210> 15
<211> 23
<212> DNA
<213> Artificial Sequence


<220>
<223> antisense oligonucleotide rPTEN694L23


<220>
<221> modified_base
<222> (1)..(7)
<223> 2'-O-methylated RNA-oligonucleotides phosphodiester internucleoti
      de-linked


<220>
<221> misc_feature
<222> (8)..(17)
<223> phosphorothioate-internucleotide linked


<220>
<221> misc_feature
<222> (8)..(16)
<223> DNA-oligonucleotides

<220>
<221> modified_base
<222> (17)..(23)
<223> 2'-O-methylated RNA-oligonucleotides phosphodiester internucleoti
de-linked


<220>
<221> misc_feature
<222> (1)..(1)
<223> covalently linked to an inverted abasic


<220>
<221> misc_feature
<222> (23)..(23)
<223> covalently linked to an inverted abasic


<400> 15
ugggucctga gttggaggag uag                                                    23


<210> 16
<211> 23
<212> DNA
<213> Artificial Sequence

<220>
<223> antisense oligonucleotide rPTEN670L23

<220>
<221> modified_base
<222> (1)..(7)
<223> 2'-O-methylated RNA oligonucleotides phosphodiester internucleoti
de-linked


<220>
<221> misc_feature
<222> (8)..(17)

<223> phosphorothioate-internucleotide linked

<220>
<221> misc_feature
<222> (8)..(16)
<223> DNA-oligonucleotides

<220>
<221> modified_base
<222> (17)..(23)
<223> 2'-O-methylated RNA oligonucleotides phosphodiester internucleoti
     de-linked

<220>
<221> misc_feature
<222> (1)..(1)
<223> covalently linked to an inverted abasic

<220>
<221> misc_feature
<222> (23)..(23)
<223> covalently linked to an inverted abasic

<400> 16
cuucaccttt agctggcaga cca                                        23

<210> 17
<211> 23
<212> DNA
<213> Artificial Sequence

<220>
<223> antisense oligonucleotide rPTEN576L23

<220>

<221> modified_base

<222> (1)..(7)

<223> 2'-O-methylated RNA-oligonucleotides phosphodiester internucleoti de-linked


<220>

<221> misc_feature

<222> (8)..(17)

<223> phosphorothioate-internucleotide linked


<220>

<221> misc_feature

<222> (8)..(16)

<223> DNA-oligonucleotides


<220>

<221> modified_base

<222> (17)..(23)

<223> 2'-O-methylated RNA-oligonucleotides phosphodiester internucleoti de-linked


<220>

<221> misc_feature

<222> (1)..(1)

<223> covalently linked to an inverted abasic


<220>

<221> misc_feature

<222> (23)..(23)

<223> covalently linked to an inverted abasic


<400> 17

ugccacuggt ctgtaaucca ggt                                    23

<210> 18

<211> 23

<212> DNA

<213> Artificial Sequence


<220>

<223> antisense oligonucleotide rPTEN485L23


<220>

<221> modified_base

<222> (1)..(7)

<223> 2'-O-methylated RNA-oligonucleotides phosphodiester internucleoti
de-linked


<220>

<221> misc_feature

<222> (8)..(17)

<223> phosphorothioate-internucleotide linked


<220>

<221> misc_feature

<222> (8)..(16)

<223> DNA-oligonucleotides


<220>

<221> modified_base

<222> (17)..(23)

<223> 2'-O-methylated RNA-oligonucleotides phosphodiester internucleoti
de-linked


<220>

<221> misc_feature

<222> (1)..(1)

<223> covalently linked to an inverted abasic


<220>

<221> misc_feature

<222> (23)..(23)

<223> covalently linked to an inverted abasic

<400> 18

ucucuggtcc ttacttcccc aua                                    23

<210> 19

<211> 23

<212> DNA

<213> Artificial Sequence

<220>

<223> antisense oligonucleotide rPTEN347L23

<220>

<221> modified_base

<222> (1)..(7)

<223> 2'-O-methylated RNA-oligonucleotides phosphodiester internucleoti
      de-linked

<220>

<221> misc_feature

<222> (8)..(17)

<223> phosphorothioate-internucleotide linked

<220>

<221> misc_feature

<222> (8)..(16)

<223> DNA oligonucleotides

<220>

<221> modified_base

<222> (17)..(23)

<223> 2'-O-methylated RNA-oligonucleotides phosphodiester internucleoti
      de-linked

<220>

<221> misc_feature

<222> (1)..(1)

<223> covalently linked to an inverted abasic


<220>

<221> misc_feature

<222> (23)..(23)

<223> covalently linked to an inverted abasic


<400> 19

ucgucuucac ttagccauug guc                                    23


<210> 20

<211> 23

<212> DNA

<213> Artificial Sequence


<220>

<223> antisense oligonucleotide rPTEN124L23


<220>

<221> modified_base

<222> (1)..(7)

<223> 2'-O-methylated RNA-oligonucleotides phosphodiester internucleoti

de-linked


<220>

<221> misc_feature

<222> (8)..(17)

<223> phosphorothioate-internucleotide linked


<220>

<221> misc_feature

.

<222> (8)..(16)

<223> DNA-oligonucleotides

<220>

<221> modified_base

<222> (17)..(23)

<223> 2'-O-methylated RNA-oligonucleotides phosphodiester internucleoti
de-linked

<220>

<221> misc_feature

<222> (1)..(1)

<223> covalently linked to an inverted abasic

<220>

<221> misc_feature

<222> (23)..(23)

<223> covalently linked to an inverted abasic

<400> 20

gucuuuctgc aggaaauccc aua                                      23

<210> 21

<211> 29

<212> DNA

<213> Artificial Sequence

<220>

<223> antisense oligonucleotide rPTEN+388L23

<220>

<221> modified_base

<222> (1)..(7)

<223> 2'-O-methylated RNA-oligonucleotides

<220>

<221> misc_feature

<222> (8)..(16)

<223> DNA-oligonucleotides


<220>

<221> modified_base

<222> (17)..(23)

<223> 2'-O-methylated RNA-oligonucleotides


<220>

<221> misc_feature

<222> (1)..(4)

<223> phosphorothioate internucleotide-linked


<220>

<221> misc_feature

<222> (4)..(8)

<223> phosphodiester-internucleotide-linked


<220>

<221> misc_feature

<222> (8)..(17)

<223> phosphorothioate-internucleotide-linked


<220>

<221> misc_feature

<222> (17)..(20)

<223> phosphodiester-internucleotide linked


<220>

<221> misc_feature

<222> (20)..(23)

<223> phosphorothioate-internucleotide linked

<220>

<221> misc_feature

<222> (1)..(1)

<223> covalently linked to an inverted abasic


<220>

<221> misc_feature

<222> (23)..(23)

<223> covalently linked to an inverted abasic


<400> 21

gsuscscuuu cccagcttta cagsusgsa                    29


<210> 22

<211> 21

<212> DNA

<213> Artificial Sequence


<220>

<223> GB 72


<400> 22

ttggagtcct caacacactc a                    21


<210> 23

<211> 21

<212> DNA

<213> Artificial Sequence


<220>

<223> GB 84


<400> 23

acacctcaat ccatgcttct c                    21

<210> 24
<211> 21
<212> DNA
<213> Artificial Sequence

<220>
<223> GB 74

<400> 24
atcgcttgta gctgatgtcc t                    21

<210> 25
<211> 21
<212> DNA
<213> Artificial Sequence

<220>
<223> GB 86

<400> 25
tcctgaaact ctggtgtgtg a                    21

<210> 26
<211> 21
<212> DNA
<213> Artificial Sequence

<220>
<223> GB 77

<400> 26
tgttgtcctg cttgttaatg c                    21

<210> 27
<211> 21
<212> DNA
<213> Artificial Sequence

<220>

<223> GB 82

<400> 27

aacctgaaac tgtcctgttg g                                21

<210> 28
<211> 21
<212> DNA
<213> Artificial Sequence

<220>
<223> GB 78

<400> 28

actgactgct cattgtcgtt g                                21

<210> 29
<211> 21
<212> DNA
<213> Artificial Sequence

<220>
<223> GB 83

<400> 29

ccacttgagg aaaaccacac t                                21

<210> 30
<211> 23
<212> DNA
<213> Artificial Sequence

<220>
<223> oligonucleotide of the amplicon: start: 1185, Tag: UPR

<400> 30

cagcattaac aagcaggaca aca                              23

<210> 31
<211> 22
<212> DNA
<213> Artificial Sequence

<220>
<223> oligonucleotide of the amplicon: start: 1271, Tag: LWR

<400> 31
ccatctacca gggacacaaa gg                    22


<210> 32
<211> 28
<212> DNA
<213> Artificial Sequence

<220>
<223> oligonucleotide of the amplicon: start: 1217, Tag: PROBE

<400> 32
aactgaagct ctcttcccac gaggaggc                    28


<210> 33
<211> 23
<212> DNA
<213> Artificial Sequence

<220>
<223> mismatch oligonucleotide

<220>
<221> modified_base
<222> (1)..(7)
<223> 2'-O-methylated RNA-oligonucleotides phosphodiester internucleoti
    de-linked

<220>

<221> misc_feature

<222> (8)..(17)

<223> phosphorothioate-internucleotide linked


<220>

<221> misc_feature

<222> (8)..(16)

<223> DNA-oligonucleotides


<220>

<221> modified_base

<222> (17)..(23)

<223> 2'-O-methylated RNA-oligonucleotides phosphodiester internucleoti
de-linked


<220>

<221> misc_feature

<222> (1)..(1)

<223> covalently linked to an inverted abasic


<400> 33

ucucauuttc tttgtgcuca cga                        23


<210> 34

<211> 23

<212> DNA

<213> Artificial Sequence


<220>

<223> PTEN 48


<220>

<221> misc_feature

<222> (1)..(7)

<223> RNA-oligonucleotides

<220>

<221> misc_feature

<222> (8)..(16)

<223> DNA-oligonucleotides


<220>

<221> misc_feature

<222> (17)..(23)

<223> RNA-oligonucleotides


<400> 34

guccuuuccc agctttacag uga                                    23


<210> 35

<211> 23

<212> DNA

<213> Artificial Sequence


<220>

<223> PTEN 52


<220>

<221> misc_feature

<222> (1)..(7)

<223> RNA-oligonucleotides


<220>

<221> misc_feature

<222> (8)..(16)

<223> DNA-oligonucleotides


<220>

<221> misc_feature

<222> (17)..(23)

<223> RNA-oligonucleotides

<400> 35
cuggaucaga gtcagtggug uca                                23

<210> 36
<211> 23
<212> DNA
<213> Artificial Sequence

<220>
<223> PTEN 53

<220>
<221> misc_feature
<222> (1)..(7)
<223> RNA-oligonucleotides

<220>
<221> misc_feature
<222> (8)..(16)
<223> DNA-oligonucleotides

<220>
<221> misc_feature
<222> (17)..(23)
<223> RNA-oligonucleotides

<400> 36
ucuccuuttg tttctgcuaa cga                                23

<210> 37
<211> 23
<212> DNA
<213> Artificial Sequence

<220>
<223> PTEN 57

<220>
<221> misc_feature
<222> (1)..(7)
<223> RNA-oligonucleotides

<220>
<221> misc_feature
<222> (8)..(23)
<223> DNA-oligonucleotides

<220>
<221> misc_feature
<222> (17)..(23)
<223> RNA-oligonucleotides

<400> 37
ugccacuggt ctgtaaucca ggt                     23

<210> 38
<211> 23
<212> DNA
<213> Artificial Sequence

<220>
<223> mm PTEN 52

<220>
<221> misc_feature
<222> (1)..(7)
<223> RNA-oligonucleotides

<220>

<221> misc_feature

<222> (8)..(16)

<223> DNA-oligonucleotides


<220>

<221> misc_feature

<222> (17)..(23)

<223> RNA-oligonucleotides


<400> 38

cuggaugaga ctgagtgcug uca                                23


<210> 39

<211> 23

<212> DNA

<213> Artificial Sequence


<220>

<223> mm PTEN 53


<220>

<221> misc_feature

<222> (1)..(7)

<223> RNA-oligonucleotides


<220>

<221> misc_feature

<222> (8)..(16)

<223> DNA-oligonucleotides


<220>

<221> misc_feature

<222> (17)..(23)

<223> RNA-oligonucleotides

<400> 39

ucucauuttc tttgtgcuca cga                    23


<210> 40

<211> 23

<212> DNA

<213> Artificial Sequence


<220>

<223> Luciferase


<220>

<221> misc_feature

<222> (1)..(7)

<223> RNA-oligonucleotides


<220>

<221> misc_feature

<222> (8)..(16)

<223> DNA-oligonucleotides


<220>

<221> misc_feature

<222> (17)..(23)

<223> RNA-oligonucleotides


<400> 40

cagaaugtag ccatccaucc uug                    23


<210> 41

<211> 23

<212> DNA

<213> Artificial Sequence


<220>

<223> p110alpha 79

<220>
<221> misc_feature
<222> (1)..(7)
<223> RNA-oligonucleotides


<220>
<221> misc_feature
<222> (8)..(16)
<223> DNA-oligonucleotides


<220>
<221> misc_feature
<222> (17)..(23)
<223> RNA-oligonucleotides


<400> 41
acuccaaagc ctcttgcuca guu                                    23


<210> 42
<211> 23
<212> DNA
<213> Artificial Sequence

<220>
<223> p110alpha 82

<220>
<221> misc_feature
<222> (1)..(7)
<223> RNA-oligonucleotides


<220>
<221> misc_feature
<222> (8)..(16)
<223> DNA-oligonucleotides

<220>

<221> misc_feature

<222> (17)..(23)

<223> RNA-oligonucleotides


<400> 42

uaccacactg ctgaaccagu caa                                    23


<210> 43

<211> 23

<212> DNA

<213> Artificial Sequence


<220>

<223> p110beta 88


<220>

<221> misc_feature

<222> (1)..(7)

<223> RNA-oligonucleotides


<220>

<221> misc_feature

<222> (8)..(16)

<223> DNA-oligonucleotides


<220>

<221> misc_feature

<222> (17)..(23)

<223> RNA-oligonucleotides


<400> 43

caaauuccag tggttcauuc caa                                    23

<210> 44

<211> 23

<212> DNA

<213> Artificial Sequence

<220>

<223> p110beta 93                                '

<220>

<221> misc_feature

<222> (1)..(7)

<223> RNA-oligonucleotides

<220>

<221> misc_feature

<222> (8)..(16)

<223> DNA-oligonucleotides

<220>

<221> misc_feature

<222> (17)..(23)

<223> RNA-oligonucleotides

<400> 44

ggcuaacttc atcttccuuc cca                                23

<210> 45

<211> 23

<212> DNA

<213> Artificial Sequence

<220>

<223> mm p110alpha 79

<220>

<221> misc_feature

<222> (1)..(7)

<223> RNA-oligonucleotides

<220>

<221> misc_feature

<222> (8)..(16)

<223> DNA-oligonucleotides

<220>

<221> misc_feature

<222> (17)..(23)

<223> RNA-oligonucleotides

<400> 45

acugcaaacc ctgttgcuca cuu                                                   23

<210> 46

<211> 23

<212> DNA

<213> Artificial Sequence

<220>

<223> mm p110alpha 93

<220>

<221> misc_feature

<222> (1)..(7)

<223> RNA-oligonucleotides

<220>

<221> misc_feature

<222> (8)..(16)

<223> DNA-oligonucleotides

<220>

<221> misc_feature

<222> (17)..(23)

<223> RNA-oligonucleotides

<400> 46

ggcuaagttc ttcatccuug cca                 23

<210> 47

<211> 22

<212> DNA

<213> Artificial Sequence

<220>

<223> PTEN 5'-primer

<400> 47

caccgccaaa tttaactgca ga                  22

<210> 48

<211> 24

<212> DNA

<213> Artificial Sequence

<220>

<223> PTEN 3'-primer

<400> 48

aagggtttga taagttctag ctgt                24

<210> 49

<211> 31

<212> DNA

<213> Artificial Sequence

<220>

<223> Taqman probe

<220>

<221> modified_base

<222> (1)..(1)

<223> linked to Fam


<220>

<221> modified_base

<222> (31)..(31)

<223> linked to Tamra


<400> 49

tgcacagtat ccttttgaag accataaccc a      31


<210> 50

<211> 21

<212> DNA

<213> Artificial sequence


<220>

<223> antisense oligonucleotide huPTEN:1686L21


<220>

<221> misc_feature

<222> (1)..(6)

<223> 2'-O-methyl ribonucleotide


<220>

<221> misc_feature

<222> (7)..(15)

<223> phosphorothioate-linked deoxyribonucleotide


<220>

<221> misc_feature

<222> (16)..(21)

<223> 2'-O-methyl ribonucleotide

<220>
<221> misc_feature
<222> (1)..(1)
<223> covalently linked to an inverted abasic nucleotide


<220>
<221> misc_feature
<222> (21)..(21)
<223> covalently linked to an inverted abasic nucleotide


<400> 50
agaccacaaa ctgaggauug c                                    21


<210> 51
<211> 21
<212> DNA
<213> Artificial sequence

<220>
<223> antisense oligonucleotide huPTEN:1686L21 4MM

<220>
<221> misc_feature
<222> (1)..(6)
<223> 2'-O-methyl ribonucleotide


<220>
<221> misc_feature
<222> (7)..(15)
<223> phosphorothioate-linked deoxyribonucleotide


<220>
<221> misc_feature
<222> (16)..(21)
<223> 2'-O-methyl ribonucleotide

EP 1 325 955 A1

<220>

<221> misc_feature

<222> (1)..(1)

<223> covalently linked ito an nverted abasic nucleotide


<220>

<221> misc_feature

<222> (21)..(21)

<223> covalently linked to an inverted abasic nucleotide


<400> 51

agacgactaa ctcagcauug c                21


<210> 52

<211> 21

<212> DNA

<213> Artificial sequence


<220>

<223> antisense oligonucleotide huPTEN:1420L21


<220>

<221> misc_feature

<222> (1)..(6)

<223> 2'-O-methyl ribonucleotide


<220>

<221> misc_feature

<222> (7)..(15)

<223> phosphorothioate-linked deoxyribonucleotide


<220>

<221> misc_feature

<222> (15)..(21)

73

<223> 2'-O-methyl ribonucleotide

<220>
<221> misc_feature
<222> (1)..(1)
<223> covalently linked to an inverted abasic nucleotide

<220>
<221> misc_feature
<222> (21)..(21)
<223> covalently linked to an inverted abasic nucleotide

<400> 52
cccuuuccag ctttacagug a                                    21

<210> 53
<211> 21
<212> DNA
<213> Artificial sequence

<220>
<223> antisense oligonucleotide huPTEN:1420 L21 4MM

<220>
<221> misc_feature
<222> (1)..(6)
<223> 2'-O-methyl ribonucleotide

<220>
<221> misc_feature
<222> (7)..(15)
<223> phosphorothioate-linked deoxyribonucleotide

<220>
<221> misc_feature

<222> (16)..(21)

<223> 2'-O-methyl ribonucleotide

<220>

<221> misc_feature

<222> (1)..(1)

<223> covalently linked to an inverted abasic nucleotide

<220>

<221> misc_feature

<222> (21)..(21)

<223> covalently linked to an  inverted abasic nucleotide

<400> 53

ccguuugcac ctttagagug a                                                        21

<210> 54

<211> 21

<212> DNA

<213> Artificial sequence huPTEN intron

<220>

<221> misc_feature

<222> (1)..(6)

<223> 2'-O-methyl ribonucleotide

<220>

<221> misc_feature

<222> (7)..(15)

<223> phosphorothioate-linked deoxyribonucleotide

<220>

<221> misc_feature

<222> (16)..(21)

<223> 2'-O-methyl ribonucleotide

<220>

<221> misc_feature

<222> (1)..(1)

<223> covalently linked to an inverted abasic nucleotide

<220>

<221> misc_feature

<222> (21)..(21)

<223> covalently linked to an inverted abasic nucleotide

<400> 54

aagcagcaaa gtcctaagca g                                    21

<210> 55

<211> 21

<212> DNA

<213> Artificial sequence

<220>

<223> antisense oligonucleotide huPTEN: intron

<220>

<221> misc_feature

<222> (1)..(6)

<223> 2'-O-methyl ribonucleotide

<220>

<221> misc_feature

<222> (7)..(15)

<223> phosphorothioate-linked deoxyribonucleotide

<220>

<221> misc_feature

<222> (16)..(21)

<223> 2'-O-methyl ribonucleotide

<220>
<221> misc_feature
<222> (1)..(1)
<223> covalently linked to an inverted abasic nucleotide

<220>
<221> misc_feature
<222> (21)..(21)
<223> covalently linked to an inverted abasic nucleotide

<400> 55
cagaautggg ctgtauuugg u                                           21

<210> 56
<211> 23
<212> DNA
<213> Artificial sequence

<220>
<223> One strand (5' - 3') of PTEN specific RNAi

<400> 56
cuccuuuugu uucugcuaac gtt                                         23